# EUROPÄISCHE PATENTSCHRIFT

(11) **EP 3 016 987 B1**
(45) Veröffentlichungstag und Bekanntmachung des Hinweises auf die Patenterteilung: **03.06.2020**
(21) Anmeldenummer: 14734494.9
(22) Anmeldetag: 02.07.2014
(51) Int. Cl.: C08F 220/06, C08F 283/06, C09D 133/02, C09J 133/02, C11D 3/37, C08L 33/02, C08L 71/02, C08F 2/24, C08F 2/44, C08F 120/06

(54) **FESTE POLYMERZUSAMMENSETZUNG, ERHALTEN DURCH POLYMERISATION EINES SÄUREGRUPPENHALTIGEN MONOMERS IN GEGENWART EINER POLYETHERVERBINDUNG**
POLYMER COMPOSITION, OBTAINED BY POLYMERISATION OF A MONOMER CONTAINING ACID GROUPS IN THE PRESENCE OF A POLYETHER COMPOUND
COMPOSITION POLYMÈRE OBTENUE PAR POLYMÉRISATION D'UN MONOMÈRE CONTENANT DES GROUPES ACIDES EN PRÉSENCE D'UN COMPOSÉ POLYÉTHER

(30) Priorität: 03.07.2013 EP 13174938
(43) Veröffentlichungstag der Anmeldung: 11.05.2016
(73) Patentinhaber: BASF SE, 67056 Ludwigshafen am Rhein (DE)
(72) Erfinder: FUCHS, Yannick, 69469 Weinheim (DE); WITTELER, Helmut, 67157 Wachenheim (DE); WEBER, Heike, 68239 Mannheim (DE); GARCIA MARCOS, Alejandra, 67063 Ludwigshafen (DE); DETERING, Jürgen, 67117 Limburgerhof (DE)
(86) Internationale Anmeldenummer: PCT/EP2014/064084
(87) Internationale Veröffentlichungsnummer: WO 2015/000970

(56) Entgegenhaltungen:
- WO-A1-2005/012378
- WO-A1-2012/069440
- LEV BROMBERG: "Polyether-Modified Poly(acrylic acid): Synthesis and Applications", INDUSTRIAL & ENGINEERING CHEMISTRY RESEARCH, Bd. 37, Nr. 11, 1. November 1998 (1998-11-01), Seiten 4267-4274, XP055139606, ISSN: 0888-5885, DOI: 10.1021/ie980358s
- LEV BROMBERG: "Novel Family of Thermogelling Materials via C-C Bonding between Poly(acrylic acid) and Poly(ethylene oxide)- b -poly(propylene oxide)- b -poly(ethylene oxide)", THE JOURNAL OF PHYSICAL CHEMISTRY B, Bd. 102, Nr. 11, 20. Februar 1998 (1998-02-20), Seiten 1956-1963, XP055113924, ISSN: 1520-6106, DOI: 10.1021/jp9803687

## Beschreibung

### HINTERGRUND DER ERFINDUNG

Die vorliegende Erfindung betrifft ein Verfahren zur Herstellung einer Polymerzusammensetzung, bei dem man eine α,β-ethylenisch ungesättigte Carbonsäure einer radikalischen Polymerisation in Gegenwart wenigstens einer Polyetherkomponente unterzieht, die nach diesem Verfahren erhältliche Polymerzusammensetzung in Form einer Festsubstanz, speziell einer Folie, einer Beschichtung auf einem Substrat oder eines teilchenförmigen Feststoffs, und die Verwendung einer solchen Polymerzusammensetzung.

### STAND DER TECHNIK

An Wasch-, Reinigungs- und Geschirrspülmittel wird eine Vielzahl von gleichzeitig zu erfüllenden Anforderungen gestellt, sowohl hinsichtlich ihrer Reinigungsleistung als auch der Konfektions- und Angebotsformen. Gute anwendungstechnische Eigenschaften sollen mit einer bequemen Dosierung durch den Verbraucher und der Vereinfachung der zur Durchführung eines Wasch- oder Reinigungsverfahren notwendigen Arbeitsschritte einhergehen. In diesem Zusammenhang werden in jüngster Zeit eine Vielzahl von Dosierungsformen vermarktet, die neben den klassischen Pulvern und Flüssigformulierungen auch tablettenförmige Produkte ("Tabs"), folienummantelte Einzelportionen und Dosiersysteme zur Mehrfachdosierung von Wasch- und Reinigungsmitteln umfassen. Aktuelle Dosierungsformen können eine Vielzahl von separat formulierten Wirk- und Hilfsstoffen enthalten, die im Reinigungsprozess individuell freigesetzt werden. Es besteht hier weiterhin ein großer Bedarf sowohl an wirkaktiven Substanzen als auch an Komponenten, die in Folien und Beschichtungen der Dosierungsformen zum Einsatz kommen. Bevorzugt sind dabei Komponenten, die eine Mehrfachwirkung entfalten, z.B. als filmbildende Komponente, die unter den Reinigungsbedingungen löslich ist und nach ihrer Auflösung am Reinigungsprozess beteiligt ist.

Es ist bekannt, Polyvinylalkohol als wasserlösliches Verpackungs- und Beschichtungsmaterial für Wasch-, Reinigungs- und Geschirrspülmittel, etc. einzusetzen. Nachteilig daran ist, dass der Polyvinylalkohol selbst keine Detergenzwirkung hat und nicht aktiv am Reinigungsprozess beteiligt ist.

In Haushaltsreinigern, Waschmitteln, Geschirrspülmitteln, Klarspülern, Kosmetika, Pharmazeutika und in Formulierungen für technische Anwendungen werden häufig Polyether oder Ethergruppen-haltige Tenside zusammen mit Polymeren α,β-ethylenisch ungesättigter Carbonsäuren und speziell Polyacrylsäure eingesetzt, wobei der Polyacrylsäure oft die Rolle eines Inkrustierungsinhibitors oder Dispergiermittels zukommt. Dabei besteht das Problem, dass Polyether und Ethergruppen-haltige Tenside mit Polyacrylsäure häufig nicht oder nur bedingt verträglich sind, so dass es beim Mischen zur Phasenseparation oder zu Ausfällungen kommt, was die Einsatzmöglichkeiten wenigstens einer der Komponenten stark einschränkt. Weiterhin liegen Polyether und Tenside häufig in wachsartiger oder klebriger Konsistenz vor. Während dies für die Herstellung von Klebstoffformulierungen in der Regel unkritisch ist, können diese Komponenten pulverförmigen Formulierungen nur in geringem Maße zugesetzt werden, weil sonst die Rieselfähigkeit des Pulvers verloren geht. Es ist insbesondere auch schwierig, transparente Folien und Beschichtungen auf Basis von Polyethern oder Ethergruppen-haltigen Tensiden zusammen mit Polymeren ungesättigter Carbonsäuren bereitzustellen, wie sie der Verbraucher sowohl aufgrund ihrer anwendungstechnischen Eigenschaften, wie auch aus ästhetischen Gründen bevorzugt. Es besteht somit ein Bedarf an Zusammensetzungen auf Basis von Polymeren ungesättigter Carbonsäuren und Polyethern, die sich in Form einer Festsubstanz bereitstellen lassen, speziell in Form einer Folie (z.B. als Umhüllung eines flüssigen Spül-, Wasch- oder Reinigungsmittels) oder einer Beschichtung auf einem Substrat (z.B. auf einer Spül-, Wasch- oder Reinigungsmitteltablette) oder in Form eines teilchenförmigen Feststoffs (z.B. eines Granulats oder Pulvers).

F. E. Bailey et al. beschreiben in Polymer Preprints, American Chemical Society, Division of Polymer Chemistry, 1960, vol. 1, issue 2, p. 202 - 205 und der darin zitierten Literatur die Bildung von molekularen Assoziationskomplexen von Ethylenoxid-Polymeren, die ein sehr hohes Molekulargewicht aufweisen, mit polymeren Säuren, wie Polyacrylsäure, in wässrigen Lösungen. Die Polymerisation von Acrylsäure in Gegenwart der Ethylenoxid-Polymere ist nicht beschrieben.

In der EP 0971997 B1 wird eine flüssige Reinigerformulierung beschrieben, die ein nichtionisches Tensid und ein anionisches Polymer enthält. Bei dem nichtionischen Tensid kann es sich um einen ethoxilierten C₈-C₁₈-Alkohol und bei dem anionischen Polymer um Polyacrylsäure handeln. Das Polymer weist ein Molekulargewicht von mehr als 100 000 g/mol auf. Es wird nicht beschrieben, zur Herstellung der Formulierung wenigstens ein säuregruppenhaltiges Polymer in Gegenwart des nichtionischen Tensids zu polymerisieren.

In der WO 2001/83660 wird die Herstellung von Pulvern aus Tensiden und wasserlöslichen Polymeren durch Sprühtrocknung beschrieben. Das Verfahren der Sprühtrocknung ist speziell nicht auf Mischungen mit Polyacrylsäure anwendbar, weil diese mit Polyethern und Polyether-haltigen Tensiden ein gummiartiges Koagulat bildet, das aufgrund seiner Zähigkeit nicht im Sprühturm verarbeitet werden kann.

Die EP 0 499 068 A1 beschreibt Reaktionsprodukte aus Alkoxilaten und vinylischen Monomeren, wobei diese zumindest teilweise funktionelle Gruppen tragen, die mit den OH-Gruppen der Alkoxilate in einer Kondensation reagieren können. Zur Herstellung dieser Reaktionsprodukte polymerisiert man entweder die vinylischen Monomere in Gegenwart der Alkoxilate und unterzieht das Produkt der Polymerisation anschließend einer Kondensation oder man polymerisiert zuerst die vinylischen Monomere und unterzieht anschließend das Produkt der Polymerisation einer Kondensation mit den Alkoxilaten. Im Falle das Acrylsäure als vinylisches Monomer eingesetzt wird, ist das Reaktionsprodukt somit in jedem Fall ein Ester der Polyacrylsäure. In den Ausführungsbeispielen werden als Alkoxilate ausschließlich EO-PO-Blockcopolymere mit hohem PO-Gehalt und Polytetrahydrofuran eingesetzt. Die beschriebenen Polymere dienen als Emulsionsspalter zur Schnellentwässerung von Rohölen. Ihre Herstellung erfolgt in einer ersten Variante durch Massepolymerisation, wobei das Endprodukt als erstarrte Schmelze oder nach Aufnahme in einem Lösungsmittel der Rohölemulsion zugesetzt wird. In einer zweiten Variante erfolgt die Herstellung durch Lösungspolymerisation in einem für den Einsatz als Emulsionsspalter geeigneten Lösungsmittel. Geeignete Lösungsmittel sind Aromaten, aliphatische und cycloaliphatische Kohlenwasserstoffe, Aceton, Cyclohexanon, THF und Dioxan. Ein Einsatz der Polymere in fester Form, speziell in Form einer Folie, einer festen Beschichtung auf einem Substrat oder teilchenförmig ist nicht beschrieben.

Ein ähnliches Verfahren wird in der DE 4326772 A1 beschrieben, wobei Toluol oder Xylol als Lösungsmittel verwendet wird. Die Verwendung von aromatischen Lösungsmitteln ist für Produkte, die in Konsumgütern eingesetzt werden sollen, unerwünscht, da die vollständige Entfernung des Lösemittels sehr zeit- und energieaufwendig ist. Die Reaktionsprodukte sind Flüssigkeiten, die als veresterte Polyacrylsäuren beschrieben werden. Esterbildung läuft der Wirkung des Polymers, beispielsweise als Inkrustierungsinhibitor zuwider und ist nicht erwünscht in Anwendungen, in denen der Einsatz möglichst reiner Polyacrylsäure gefordert ist.

X. Li et al. beschreiben im Journal of Solid State Electrochemistry (2011), 15(6), 1271-1277) und J. H. Wu et al. beschreiben in Advanced Materials, 2007, 19, 4006-4011, Herstellungsverfahren für Polyacrylsäure-Polyethylenglykol-Gele. Diese Publikationen lehren den Einsatz von N, N'-Methylenbisacrylamid als Vernetzer bei der Acrylsäurepolymerisation, so dass die erhaltenen Polymere nicht wasserlöslich sind. Außerdem wird durch den Einsatz eines Vernetzers ein sehr hohes Molekulargewicht erhalten, was die Polymere als Inkrustierungsinhibtoren ungeeignet macht. Ein Einsatz der Polymere in Form von Folien, festen Beschichtungen oder Polymerteilchen ist nicht beschrieben.

In der WO 2008/139151 A1 wird ein Verfahren beschrieben, in dem Polyethylenglyol mit Acrylsäure, Isobornylacrylat und weiteren Komponente gemischt und durch UV-Belichtung zu einem festen Gel ausgehärtet ist. Aufgrund der Zusammensetzung ist für den Fachmann ersichtlich, dass das Gel nicht wasserlöslich ist. Die Gele dienen als Indikator, dass ein Datenträger, z. B. eine computerlesbare Compactdisc, nicht zuvor benutzt wurde.

In der WO 2010/026178/A2 wird auf Seite 62 und in Beispiel 19 eine Fällungspolymerisation beschrieben, bei der Acrylsäure in Gegenwart von Glycerinmonostearat und einem Alkyl-terminierten Polyethylenglycolmethacrylat polymerisiert wird. Letzteres ist ein Assoziativmonomer und kein Polyethergruppenhaltiges Tensid im Sinne der vorliegenden Erfindung. Das Verfahren erfordert zudem den Einsatz einer großen Menge an organischen Lösungsmitteln bezogen auf relativ wenig Acrylsäure und Tensid.

Lev Bromberg beschreibt im Journal of Physical Chemistry B (1998), 102, 11, 1956-1963, ein Material mit thermoreversibler Gelbildung, zu dessen Herstellung man Acrylsäure in Gegenwart eines PEO-PPO-PEO-Blockcopolymers polymerisiert. Die Reaktion erfolgt in Abwesenheit externer Lösungsmittel, um einen hohen Anteil an Verzweigung und Vernetzung in den erhaltenen Produkten zu erzielen. Diese sind weder wasserlöslich noch transparent. Als mögliche Einsatzbereiche für diese Polymere werden nur ganz allgemein die Pharmazie und die Nahrungsergänzung genannt (S. 1956, linke Spalte, "Introduction").

WO 2005/012378 beschreibt wässrige Dispersionen von wasserlöslichen Polymeren von anionischen Monomeren und deren Verwendung als Verdickungsmittel für wässrige Systeme. Zu ihrer Herstellung werden anionische Monomere in Gegenwart von zwei wasserlöslichen Polymeren aus verschiedenen Klassen polymerisiert, wobei es sich unter anderem auch um Polyalkylenglycole handeln kann. Beispiel 4 (Seite 19, Zeilen 14-27) betrifft die Polymerisation von Acrylsäure in Gegenwart von zwei verschiedenen Polypropylenglycolen und von Maltodextrin. Die Dispersionen werden unter Anderem in Personal care-Produkten sowie in Wasch- und Reinigungsmitteln eingesetzt. Ein Einsatz in Form von Folien, festen Beschichtungen oder Polymerteilchen ist nicht beschrieben.

EP 0 639 592 A1 beschreibt Pfropfcopolymere, die durch Polymerisation einer (Meth)acrylsäure-haltigen Polymerzusammensetzung in Gegenwart einer Polyetherverbindung mit mehr als 80 Mol-% Ethylenoxideinheiten erhältlich sind. Die Polymerisation erfolgt im Wesentlichen ohne Lösungsmittel und bei Temperaturen oberhalb von 100 °C. Es wird als kritisch für die Erzielung hoher Pfropfgrade angesehen, dass der Lösungsmittelgehalt des Reaktionsgemischs keinesfalls mehr als 5 Gew.-% beträgt. Die erhaltenen Polymere dienen als Builder für Flüssigwaschmittel oder, gegebenenfalls nach einer Nachvernetzung, als wasserabsorbierende Harze.

WO 2004/099274 beschreibt ein Verfahren zur Herstellung von Polymermischungen durch Polymerisation von Monomeren vom (Meth)acrylsäuretyp in Gegenwart einer Verbindung mit einer Polyalkylenglycol-Struktur. Die erhaltenen Polymermischungen sollen Anteile von Pfropfcopolymeren enthalten, wobei auch nach längerer Lagerung die Polyalkylenglycol-Komponente und die (Meth)acrylsäure-Komponente homogen in der Mischung verbleiben. Die Polymerisation erfolgt zwingend in Gegenwart von Wasser mit einem hohen Wassergehalt in der Vorlage zu Beginn der Reaktion. Die nach dem Verfahren erhaltenen Polymermischungen eignen sich für diverse Detergenzanwendungen, speziell zur Wiederanschmutzverhinderung.

WO2012/069440 offenbart Copolymere, die Carbonsäuregruppen, Sulfonsäuregruppen und Polyalkylenoxidgruppen enthalten, sowie deren Verwendung als belagsinhibierender Zusatz zu Waschund Reinigungsmitteln, insbesondere zu phosphathaltigen und phosphatfreien Reinigungsformulierungen für die maschinelle Geschirrreinigung.

Der vorliegenden Erfindung liegt die Aufgabe zugrunde, eine neue Polymerzusammensetzung und ein Verfahren zur ihrer Herstellung bereitzustellen, wobei die Polymerzusammensetzung ein Homo- oder Copolymer mit einem hohen Anteil an eingebauten Säuremonomeren und eine Polyetherkomponente enthält. Dabei sollen die zuvor beschriebenen Nachteile des Stands der Technik vermieden werden. Die Polymerzusammensetzungen sollen sich in Form einer Festsubstanz bereitstellen lassen, speziell in Form einer Folie, einer Beschichtung auf einem Substrat oder eines teilchenförmigen Feststoffs. Sie sollen sich insbesondere als wasserlösliches Verpackungs- oder Beschichtungsmaterial z.B. für Wasch-, Reinigungs- und Geschirrspülmittel eignen. Des Weiteren sollen Sie selbst über Anwendungseigenschaften verfügen, wie sie für Polymere auf Basis wenigstens einer α,β-ethylenisch ungesättigten Carbonsäure und Polyether typisch sind, z.B. grenzflächenaktive Wirkung, belagsinhibierende Wirkung, inkrustierungsinhibierende Wirkung, etc. Die erfindungsgemäßen Polymerzusammensetzungen sollen sich insbesondere auch in Form von Teilchen (z.B. Pulver oder Granulat) bereitstellen lassen und z.B. als Aktivkomponente für feste Wasch-, Reinigungs- und Geschirrspülmittel eignen.

Überraschenderweise wurde nun gefunden, dass diese Aufgabe gelöst wird, wenn zur Herstellung der Polymerzusammensetzung eine Monomerzusammensetzung auf Basis wenigstens einer α,β-ethylenisch ungesättigten Carbonsäure einer radikalischen Polymerisation in Gegenwart der Polyetherkomponente unterzogen wird.

### ZUSAMMENFASSUNG DER ERFINDUNG

Ein erster Gegenstand der Erfindung ist ein Verfahren zur Herstellung einer Polymerzusammensetzung, bei dem man
a) eine Monomerzusammensetzung M) bereitstellt, die
   A) wenigstens eine α,β-ethylenisch ungesättigte Carbonsäure, und
   B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
   enthält,
b) die in Schritt a) bereitgestellte Monomerzusammensetzung M) einer radikalischen Polymerisation in Gegenwart wenigstens einer Polyetherkomponente PE) unterzieht, die keine copolymerisierbare Doppelbindung aufweist ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, wobei, wenn die Polyetherkomponente PE) ein Polyetherol mit Propylenoxid-Wiederholungseinheiten oder einen Mono- oder Di-(C1-C6 - alkylether) eines Polyetherols mit Propylenoxid-Wiederholungseinheiten umfasst, der Anteil dieser Propylenoxid-Wiederholungseinheiten im Mittel höchstens 10 Einheiten pro Molekül beträgt und die Polyetherole PE Ethylenoxid-Homopolymere oder Ethylenoxid/Propylenoxid-Copolymere sind.

Speziell betrifft die Erfindung ein Verfahren zur Herstellung einer festen Polymerzusammensetzung, insbesondere in Form einer Folie oder in Form einer festen Beschichtung auf einem Substrat oder in Teilchenform.

In einer speziellen Ausführungsform ist die wenigstens eine α,β-ethylenisch ungesättigte Carbonsäure A) teilweise oder vollständig durch wenigstens eine ungesättigte Sulfonsäure oder wenigstens eine ungesättigte Phosphonsäure (= Komponente C) ersetzt.

In einer speziellen Ausführungsform umfasst die Polyetherkomponente PE) wenigstens ein Polyetherol oder einen Mono- oder Di-(C₁-C₂-alkylether) davon oder besteht die Polyetherkomponente PE) aus wenigstens einem Polyetherol oder einem Mono- oder Di-(C₁-C₂-alkylether) davon, das als Alkylenoxideinheiten überwiegend oder ausschließlich Ethylenoxideinheiten eingebaut enthält.

In einer weiteren speziellen Ausführungsform unterzieht man das Reaktionsgemisch während der Polymerisation in Schritt b) und die in Schritt b) erhaltene Polymerzusammensetzung keiner Kondensation unter Entfernung eines niedermolekularen Reaktionsprodukts und/oder in Gegenwart eines Kondensationskatalysators.

In einer ersten Variante des erfindungsgemäßen Verfahrens erhält man eine Polymerzusammensetzung in Form einer transparenten Folie. In einer zweiten Variante des erfindungsgemäßen Verfahrens erhält man eine Polymerzusammensetzung in Form einer festen Beschichtung auf einem Substrat. In einer dritten Variante des erfindungsgemäßen Verfahrens erhält man eine Polymerzusammensetzung in Form einer teilchenförmigen Festsubstanz.

Ein weiterer Gegenstand der Erfindung ist ein Verfahren zur Herstellung einer Polymerzusammensetzung, wie zuvor und im Folgenden definiert, in Form einer Folie, bei dem man
a) eine Monomerzusammensetzung M) bereitstellt, die
   A) wenigstens eine α,β-ethylenisch ungesättigte Carbonsäure, und
   B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
   enthält,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, und
c) das in Schritt b) erhaltene Polymerisat, gegebenenfalls nach Zugabe wenigstens eines Wirkstoffes und/oder wenigstens eines Zusatzstoffes, einer Folienbildung unterzieht, vorzugsweise ausgewählt unter Blasformen, Thermoformen, Gießen und Kalandrieren.

Ein weiterer Gegenstand der Erfindung ist ein Verfahren zur Herstellung einer Polymerzusammensetzung, wie zuvor und im Folgenden definiert, in Form einer festen Beschichtung auf einem Substrat, bei dem man
a) eine Monomerzusammensetzung M) bereitstellt, die
   A) wenigstens eine α,β-ethylenisch ungesättigte Carbonsäure, und
   B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
   enthält,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, und
c) das in Schritt b) erhaltene Polymerisat, gegebenenfalls nach Zugabe wenigstens eines Wirkstoffes und/oder wenigstens eines Zusatzstoffes und/oder wenigstens eines Lösungsmittels und gegebenenfalls unter Erwärmen, in eine fließfähige Form bringt, auf ein Substrat aufträgt und verfestigen lässt.

Ein weiterer Gegenstand der Erfindung ist ein Verfahren zur Herstellung einer Polymerzusammensetzung, wie zuvor und im Folgenden definiert, in Teilchenform, bei dem man
a) eine Monomerzusammensetzung M) bereitstellt, die
   A) wenigstens eine α,β-ethylenisch ungesättigte Carbonsäure, und
   B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
   enthält,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, und
c) das in Schritt b) erhaltene Polymerisat, gegebenenfalls nach Zugabe wenigstens eines Zusatzstoffes und/oder wenigstens eines Lösungsmittels und gegebenenfalls unter Erwärmen, in eine fließfähige Form bringt und daraus eine teilchenförmige Zusammensetzung formt.

Ein weiterer Gegenstand der Erfindung ist eine Polymerzusammensetzung, erhältlich durch ein Verfahren, wie zuvor und im Folgenden definiert.

Ein weiterer Gegenstand der Erfindung ist eine Polymerzusammensetzung, wie zuvor und im Folgenden definiert, in Form einer transparenten Folie, einer festen Beschichtung auf einem Substrat oder einer teilchenförmigen Festsubstanz.

Ein weiterer Gegenstand der Erfindung ist eine Polymerzusammensetzung in Form einer Folie oder einer Beschichtung auf einem Substrat, die wenigstens einen Wirkstoff enthält.

In einer speziellen Ausführung ist der Wirkstoff ausgewählt unter Enzymen, Buildern, Bleichmitteln, Tensiden, Basen, Korrosionsinhibitoren, Entschäumern, Farbstoffen, Duftstoffen, Füllstoffen, Tablettierhilfsmitteln, Desintegrationsmiteln, Verdickern, Löslichkeitsvermittlern, organischen Lösemitteln, Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln, UV-Absorbern und Mischungen davon.

Eine spezielle Ausführung der Erfindung ist eine Polymerzusammensetzung in Form einer Folie oder einer Beschichtung auf einem Substrat, die wenigstens ein Enzym enthält. Eine weitere spezielle Ausführung der Erfindung ist eine Umhüllung für eine Waschmittel-, Reinigungsmittel- oder Geschirrspülmittelportion, in Form einer Folie oder einer Beschichtung auf einem Substrat, die wenigstens ein Enzym enthält.

Ein weiterer Gegenstand der Erfindung ist die Verwendung einer Polymerzusammensetzung, wie zuvor und im Folgenden definiert,
- in Wasch- und Reinigungsmitteln,
- in Geschirrspülmitteln und in Klarspülern,
- in Hygieneprodukten,
- in kosmetischen Mitteln,
- in pharmazeutischen Mitteln,
- in Pflanzenschutzmitteln,
- in Netzmitteln,
- in Lacken, Beschichtungsmitteln, Klebstoffen, Lederbehandlungs- oder Textilbehandlungsmitteln, etc.,
- bei der Erschließung und/oder Ausbeutung unterirdischer Erdöl- und/oder Erdgaslagerstätten.

Ein weiterer Gegenstand der Erfindung ist die Verwendung einer Polymerzusammensetzung, wie zuvor und im Folgenden definiert, in flüssigen oder festen Waschmitteln und in Reinigungsmitteln für feste Oberflächen.

Ein weiterer Gegenstand der Erfindung ist die Verwendung einer Polymerzusammensetzung, wie zuvor und im Folgenden definiert, zur oder in der Umhüllung eines flüssigen oder festen Wasch- und Reinigungsmittel.

Ein weiterer Gegenstand der Erfindung ist die Verwendung einer Polymerzusammensetzung, wie zuvor und im Folgenden definiert, als Überzugsmittel oder als Komponente eines Überzugsmittels für ein pharmazeutisches Mittel, Tierarzneimittel oder Lebensmittel, speziell zur Herstellung überzogener Tabletten, Pellets, Mikrokapseln, Granulate oder Kristalle.

Ein weiterer Gegenstand der Erfindung ist eine Umhüllung für eine Waschmittel-, Reinigungsmittel- oder Geschirrspülmittelportion, umfassend oder bestehend aus einer Polymerzusammensetzung, erhältlich durch ein Verfahren, wie zuvor und im Folgenden definiert.

Ein weiterer Gegenstand der Erfindung ist eine Klebstoffzusammensetzung, umfassend oder bestehend aus einer Polymerzusammensetzung, erhältlich durch ein Verfahren, wie zuvor und im Folgenden definiert. Die erfindungsgemäße Klebstoffzusammensetzung eignet sich für eine Vielzahl von Anwendungen, z.B. für beschichtete Etiketten, speziell weichmacherfreie und/oder selbstklebende und/oder wiederablösbare Papieretiketten.

### BESCHREIBUNG DER ERFINDUNG

Das erfindungsgemäße Verfahren und die danach erhaltenen Polymerzusammensetzungen weisen die folgenden Vorteile auf:
- Das erfindungsgemäße Verfahren eignet sich zur Herstellung von Zusammensetzungen auf Basis von Polymeren ungesättigter Säuren (speziell ungesättigter Carbonsäuren) und Polyethern, die sich in Form einer Festsubstanz bereitstellen lassen. Das Verfahren ist variablen bezüglich der erhaltenen festen Polymerzusammensetzung. Es eignet sich zum einen zur Herstellung fester Polymerzusammensetzungen in Form einer Folie. Dabei ist es erstmals möglich, transparente wasserlösliche Folien auf Basis von Polyacrylsäure und Polyethern bereit zu stellen.
- Das erfindungsgemäße Verfahren eignet sich weiterhin zur Herstellung einer Beschichtung auf einem Substrat, z.B. in oder auf einer Spül-, Wasch- oder Reinigungsmitteltablette.
- Das erfindungsgemäße Verfahren eignet sich weiterhin zur Herstellung fester teilchenförmiger und speziell granulat- und pulverförmiger Polymerzusammensetzungen. Vorteilhafterweise sind diese Zusammensetzungen nicht oder nur in geringem Ausmaß klebrig und eignen sich zur Herstellung rieselfähiger fester Formulierungen. Die Bereitstellung dieser speziellen teilchenförmigen Polymerzusammensetzungen gelingt durch Auswahl einer geeigneten Glastemperatur und/oder Polyetherkomponente PE), wie im Folgenden näher ausgeführt.
- Die nach dem erfindungsgemäßen Verfahren erhaltenen Polymerzusammensetzungen zeichnen sich zum einen durch gute filmbildende Eigenschaften und zum anderen durch ihre Eignung als aktive Komponente z.B. von Wasch-, Reinigungs- und Geschirrspülformulierungen aus. So eignen sich die erfindungsgemäßen Polymerzusammensetzungen z.B. als Komplexbildner, soil release Polymere oder Inkrustierungsinhibitoren. Sie eignen sich in vorteilhafter Weise als Ersatz von Polyvinylalkohol bei der Bereitstellung von Folien und Beschichtungen für diverse Dosierungsformen.
- Aufgrund der zuvor genannten Eigenschaften lassen sich die erfindungsgemäßen Polymerzusammensetzungen in einer Vielzahl von Dosierungsformen und in mehreren Komponenten dieser Dosierungsformen einsetzten. So kann beispielsweise eine Einzelportion einer Wasch-, Reinigungs- oder Geschirrspülformulierung die erfindungsgemäße Polymerkomponente in einer flüssigen oder festen Aktivkomponente und/oder in der Beschichtung auf wenigstens einer Festkomponente und/oder in der Ummantelung wenigstes einer flüssigen oder festen Aktivkomponente enthalten.
- Das erfindungsgemäße Verfahren ermöglicht den weitgehenden oder vollständigen Verzicht vernetzender Monomere bei der Herstellung der Polymerzusammensetzungen. Diese sind somit vorteilhafterweise wasserlöslich.
- Die nach dem erfindungsgemäßen Verfahren erhaltenen Polymerzusammensetzungen zeichnen sich sowohl durch eine hohe Verträglichkeit zwischen Polyetherkomponente und Polyacrylsäure als auch durch eine hohe Verträglichkeit mit weiteren Tensiden aus.
- Das Auflöseverhalten der nach dem erfindungsgemäßen Verfahren erhaltenen Polymerzusammensetzungen lässt sich in einem weiten Bereich steuern, z.B. über die Art und Menge der eingesetzten Monomere. Unlösliche Zusammensetzungen oder solche mit einer langsamen Auflöserate eignen sich vorteilhaft, um Wirkstoffkombinationen mit einem verzögerten Freisetzungsprofil zu versehen.
- Die nach dem erfindungsgemäßen Verfahren erhaltenen Polymerzusammensetzungen eignen sich vorteilhaft zur Herstellung von Folien oder Beschichtungen, die wenigstens einen Wirkstoff enthalten. Somit ist es beispielsweise möglich, eine Umhüllung für eine Waschmittel-, Reinigungsmittel- oder Geschirrspülmittelportion bereitzustellen, aus der kontrolliert wenigstens ein Wirkstoff beim Waschvorgang, Reinigungsvorgang oder Spülvorgang freigesetzt wird.
- Das erfindungsgemäße Verfahren eignet sich weiterhin zur Herstellung von Polymerzusammensetzungen für Klebstoffzusammensetzungen. Die Bereitstellung dieser speziellen Polymerzusammensetzungen gelingt durch Auswahl einer geeigneten Glastemperatur und/oder Polyetherkomponente PE), wie im Folgenden näher ausgeführt.

Im Rahmen dieser Anmeldung werden Verbindungen, die von Acrylsäure und Methacrylsäure abgeleitet sein können teilweise durch Einfügen der Silbe "(meth)" in die von der Acrylsäure abgeleitete Verbindung bezeichnet.

Im Rahmen der vorliegenden Erfindung steht C₁-C₆-Alkyl für einen linearen oder verzweigten Alkylrest mit 1 bis 5 Kohlenstoffatomen. Beispiele hierfür sind Methyl, Ethyl, Propyl, Isopropyl, n-Butyl, sec-Butyl, Isobutyl, tert-Butyl, Pentyl und Stellungsisomere davon.

C₇-C₃₀-Alkyl steht für einen linearen oder verzweigten Alkylrest mit 6 bis 30 Kohlenstoffatomen. Beispiele hierfür sind Heptyl, Octyl, 2-Ethylhexyl, Nonyl, Decyl, 2-Propylheptyl, Undecyl, Dodecyl, Tridecyl, Tetradecyl, Pentadecyl, Hexadecyl, Heptadecyl, Octadecyl, Nonadecyl, Eicosyl, Henicosyl, Docosyl, Tricosyl, Tetracosyl und Stellungsisomere davon.

Die Herstellung der erfindungsgemäßen Polymerzusammensetzungen erfolgt durch radikalische Polymerisation der Monomerzusammensetzung M) in Gegenwart wenigstens einer Polyetherkomponente PE), die in der Regel keine copolymerisierbare Doppelbindung aufweist. Dabei werden spezielle Polymerzusammensetzungen mit vorteilhaften Eigenschaften erhalten. Ohne an eine Theorie gebunden zu sein, kann dies beispielsweise auf eine Wirkung der Polyetherkomponente PE) als Schutzkolloid oder Emulgator zurückzuführen sein. Dies kann beispielsweise auch aus einer zumindest teilweisen Pfropfung auf die Polyetherkomponente als Pfropfgrundlage resultieren. Es sind jedoch auch andere Mechanismen als eine Pfropfung vorstellbar. Die erfindungsgemäßen Polymerzusammensetzungen umfassen ganz allgemein die Verfahrensprodukte der radikalischen Polymerisation, worunter z. B. Pfropfpolymerisate, Homo- und Copolymere der in der Monomermischung M) enthaltenen Monomere, Mischungen von Pfropfpolymerisaten mit ungepfropften Verbindungen der Polyetherkomponente PE) sowie beliebige Mischungen verstanden werden.

In einer speziellen bevorzugten Ausführung werden während und im Anschluss an die Polymerisation in Schritt b) das Reaktionsgemisch und die in Schritt b) erhaltene Polymerzusammensetzung keiner Kondensation unter Entfernung eines niedermolekularen Reaktionsprodukts und/oder in Gegenwart eines Kondensationskatalysators unterzogen. Darunter wird verstanden, dass speziell keine zusätzlichen Maßnahmen durchgeführt werden mit dem Ziel, den Estergruppengehalt der nach dem erfindungsgemäßen Verfahren erhaltenen Polymerzusammensetzung zu erhöhen. In einer anderen speziellen Ausführung ist die Esterbildung während und/oder im Anschluss an die Polymerisation in Schritt b) zugelassen.

Speziell liegt die erfindungsgemäße Polymerzusammensetzung bei Normalbedingungen (23°C, 1013 mbar) als Feststoff vor. Im Rahmen der Erfindung bezeichnen die Begriffe "Festsubstanz" und "in fester Form" sowohl einen lösungsmittelfreien Feststoff als auch feste Zusammensetzungen, die eine erfindungsgemäße Polymerzusammensetzung und wenigstens ein Lösungsmittel enthalten. Bevorzugt beträgt der Lösungsmittelgehalt der festen Zusammensetzungen höchstens 10 Gew.-%, besonders bevorzugt höchstens 5 Gew.-%, insbesondere höchstens 2 Gew.-%, bezogen auf das Gesamtgewicht aus Polymerzusammensetzung und Lösungsmittel. Geeignete Lösungsmittel- und Lösungsmittelgemische sind im Folgenden als Komponente LM) beschrieben.

Die erfindungsgemäße Polymerzusammensetzung liegt nicht in Form eines Gels vor. Gelförmige Zusammensetzungen sind Gegenstand von parallelen Anmeldungen. In der Regel können erfindungsgemäße Polymerzusammensetzungen, die mehr als 10 Gew.-% Lösungsmittel, bezogen auf das Gesamtgewicht aus Polymerzusammensetzung und Lösungsmittel aufweisen, Gele bilden.

Im Rahmen der vorliegenden Erfindung bezeichnet der Begriff "Folie" ein flächiges Gebilde, das eine im Wesentlichen zweidimensionale Ausdehnung aufweist. Die Dicke der erfindungsgemäßen Folien beträgt vorzugsweise 0,5 µm bis 10 mm, besonders bevorzugt 1 µm bis 5 mm. Die Länge und/oder Breite der Folie beträgt in der Regel mindestens 5 mm und bevorzugt mindestens 10 mm. Die maximale Länge und/oder Breite der Folie ist in der Regel unkritisch und kann je nach Anwendungsbereich im Millimeter-, Zentimeter- oder Meterbereich liegen.

Liegt die erfindungsgemäßen Polymerzusammensetzungen als feste Beschichtung auf einem Substrat vor, so liegt die Dicke der Beschichtung vorzugsweise in einem Bereich von 0,5 µm bis 10 mm, besonders bevorzugt 1 µm bis 5 mm.

Im Rahmen der vorliegenden Erfindung bezeichnen die Begriffe "teilchenförmige Festsubstanz" und "teilchenförmige Polymerzusammensetzung" eine Zusammensetzung, die Teilchen enthält, deren Teilchengröße vorzugsweise von pulverförmigen Partikeln bis Granulatgröße reicht. Bevorzugt liegt die Teilchengröße in einem Bereich von etwa 50 µm bis 10 mm, besonders bevorzugt 100 µm bis 5 mm.

Die im Rahmen dieser Anmeldung beschriebenen Glasübergangstemperaturen (Tg) können mittels Differential Scanning Calorimetry (DSC) bestimmt werden. Die DSC-Messung wird an ein- und derselben Probe zweckmäßigerweise ein- bis zweimal wiederholt, um eine definierte thermische Vorgeschichte des jeweiligen Polyamids sicherzustellen. Die Aufheiz- und Abkühlrate betrug dabei 20 K/min.

Für den Einsatz in Form einer Folie oder in Form einer Beschichtung auf einem Substrat werden vorzugsweise flexible erfindungsgemäße Polymerzusammensetzungen mit einer niedrigen Glasübergangstemperatur T_{G} eingesetzt. Vorzugsweise weisen die erfindungsgemäßen Polymerzusammensetzungen für den Einsatz in Form einer Folie oder in Form einer Beschichtung auf einem Substrat eine Glasübergangstemperatur T_{G} im Bereich von 0 bis 50°C, vorzugsweise von 5 bis 20 °C, auf.

Für den Einsatz in Form einer teilchenförmigen Festsubstanz werden vorzugsweise spröde erfindungsgemäße Polymerzusammensetzungen mit einer hohen Glasübergangstemperatur T_{G} eingesetzt. Vorzugsweise weisen die erfindungsgemäßen Polymerzusammensetzungen für den Einsatz in Form einer teilchenförmigen Festsubstanz eine Glasübergangstemperatur T_{G} im Bereich von 15 bis 150°C, vorzugsweise von 30 bis 130 °C, auf.

Für den Einsatz in einer Klebstoffzusammensetzung werden vorzugsweise erfindungsgemäße Polymerzusammensetzungen mit niedriger Glasübergangstemperatur eingesetzt. Vorzugsweise weisen die erfindungsgemäßen Polymerzusammensetzungen für den Einsatz in Klebstoffzusammensetzungen eine Glasübergangstemperatur T_{G} im Bereich von - 100 bis + 10°C, vorzugsweise von - 80 bis 0 °C, auf.

Die Steuerung der Glasübergangstemperatur T_{G} steuern kann prinzipiell über die Art und den Anteil der Monomere sowie das Molekulargewicht erfolgen. Eine weitere Möglichkeit zur Steuerung der Glasübergangstemperatur T_{G} liegt in der Auswahl der Polyetherkomponente PE. Die Bereitstellung einer erfindungsgemäßen Polymerzusammensetzung mit gewünschter Glasübergangstemperatur T_{G} liegt im Können des Fachmanns und kann mittels Routinemessungen überprüft werden.

In einer bevorzugten Ausführung der ersten Variante liegen die erfindungsgemäßen Polymerzusammensetzungen in Form einer transparenten Folie vor. Die Transparenz eines Materials wird bestimmt durch sein Absorptions- und Streuverhalten, d.h. die durchgelassene Lichtmenge und das Erscheinungsbild in der Durchsicht. Die Gesamttransmission (Lichtdurchlässigkeit) ist das Verhältnis von durchgelassenem Licht zu einfallendem Licht. Als Maß für die Lichtdurchlässigkeit wird der Transmissionsgrad τ verwendet: Er ist der Quotient aus dem Lichtstrom Φₙ hinter und Φᵥ vor dem zu prüfenden Material und wird in Prozent angegeben. Dieser Wert enthält neben der Absorbtion auch die Streu- und Reflexionsverluste. Der Transmissionsgrad wird im allgemeinen in Luft bestimmt und wird als Funktion der Wellenlänge angegeben.

Im Rahmen der Erfindung wird die Lichtdurchlässigkeit (T_{L}) bei einer Wellenlänge von 500 nm bestimmt. Als Bezugsgröße für die maximale Lichtdurchlässigkeit (T_{L}-Wert von 100 %) dient Wasser.

Vorzugsweise weist die erfindungsgemäßen Polymerzusammensetzung in Form einer transparenten Folie einen T_{L}-Wert gemessen bei 500 nm, von mindestens 85 %, besonders bevorzugt von mindestens 90 %, bezogen auf die Lichtdurchlässigkeit von Wasser, auf.

In einer zweiten Variante des erfindungsgemäßen Verfahrens erhält man eine Polymerzusammensetzung in Form einer Festsubstanz.

Geeignete Materialkennwerte zur Charakterisierung der Polymerzusammensetzung in Form einer Festsubstanz sind das Speichermodul, das Verlustmodul (G") sowie der Verlustfaktor tan(δ) entsprechend dem Quotienten G"/G'.

### Monomerzusammensetzung M)

### Carbonsäuremonomer A)

Die α,β-ethylenisch ungesättigte Carbonsäure A) ist vorzugsweise ausgewählt unter Acrylsäure, Methacrylsäure, Ethacrylsäure, α-Chloracrylsäure, Crotonsäure, Maleinsäure, Itaconsäure, Citraconsäure, Mesaconsäure, Glutaconsäure, Aconitsäure, Fumarsäure und Mischungen davon. Zu den Monomeren A) zählen auch die Salze der zuvor genannten Säuren, insbesondere die Natrium-, Kalium- und Ammoniumsalze sowie die Salze mit Aminen. Die Monomere A) können als solche oder als Mischungen untereinander eingesetzt werden. Die angegebenen Gewichtsanteile beziehen sich sämtlich auf die Säureform.

Bevorzugt wird die wenigstens eine α,β-ethylenisch ungesättigte Carbonsäure in nicht neutralisierter Form zur Polymerisation eingesetzt.

Besonders bevorzugt ist die Komponente A) ausgewählt unter Acrylsäure, Methacrylsäure und Mischungen davon.

Insbesondere wird als Komponente A) ausschließlich Acrylsäure eingesetzt.

Die Komponente A) wird vorzugsweise in einer Menge von 50 bis 100 Gew.-%, besonders bevorzugt 60 bis 100 Gew.-%, bezogen auf das Gesamtgewicht der Monomerzusammensetzung M), eingesetzt.

In einer bevorzugten Ausführungsform besteht die Monomerzusammensetzung M) zu mindestens 80 Gew-%, bevorzugt zu mindestens 90 Gew.-%, insbesondere zu mindestens 95 Gew.-%, bezogen auf das Gesamtgewicht der eingesetzten Monomere, aus Acrylsäure.

### Vernetzer B)

Die erfindungsgemäße Polymerzusammensetzung enthält im Wesentlichen unvernetzte Polymere. Die zur Herstellung der erfindungsgemäßen Polymerzusammensetzung eingesetzte Monomerzusammensetzung M) enthält somit keine oder nur geringe Mengen an vernetzend wirkenden Monomeren B). Vernetzer im Sinne der Erfindung sind Verbindungen mit zwei oder mehr als zwei polymerisierbaren ethylenisch ungesättigten Doppelbindungen pro Molekül.

Vorzugsweise werden Vernetzer B) in einer Menge von 0 bis 0,1 Gew.-%, besonders bevorzugt 0 bis 0,05 Gew.-%, bezogen auf das Gesamtgewicht der Monomerzusammensetzung M), verwendet. In einer speziellen Ausführungsform enthält die Monomerzusammensetzung M) keine vernetzend wirkenden Monomere B), die zwei oder mehr als zwei polymerisierbare α,β-ethylenisch ungesättigte Doppelbindungen pro Molekül aufweisen.

Geeignete Vernetzer B) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000 g/mol. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer B) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer B) sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer B) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000 g/mol.

Als Vernetzer B) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer B) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer B) sind Divinyldioxan, Tetraallylsilan oder Tetravinylsilan.

### Ungesättigte Sulfon- oder Phosphonsäuren C)

Die Monomerzusammensetzung M) kann anstelle der Komponente A) wenigstens eine ungesättigte Sulfonsäure oder Phosphonsäure C) enthalten. Die Monomerzusammensetzung M) kann auch zusätzlich als Komponente C) wenigstens eine ungesättigte Sulfonsäure oder Phosphonsäure enthalten.

Die Komponente C) ist vorzugsweise ausgewählt unter 2-Acrylamido-2-methylpropansulfonsäure, Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, Vinylphosphonsäure, Allylphosphonsäure sowie Mischungen davon.

Bevorzugt als Komponente C) ist 2-Acrylamido-2-methylpropansulfonsäure.

Zu den Monomeren C) zählen auch die Salze der zuvor genannten Säuren, insbesondere die Natrium-, Kalium- und Ammoniumsalze sowie die Salze mit Aminen. Die Monomere C) können als solche oder als Mischungen untereinander eingesetzt werden. Die angegebenen Gewichtsanteile beziehen sich sämtlich auf die Säureform. Bevorzugt besteht die Monomerzusammensetzung M) zu mindestens 80 Gew-%, besonders bevorzugt zu mindestens 90 Gew.-%, insbesondere zu mindestens 95 Gew.-%, bezogen auf das Gesamtgewicht der Monomerzusammensetzung M), aus Monomeren A) und C). Wenn die Monomerzusammensetzung M) wenigstens ein Monomer C) enthält, so wird dieses vorzugsweise in einer Menge von 0,1 bis 50 Gew.-%, besonders bevorzugt 1 bis 25 Gew.-%, bezogen auf das Gesamtgewicht der Monomerzusammensetzung M) eingesetzt.

### Ethergruppenhaltiges Monomer D)

Die Monomerzusammensetzung M) kann zusätzlich wenigstens ein Monomer D) enthalten, ausgewählt unter Verbindungen der allgemeinen Formeln (I.a) und (I.b) 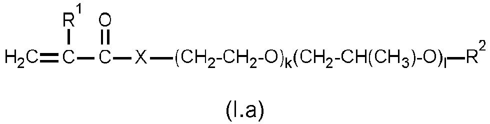 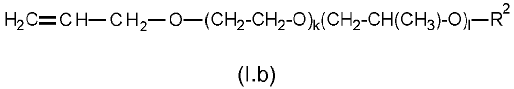 worin
die Reihenfolge der Alkylenoxideinheiten beliebig ist,
k und I unabhängig voneinander für eine ganze Zahl von 0 bis 1000 stehen, wobei die Summe aus k und I mindestens 2 beträgt, vorzugsweise mindestens 5 beträgt,
- R¹: für Wasserstoff oder C₁-C₈-Alkyl steht,
- R²: für Wasserstoff, C₁-C₃₀-Alkyl, C₂-C₃₀-Alkenyl oder C₅-C₈-Cycloalkyl steht,
- X: für O oder eine Gruppe der Formel NR³ steht, worin R³ für H, Alkyl, Alkenyl, Cycloalkyl, Heterocycloalkyl, Aryl oder Hetaryl steht.

In den Formeln I.a) und I.b) steht k vorzugsweise für eine ganze Zahl von 1 bis 500, besonders bevorzugt 2 bis 400, insbesondere 3 bis 250. Bevorzugt steht I für eine ganze Zahl von 0 bis 100.

Bevorzugt steht R¹ in der Formel I.a) für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R² in den Formeln I.a) und I.b) für n-Octyl, 1,1,3,3-Tetramethylbutyl, Ethylhexyl, n-Nonyl, n-Decyl, n-Undecyl, Tridecyl, Myristyl, Pentadecyl, Palmityl, Heptadecyl, Octadecyl, Nonadecyl, Arrachinyl, Behenyl, Lignocerenyl, Cerotinyl, Melissinyl, Palmitoleinyl, Oleyl, Linolyl, Linolenyl, Stearyl, Lauryl.

Vorzugsweise steht X in der Formel I.a) für O oder NH, insbesondere für O.

Geeignete Polyetheracrylate I.a) sind z. B. die Polykondensationsprodukte der zuvor genannten α,β-ethylenisch ungesättigten Mono- und/oder Dicarbonsäuren und deren Säurechloriden, -amiden und -anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R²-OH hergestellt werden. Die Alkylenoxide können einzeln, alternierend nacheinander oder als Mischung eingesetzt werden. Die Polyetheracrylate I.a) können allein oder in Mischungen zur Herstellung der erfindungsgemäß eingesetzten Polymere verwendet werden.

Geeignete Allylalkoholalkoxilate I.b) sind z. B. die Veretherungsprodukte von Allylchlorid mit entsprechenden Polyetherolen. Geeignete Polyetherole können leicht durch Umsetzung von Ethylenoxid, 1,2-Propylenoxid und/oder Epichlorhydrin mit einem Starteralkohol R²-OH hergestellt werden. Die Alkylenoxide können einzeln, alternierend nacheinander oder als Mischung eingesetzt werden. Die Allylalkoholalkoxilate I.b) können allein oder in Mischungen zur Herstellung der erfindungsgemäß eingesetzten Polymere verwendet werden.

Speziell wird als Monomer D) Methyldiglycolacrylat, Methyldiglycolmethacrylat, Ethyldiglycolacrylat oder Ethyldiglycolmethacrylat eingesetzt. Bevorzugt ist Ethyldiglycolacrylat.

Die Monomerzusammensetzung M) enthält vorzugsweise 0 bis 50 Gew.-%, besonders bevorzugt 0 bis 25 Gew.-%, insbesondere 0 bis 10 Gew.-%, bezogen auf das Gesamtgewicht der Monomerzusammensetzung M), wenigstens ein ethergruppenhaltiges Monomer D). Wenn die Monomerzusammensetzung M) wenigstens ein Monomer D) enthält, so vorzugsweise in einer Menge von 0,1 bis 50 Gew.-%, besonders bevorzugt 1 bis 25 Gew.-%, insbesondere 1,5 bis 10 Gew.-%, bezogen auf das Gesamtgewicht der Monomerzusammensetzung M).

### Weitere Monomere E) bis O)

Die Monomerzusammensetzung M) kann zusätzlich wenigstens ein weiteres von den Monomeren A) bis D) verschiedenes Monomer enthalten. Bevorzugt enthält die Monomerzusammensetzung M) zusätzlich wenigstens ein Comonomer, ausgewählt unter
E) Vinylaromaten,
F) C₂-C₈ Monoolefinen, nichtaromatischen Kohlenwasserstoffen mit wenigstens zwei konjugierten Doppelbindungen,
G) Estern α,β-ethylenisch ungesättigter Mono- und Dicarbonsäuren mit C₁-C₂₀-Alkanolen,
H) Verbindungen mit einer radikalisch polymerisierbaren α,β-ethylenisch ungesättigten Doppelbindung und wenigstens einer kationogenen und/oder kationischen Gruppe pro Molekül,
I) Estern von Vinylalkohol oder Allylalkohol mit C₁-C₃₀-Monocarbonsäuren,
K) Amidgruppen-haltigen Monomeren,
L) Estern α,β-ethylenisch ungesättigter Mono- und Dicarbonsäuren mit C₂-C₃₀-Alkanediolen, Amiden α,β-ethylenisch ungesättigter Mono- und Dicarbonsäuren mit C₂-C₃₀-Aminoalkoholen mit einer primären oder sekundären Aminogruppe,
M) α,β-ethylenisch ungesättigten Nitrilen,
N) Vinylhalogeniden, Vinylidenhalogeniden,
O) Monomeren mit Harnstoffgruppen,
und Mischungen davon.

Die Monomerzusammensetzung M) kann die weiteren Monomere E) bis O) jeweils vorzugsweise in einer Menge von 0 bis 50 Gew.-%, besonders bevorzugt 0 bis 25 Gew.-%, insbesondere 0 bis 10 Gew.-%, bezogen auf das Gesamtgewicht der Monomerzusammensetzung M), enthalten. Wenn die Monomerzusammensetzung M) wenigstens ein Monomer E) bis O) enthält, so jeweils vorzugsweise in einer Menge von 0,1 bis 50 Gew.-%, besonders bevorzugt 1 bis 25 Gew.-%, insbesondere 1,5 bis 10 Gew.-%, bezogen auf das Gesamtgewicht der Monomerzusammensetzung M). In einer speziellen Ausführung enthält die Monomerzusammensetzung M) keine weiteren Monomere E) bis O).

### Monomer E)

Bevorzugte Vinylaromaten E) sind Styrol, 2-Methylstyrol, 4-Methylstyrol, 2-(n-Butyl)styrol, 4-(n-Butyl)styrol, 4-(n-Decyl)styrol und Mischungen davon. Besonders bevorzugt sind Styrol und 2-Methylstyrol, insbesondere Styrol.

### Monomer F)

Bevorzugte Monomere F) sind Ethen, Propen, Buten, Isobuten, Diisobuten, Isopren, 1,3-Butadien und Mischungen davon.

### Monomer G)

Geeignete Ester α,β-ethylenisch ungesättigter Mono- und Dicarbonsäuren mit C₁-C₃₀-Alkanolen sind z. B. Methyl(meth)acrylat, Methylethacrylat, Ethyl(meth)acrylat, Ethylethacrylat, n-Propyl(meth)acrylat, Isopropyl(meth)acrylat, n-Butyl(meth)acrylat, tert.-Butyl(meth)acrylat, tert.-Butylethacrylat, n-Pentyl(meth)acrylat, n-Hexyl(meth)acrylat, n-Heptyl(meth)acrylat, n-Octyl(meth)acrylat,1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

### Monomer H)

Bevorzugt handelt es sich bei den kationogenen und/oder kationischen Gruppen der Komponente H) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung oder durch Quaternisierung mit Säuren oder Alkylierungsmitteln erzeugen. Dazu zählen z. B. Carbonsäuren, wie Milchsäure, oder Mineralsäuren, wie Phosphorsäure, Schwefelsäure und Salzsäure, oder als Alkylierungsmittel C₁-C₄-Alkylhalogenide oder -sulfate, wie Ethylchlorid, Ethylbromid, Methylchlorid, Methylbromid, Dimethylsulfat und Diethylsulfat. Eine Protonierung oder Quaternisierung kann im Allgemeinen sowohl vor als auch nach der Polymerisation erfolgen.

Vorzugsweise ist die Komponente H) ausgewählt unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen, und Mischungen davon.

Bevorzugte Verbindungen H) sind die Ester von α,β-ethylenisch ungesättigten Mono- und Dicarbonsäuren mit Aminoalkoholen. Bevorzugte Aminoalkohole sind C₂-C₁₂-Aminoalkohole, welche am Aminstickstoff C₁-C₈-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden als Säurekomponente Acrylsäure, Methacrylsäure und deren Gemische eingesetzt.

Bevorzugte Monomere H) sind N-Methylaminoethyl(meth)acrylat, N-Ethylaminoethyl(meth)acrylat, N-(n-Propyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminomethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.

Geeignete Monomere H) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Bevorzugt als Monomere H) sind z. B. N-[tert.-Butylaminoethyl](meth)acrylamid, N-[2-(Dimethylamino)ethyl]acrylamid, N-[2-(Dimethylamino)ethyl]methacrylamid, N-[3-(Dimethylamino)propyl]acrylamid, N-[3-(Dimethylamino)propyl]methacrylamid, N-[4-(Dimethylamino)butyl]acrylamid, N-[4-(Dimethylamino)butyl]methacrylamid, N-[2-(Diethylamino)ethyl]acrylamid, N-[4-(Dimethylamino)cyclohexyl]acrylamid und N-[4-(Dimethylamino)cyclohexyl]methacrylamid.

In einer geeigneten Ausführungsform umfasst die Komponente H) als vinylsubstituierte heteroaromatische Verbindung wenigstens eine N-Vinylimidazol-Verbindung. In einer speziellen Ausführungsform ist die Komponente H) ausgewählt unter N-Vinylimidazol-Verbindungen und Mischungen, die wenigstens eine N-Vinylimidazol-Verbindung enthalten.

Geeignete N-Vinylimidazol-Verbindungen sind Verbindungen der Formel (III) 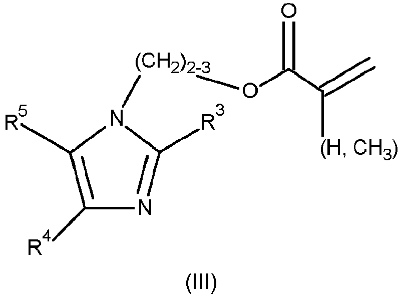 worin R³ bis R⁵ unabhängig voneinander für Wasserstoff, C₁-C₄-Alkyl oder Phenyl stehen. Bevorzugt stehen R¹ bis R³ für Wasserstoff.

Geeignet sind weiterhin N-Vinylimidazol-Verbindungen der allgemeinen Formel (IV) 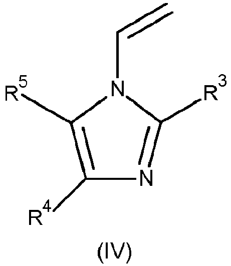 worin R³ bis R⁵ unabhängig voneinander für Wasserstoff, C₁-C₄-Alkyl oder Phenyl stehen.

Beispiele für Verbindungen der allgemeinen Formel (IV) sind folgender Tabelle zu entnehmen:

**Tabelle**

| R³ | R⁴ | R⁵ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Bevorzugt als Monomer H) ist 1-Vinylimidazol (N-Vinylimidazol) und sind Mischungen, die N-Vinylimidazol enthalten.

Geeignete Monomere H) sind auch die durch Protonierung oder Quaternisierung der zuvor genannten N-Vinylimidazolverbindungen erhältlichen Verbindungen. Beispiele für solche geladenen Monomere H) sind quaternisierte Vinylimidazole, insbesondere 3-Methyl-1-vinylimidazoliumchlorid, -methosulfat und -ethosulfat. Geeignete Säuren und Alkylierungsmittel sind die zuvor aufgeführten. Bevorzugt erfolgt eine Protonierung oder Quaternisierung nach der Polymerisation.

Geeignete Monomere H) sind weiterhin von Vinylimidazolen verschiedene vinyl- und allylsubstituierte Stickstoffheterocyclen, wie 2- und 4-Vinylpyridin, 2- und 4-Allylpyridin, und die Salze davon.

### Monomer I)

Geeignete Ester von Vinylalkohol mit C₁-C₃₀-Monocarbonsäuren sind z. B. Methylvinylester, Ethylvinylester, n-Propylvinylester, Isopropylvinylester, n-Butylvinylester,
tert.-Butylvinylester, n-Pentylvinylester, n-Hexylvinylester, n-Heptylvinylester, n-Octylvinylester, 1,1,3,3-Tetramethylbutylvinylester, Ethylhexylvinylester, n-Nonylvinylester, n-Decylvinylester, n-Undecylvinylester, Tridecylvinylester, Myristylvinylester, Pentadecylvinylester, Palmitylvinylester, Heptadecylvinylester, Octadecylvinylester, Nonadecylvinylester, Arrachinylvinylester, Behenylvinylester, Lignocerenylvinylester, Cerotinylvinylester, Melissinylvinylester, Palmitoleinylvinylester, Oleylvinylester, Linolylvinylester, Linolenylvinylester, Stearylvinylester, Laurylvinylester und Mischungen davon.

### Monomer K)

Geeignete amidgruppenhaltige Monomere K) sind Verbindungen der allgemeinen Formel (V) 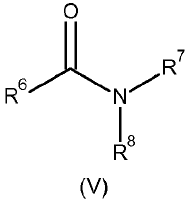 wobei
einer der Reste R⁶ bis R⁸ für eine Gruppe der Formel CH₂=CR⁹- mit R⁹ = H oder C₁-C₄-Alkyl steht und die übrigen Reste R⁶ bis R⁸ unabhängig voneinander für H, Alkyl, Cycloalkyl, Heterocycloalkyl, Aryl oder Hetaryl stehen,
wobei R⁶ und R⁷ gemeinsam mit der Amidgruppe, an die sie gebunden sind, auch für ein Lactam mit 5 bis 8 Ringatomen stehen können,
wobei R⁷ und R⁸ gemeinsam mit dem Stickstoffatom, an das sie gebunden sind, auch für einen fünf- bis siebengliedrigen Heterocyclus stehen können.

Bevorzugt sind die Verbindungen der Komponente K) ausgewählt unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

Bevorzugte Monomere K) sind N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl, etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Besonders bevorzugt werden N-Vinylpyrrolidon und/oder N-Vinylcaprolactam eingesetzt.

Geeignete Monomere K) sind weiterhin Acrylsäureamid und Methacrylsäureamid.

Geeignete N-Alkyl- und N,N-Dialkylamide α,β-ethylenisch ungesättigter Monocarbonsäuren sind beispielsweise Methyl(meth)acrylamid, Methylethacrylamid, Ethyl(meth)acrylamid, Ethylethacrylamid, n-Propyl(meth)acrylamid, Isopropyl(meth)-acrylamid, n-Butyl(meth)acrylamid, tert.-Butyl(meth)acrylamid, tert.-Butylethacrylamid, n-Pentyl(meth)acrylamid, n-Hexyl(meth)acrylamid, n-Heptyl(meth)acrylamid, n-Octyl(meth)acrylamid,1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid, N-Methyl-N-(n-octyl)(meth)acrylamid, N,N-Di-(n-octyl)(meth)acrylamid und Mischungen davon.

Als Monomere K) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon. Bevorzugt wird N-Vinylformamid eingesetzt.

### Monomer L)

Geeignete Ester α,β-ethylenisch ungesättigter Mono- und Dicarbonsäuren mit C₂-C₃₀-Alkandiolen sind 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat, etc.

Geeignete Amide α,β-ethylenisch ungesättigter Mono- und Dicarbonsäuren mit C₂-C₃₀-Aminoalkoholen mit einer primären oder sekundären Aminogruppe sind 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

### Monomer M)

Geeignete α,β-ethylenisch ungesättigte Nitrile sind Acrylnitril oder Methacrylnitril.

### Monomer N)

Geeignete Vinylhalogenide und Vinylidenhalogeniden sind Vinylchlorid, Vinylidenchlorid, Vinylfluorid, Vinylidenfluorid und Mischungen davon.

### Monomer O)

Geeignete Monomere O) mit Harnstoffgruppen sind N-vinylharnstoff, N-Allylharnstoff oder Derivative von Imidazolidin-2-on. Diese umfassen N-Vinyl- und N-Allylimidazolidin-2-on, N-Vinyloxyethylimidazolidin-2-on, N-(2-(meth)acrylamidoethyl)imidazolidin-2-on, N-(2-(meth)acryloxyethyl)imidazolidin-2-on (d.h., 2-Ureido(meth)acrylat), N-[2-((meth)acryloxyacetamido)ethyl]imidazolidin-2-on, etc.

### Polyetherkomponente PE)

Geeignet als Polyetherkomponente PE) sind Polyetherole mit einem zahlenmittleren Molekulargewicht von wenigstens 200 g/mol und deren Mono- und Di-(C₁-C₆-alkylether), wobei, wenn die Polyetherkomponente PE) ein Polyetherol mit Propylenoxid-Wiederholungseinheiten oder einen Mono- oder Di-(C1-C6 -alkylether) eines Polyetherols mit Propylenoxid-Wiederholungseinheiten umfasst, der Anteil dieser Propylenoxid-Wiederholungseinheiten im Mittel höchstens 10 Einheiten pro Molekül beträgt und die Polyetherole PE Ethylenoxid-Homopolymere oder Ethylenoxid/Propylenoxid-Copolymere sind. Geeignete Polyetherole und deren Mono- und Di-(C₁-C₆-alkylether) können linear oder verzweigt, vorzugsweise linear sein. Geeignete Polyetherole und deren Mono- und Di-(C₁-C₆-alkylether)weisen im Allgemeinen ein zahlenmittleres Molekulargewicht im Bereich von etwa 200 bis 100000, bevorzugt 300 bis 50000, besonders bevorzugt 500 bis 40000, auf. Geeignete Polyetherole sind beispielsweise wasserlösliche oder wasserdispergierbare nichtionische Polymere, die Alkylenoxid-Wiederholungseinheiten aufweisen. Vorzugsweise beträgt der Anteil an Alkylenoxid-Wiederholungseinheiten mindestens 30 Gew.-%, bezogen auf das Gesamtgewicht der Verbindung. Die Polyetherkomponente PE) sind Ethylenoxid-Homopolymere und Ethylenoxid/Propylenoxid-Copolymere.

Hier beschrieben als Polyetherkomponente PE) sind weiterhin die Mono- und Di-(C₁-C₂-alkylether) der zuvor beschriebenen Polyetherole. Bevorzugt sind Polyalkylenglycolmonomethylether und Polyalkylenglycoldimethylether.

Geeignet als Polyetherkomponente PE) sind weiterhin Polyethergruppen-haltige Tenside. Geeignet sind allgemein nichtionische und ionische Tenside, die wenigstens eine unpolare und wenigstens eine polare Gruppe aufweisen und die eine Polyethergruppe umfassen.

Die Polyethergruppen-haltigen Tenside PE) sind vorzugsweise ausgewählt unter Alkylpolyoxyalkylenether, Arylpolyoxyalkylenether, Alkylarylpolyoxyalkylenether, alkoxylierte tierische und/oder pflanzliche Fette und/oder Öle, Fettaminalkoxylate, Fettsäureamidalkoxylate, Fettsäurediethanolamidalkoxylate, Polyoxyethylensorbitanfettsäureester, Alkylpolyethersulfate, Arylpolyethersulfate, Alkylarylpolyethersulfate, Alkylpolyethersulfonate, Arylpolyethersulfonate, Alkylarylpolyethersulfonate, Alkylpolyetherphosphateate, Arylpolyetherphosphate, Alkylarylpolyetherphosphate, Glycerinethersulfonate, Glycerinethersulfate, Monoglycerid(ether)sulfate, Fettsäureamidethersulfate, Polyoxyalkylensorbitanfettsäureestern und Mischungen davon.

Zu den bevorzugten nichtionischen Tensiden Polyethergruppen-haltigen Tenside PE) gehören beispielsweise:
- Alkylpolyoxyalkylenether, die sich von niedermolekularen C₃-C₆-Alkoholen oder von C₇-C₃₀-Fettalkoholen ableiten. Dabei kann die Etherkomponente aus Ethylenoxideinheiten, Propylenoxideinheiten, 1,2-Butylenoxideinheiten, 1,4-Butylenoxideinheiten und statistischen Copolymeren und Blockcopolymeren davon abgeleitet sein. Geeignete nichtionische Tenside umfassen unter anderem Tenside der allgemeinen Formel (VI)

   R¹⁰-O-(CH₂CH₂O)ₓ-(CHR¹¹CH₂O)_{y}-R¹² (VI),

   worin R¹⁰ ein linearer oder verzweigter Alkylrest mit 6 bis 22 C-Atomen ist,
   R¹¹ und R¹² unabhängig voneinander Wasserstoff oder ein linearer oder verzweigter Alkylrest mit 1 bis 10 C-Atomen oder H sind, wobei R¹² bevorzugt Methyl ist, und
   x und y unabhängig voneinander 0 bis 300 sind. Bevorzugt ist x = 1 bis 100 und y = 0 bis 30.
   Dazu zählen speziell auch Fettalkoholalkoxylate und Oxoalkoholalkoxylate, wie iso-Tridecylalkohol- und Oleylalkoholpolyoxyethylenether.
- Hydroxylgruppen-haltige Tenside der allgemeinen Formel (VII)

   R¹³-O-(CH₂CH₂O)ₛ-(CH₂CH₂CH₂O)ₜ-(CH₂CH₂CH₂CH₂O)ᵤ-(CH₂CHR¹⁴O)ᵥ-CH₂CH(OH)R¹⁵ (VII)

   wobei
   in den Verbindungen der Formel (VII) die Reihenfolge der Alkylenoxideinheiten beliebig ist,
   s, t, u und v unabhängig voneinander für eine ganze Zahl von 0 bis 500 stehen, wobei die Summe aus s, t, u und v > 0 ist,
   R¹³ und R¹⁵ unabhängig voneinander für einen geradkettigen oder verzweigten, gesättigten C₁-C₄₀-Alkylrest oder einen ein- oder mehrfach ungesättigten C₂-C₄₀-Alkenylrest stehen, und
   R¹⁴ ausgewählt ist unter Methyl, Ethyl, n-Propyl, Isopropyl oder n-Butyl.

In den Verbindungen der allgemeinen Formel (VII) steht die Summe aus s, t, u und v vorzugsweise für einen Wert von 10 bis 300, besonders bevorzugt von 15 bis 200 und insbesondere von 20 bis 150.

Bevorzugt stehen t und u für 0. Dann steht die Summe aus s und v vorzugsweise für einen Wert von 10 bis 300, besonders bevorzugt von 15 bis 200 und insbesondere von 20 bis 150.

In den Verbindungen der allgemeinen Formel (VII) stehen vorzugsweise R¹³ und R¹⁵ unabhängig voneinander für einen geradkettigen oder verzweigten, gesättigten C₂-C₃₀-Alkylrest. Dabei können R¹³ und R¹⁵ auch für Mischungen verschiedener Alkylreste stehen.

In den Verbindungen der allgemeinen Formel (VII) steht R¹⁴ vorzugsweise für Methyl oder Ethyl, insbesondere für Methyl.

Eine bevorzugte Ausführung sind Hydroxylgruppen-haltige Tenside der allgemeinen Formel (VII.1)

R¹³-0-(CH₂CH₂0)ₛ-(CH₂CH(CH₃)O)ᵥ-CH₂CH(OH)R¹⁵ (VII.1)

wobei
die Reihenfolge der -(CH₂CH₂O)- und der (CH₂CH(CH₃)O)-Einheiten beliebig ist,
s und v unabhängig voneinander für eine ganze Zahl von 0 bis 500 stehen, wobei die Summe aus s und v > 0 ist, und
R¹³ und R¹⁵ unabhängig voneinander für einen geradkettigen, gesättigten C₁-C₃₀-Alkylrest oder einen verzweigten, gesättigten C₃-C₃₀-Alkylrest oder einen ein- oder mehrfach ungesättigten C₂-C₃₀-Alkenylrest stehen.

In den Verbindungen der allgemeinen Formel (VII.1) steht die Summe aus s und v vorzugsweise für einen Wert von 10 bis 300, besonders bevorzugt von 15 bis 200 und insbesondere von 20 bis 150.

Zur Gruppe dieser nichtionischen Tenside zählen beispielsweise Hydroxymischether der allgemeinen Formel (C₆₋₂₂-Alkyl)-CH(OH)CH₂O-(EO)₂₀₋₁₂₀-(C₂₋₂₆-Alkyl).
- Alkoholpolyoxyalkylenester der allgemeinen Formel (VIII)

   R¹⁶-O-(CH₂CH₂O)ₚ-(CH₂CHR¹⁷O)_{q}-C(=O)R¹⁸ (VIII)

   wobei
   in den Verbindungen der Formel (VIII) die Reihenfolge der Alkylenoxideinheiten beliebig ist,
   p und q unabhängig voneinander für eine ganze Zahl von 0 bis 500 stehen, wobei die Summe aus p und q > 0 ist,
   R¹⁶ und R¹⁸ unabhängig voneinander für einen geradkettigen oder verzweigten, gesättigten C₁-C₄₀-Alkylrest oder einen ein- oder mehrfach ungesättigten C₂-C₄₀-Alkenylrest stehen, und
   R¹⁷ ausgewählt ist unter Methyl, Ethyl, n-Propyl, Isopropyl oder n-Butyl.

In den Verbindungen der allgemeinen Formel (VIII) steht die Summe aus p und q vorzugsweise für einen Wert von 10 bis 300, besonders bevorzugt von 15 bis 200 und insbesondere von 20 bis 150.

In den Verbindungen der allgemeinen Formel (VIII) stehen vorzugsweise R¹⁶ und R¹⁸ unabhängig voneinander für einen geradkettigen oder verzweigten, gesättigten C₄-C₃₀-Alkylrest. Dabei können R¹⁶ und R¹⁸ auch für Mischungen verschiedener Alkylreste stehen.

In den Verbindungen der allgemeinen Formel (VIII) steht R¹⁷ vorzugsweise für Methyl oder Ethyl, insbesondere für Methyl.

Dazu zählt beispielsweise Laurylalkoholpolyoxyethylenacetat.
- Alkylarylalkoholpolyoxyethylenether, z. B. Octylphenol-polyoxyethylenether,
- alkoxylierte tierische und/oder pflanzliche Fette und/oder Öle, beispielsweise Maisölethoxylate, Rizinusölethoxylate, Talgfettethoxylate,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Polyoxyalkylensorbitanfettsäureester.

Ein Beispiel für ein Alkylpolyethersulfat ist Natriumdodecylpoly(oxyethylen)sulfat (Natriumlaurylethersulfat, SLES). Ein bevorzugter kommerziell erhältlicher modifizierter Fettalkoholpolyglycolether ist ein beidseitig endständig CₓH₂ₓ₊₁/C_{y}H_{2y+1}-terminiertes Polyethylenoxid mit einer freien OH-Gruppe und x, y = 6 - 14.

### Lösungsmittel LM)

Die radikalische Polymerisation in Schritt b) kann in Gegenwart eines Lösungsmittels LM) erfolgen, das ausgewählt ist unter Wasser, C₁-C₆-Alkanolen, von PE) verschiedenen Polyolen, deren Mono- und Dialkylethern und Mischungen davon. Geeignete Polyole und deren Mono- und Dialkylether umfassen auch Alkylenglycolmono(C₁-C₄-alkyl)ether, Alkylenglycoldi(C₁-C₄-alkyl)ether, Oligoalkylenglycole mit einem zahlenmittleren Molekulargewicht von weniger als 200 g/mol und deren Mono(C₁-C₄-alkyl)ether und Di(C₁-C₄-alkyl)ether.

Das Lösungsmittel LM) ist vorzugsweise ausgewählt unter Wasser, Methanol, Ethanol, n-Propanol, Isopropanol, n-Butanol, Ethylenglycol, Ethylenglycolmono(C₁-C₄-alkyl)ethern, Ethylenglycoldi(C₁-C₄-alkyl)ethern, 1,2-Propylenglycol, 1,2-Propylenglycolmono(C₁-C₄-alkyl)ethern, 1,2-Propylenglycoldi(C₁-C₄-alkyl)ethern, Glycerin, Polyglycerinen, Oligoalkylenglycolen mit einem zahlenmittleren Molekulargewicht von weniger als 200 g/mol und Mischungen davon.

Geeignete Oligoethylenglycole sind unter den CTFA-Bezeichnungen PEG-6, PEG-8, PEG-12, PEG-6-32, PEG-20, PEG-150, PEG-7M, PEG-12M and PEG-115M kommerziell erhältlich. Dazu zählen speziell die Pluriol E ® Marken der BASF SE. Geeignete Alkylpolyalkylenglycole sind die entsprechenden Pluriol A ...E ® Marken der BASF SE.

Das Lösungsmittel LM) ist besonders bevorzugt ausgewählt unter Wasser, Ethanol, n-Propanol, Isopropanol, Ethylenglycol, Diethylenglycol, 1,2-Propylenglycol, 1,2-Dipropylenglycol und Mischungen davon.

In einer speziellen Ausführungsform wird als Lösungsmittel LM) Wasser oder ein Gemisch aus Wasser und wenigstens einem von Wasser verschieden Lösungsmittel LM) eingesetzt, ausgewählt unter Ethanol, n-Propanol, Isopropanol, Ethylenglycol, Diethylenglycol, 1,2-Propylenglycol, 1,2-Dipropylenglycol und Mischungen davon.

In einer speziellen Ausführungsform erfolgt die radikalische Polymerisation in Schritt c) in Gegenwart eines Lösungsmittels LM), das zu wenigstens 50 Gew.-%, vorzugsweise zu wenigstens 75 Gew.-%, speziell zu wenigstens 90 Gew.-%, bezogen auf das Gesamtgewicht des Lösungsmittels LM), aus Wasser besteht. Insbesondere erfolgt die radikalische Polymerisation in Schritt c) in Gegenwart eines Lösungsmittels LM), dass vollständig aus Wasser besteht.

### Herstellung der erfindungsgemäßen Polymerzusammensetzungen

Die Herstellung der erfindungsgemäßen Polymerzusammensetzungen umfasst eine radikalische Polymerisation der Monomerzusammensetzung M) in Gegenwart wenigstens einer Polyetherkomponente PE). Sie wird vorzugsweise im Zulaufverfahren durchgeführt. Dabei werden im Allgemeinen zumindest die Monomere in flüssiger Form dem Reaktionsansatz zudosiert. Unter den Dosierbedingungen flüssige Monomere können dem Reaktionsansatz ohne Zugabe eines Lösungsmittels LM) zugeführt werden, ansonsten werden die Monomere als Lösung in einem geeigneten Lösungsmittel LM) eingesetzt.

Bevorzugt erfolgt die radikalische Polymerisation in Schritt b) in Zulauf-Fahrweise, wobei Zuläufe, die wenigstens eine α,β-ethylenisch ungesättigte Carbonsäure enthalten, kein Lösungsmittel LM) enthalten.

Die Dosierraten des Monomerzulaufs / der Monomerzuläufe sowie etwaiger weiterer Zuläufe (Initiator, Regler, etc.) werden vorzugsweise so gewählt, dass die Polymerisation mit der gewünschten Umsatzrate aufrechterhalten wird. Die Zugabe der einzelnen Zuläufe kann dabei kontinuierlich, periodisch, mit konstanter oder wechselnder Dosierrate, im Wesentlichen zeitgleich oder zeitversetzt erfolgen. Vorzugsweise erfolgt die Zugabe aller Zuläufe zum Reaktionsansatz kontinuierlich.

Das Gewichtsverhältnis der Monomermischung M) zur Komponente PE) liegt vorzugsweise im Bereich von 1 : 10 bis 10 : 1 liegt.

Sofern zur Herstellung der Polymerzusammensetzung ein Lösungsmittel LM) eingesetzt wird, liegt das Gewichtsverhältnis der Komponente PE) zur Komponente LM) vorzugsweise im Bereich von 0,3 : 1 bis 5 : 1, besonders bevorzugt von 0,5 : 1 bis 3 : 1.

Bevorzugt erfolgt die radikalische Polymerisation in Schritt b) bei einer Temperatur im Bereich von 20 bis 95°C, besonders bevorzugt von 30 bis 90°C, insbesondere von 40 bis 80°C.

Bevorzugt umfasst die radikalische Polymerisation in Schritt b) des erfindungsgemäßen Verfahrens
b1) Bereitstellung einer Vorlage, die wenigstens einen Teil der Polyetherkomponente PE), gegebenenfalls wenigstens einen Teil des Reglers R) und, falls die Polymerisation in Gegenwart eines Lösungsmittels LM) erfolgt, gegebenenfalls wenigstens einen Teil von LM) enthält;
b2) Zugabe der Monomerzusammensetzung M) in einem Zulauf oder in mehreren Zuläufe sowie Zugabe eines Zulaufs, der den Radikalstarter S), gelöst in einem Teil der wenigstens einen Polyetherkomponente PE) und/oder des Lösungsmittels LM) enthält und gegebenenfalls Zugabe eines Zulaufs der die Menge des Reglers R) enthält, die nicht in der Vorlage eingesetzt wird,
b3) gegebenenfalls Nachpolymerisierung der in Schritt b2) erhaltenen Reaktionsmischung.

Üblicherweise wird die Vorlage vor der Zugabe der Zuläufe unter Rühren auf die Polymerisationstemperatur erwärmt.

Bevorzugt werden die einzelnen Reaktanden simultan in getrennten Zuläufen zugegeben, wobei die Flussraten der Zuläufe in der Regel über den Zeitraum der Zugabe möglichst konstant gehalten werden.

Die Zugabe der Zuläufe in Schritt b2) erfolgt über einen Zeitraum der in vorteilhafter Weise so gewählt wird, dass die bei der exothermen Polymerisationsreaktion entstehende Reaktionswärme ohne größeren technischen Aufwand, z.B. ohne die Verwendung eines Rückflusskühlers, abgeführt werden kann. Üblicherweise erfolgt die Zugabe der Zuläufe über einen Zeitraum von 1 bis 10 Stunden. Bevorzugt erfolgt die Zugabe der Zuläufe über einen Zeitraum von 2 bis 8 Stunden, besonders bevorzugt über 3 bis 5 Stunden.

Während der radikalischen Polymerisation werden in der Regel das optional eingesetzte Lösungsmittel und/oder etwaig entstehende Kondensationsprodukte nicht abgeführt. D. h. während der Polymerisation findet üblicherweise kein oder ein im Rahmen der technischen Möglichkeiten nur sehr geringer Stoffaustausch mit der Umgebung statt.

Die Polymerisation kann in der Regel bei Umgebungsdruck oder vermindertem oder erhöhtem Druck erfolgen. Bevorzugt wird die Polymerisation bei Umgebungsdruck durchgeführt.

Die Polymerisation erfolgt in der Regel bei konstanter Temperatur, kann aber auch bei Bedarf während der Polymerisation variiert werden. Bevorzugt wird die Polymerisationstemperatur über den gesamten Reaktionszeitraum, d.h. der Schritte b2) und b3), möglichst konstant gehalten. Je nachdem welche Einsatzstoffe im erfindungsgemäßen Verfahren eingesetzt werden, bewegt sich die Polymerisationstemperatur üblicherweise im Bereich von 20 bis 95 °C. Bevorzugt bewegt sich die Polymerisationstemperatur im Bereich von 30 bis 90 °C und insbesondere im Bereich von 40 bis 80 °C. Sofern die Polymerisation nicht unter erhöhtem Druck durchgeführt wird und der Reaktionsmischung wenigstens ein optionales Lösungsmittel LM) zugesetzt wurde, bestimmt das Lösungsmittel bzw. Lösungsmittelgemisch durch die entsprechenden Siedetemperaturen die maximale Reaktionstemperatur.

Die Polymerisation kann in Abwesenheit oder in Gegenwart eines Inertgases erfolgen. Üblicherweise wird die Polymerisation in Anwesenheit eines Inertgases durchgeführt. Unter Inertgas wird in der Regel ein Gas verstanden, welches unter den gegebenen Reaktionsbedingungen keine Reaktion mit den an den Reaktion beteiligten Edukten, Reagenzien, Lösungsmitteln oder den entstehenden Produkten eingeht.

Falls die Polymerisation in Gegenwart eines Lösungsmittels durchgeführt wird, so ist dieses ausgewählt unter den zuvor beschriebenen Lösungsmitteln LM).

Zur Herstellung der Polymerisate können die Monomeren mit Hilfe von Radikale bildenden Initiatoren, im Nachfolgenden auch als Radikalstarter oder Starter bezeichnet, polymerisiert werden. Als Radikalstarter (Initiatoren) für die radikalische Polymerisation kommen grundsätzlich alle Radikalstarter in Frage, die im Reaktionsmedium, wie es zum Zeitpunkt ihrer Zugabe vorherrscht, im Wesentlichen löslich sind und bei den gegebenen Reaktionstemperaturen eine ausreichende Aktivität besitzen, um die Polymerisation zu starten. In das erfindungsgemäße Verfahren kann ein einzelner Radikalstarter oder eine Kombination wenigstens zweier Radikalstarter eingesetzt werden. Im letzteren Fall können die wenigstens zwei Radikalstarter im Gemisch oder vorzugsweise getrennt, gleichzeitig oder nacheinander, z.B. zu unterschiedlichen Zeitpunkten im Reaktionsverlauf, eingesetzt werden.

Als Radikalstarter für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, tert.-Butylperoctoat, Dilauroylperoxid, tert.-Butylperisobutyrat, tert.-Butylperacetat, Di-tert.-amylperoxid, tert.-Butylhydroperoxid, 2,2'-Azo-bis-isobutyronitril, Azo-bis-(2-amidinopropan)dihydrochlorid, Azobis(2,4-dimethylvaleronitril) oder 2,2'-Azo-bis-(2-methyl-butyronitril).

Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat,

H₂O₂/Cu^{I}.

In dem erfindungsgemäßen Verfahren bewegt sich die Menge an eingesetztem Initiatorsystem (Starter) im Bereich von 0,01 bis 10 Gew.-%, bevorzugt im Bereich von 0,1 bis 5 Gew.-%, besonders bevorzugt im Bereich von 0,2 bis 2 Gew.-% und insbesondere im Bereich von 0,3 bis 1,5 Gew.-%.

Im erfindungsgemäßen Verfahren wird der Radikalstarter in der Regel als Lösung in einem Lösungsmittel bereitgestellt, das wenigstens eines der zuvor genannten Lösungsmittel LM) umfasst.

Die Menge an Regler, die üblicherweise in dem erfindungsgemäßen Verfahren eingesetzt wird, beträgt 1 bis 40 pphm ("parts per hundred monomer", d.h. Gewichtsteile bezogen auf hundert Gewichtsteile Monomerzusammensetzung). Bevorzugt liegt die in dem erfindungsgemäßen Verfahren eingesetzte Menge an Regler im Bereich von 3 bis 30 pphm, besonders bevorzugt im Bereich von 5 bis 25 pphm.

Üblicherweise wird der Regler in Schritt b2) vollständig über einen der Zuläufe kontinuierlich zur Polymerisationsmischung zugegeben. Es ist jedoch auch möglich, den Regler entweder vollständig zur Vorlage, d.h. vor der eigentlichen Polymerisation, zuzugeben oder es wird nur ein Teil des Reglers in der Vorlage vorgelegt und der Rest in Schritt b2) über einen der Zuläufe kontinuierlich zur Polymerisationsmischung zugegeben. Die Zugabe des Reglers kann dabei jeweils ohne oder mit Lösungsmittel LM) erfolgen.

Die Menge des Reglers und die Art wie dieser zur Reaktionsmischung zugegeben wird, haben einen starken Einfluss auf das mittlere Molekulargewicht der Polymerzusammensetzung. Wenn weniger Regler eingesetzt wird und/oder wenn die Zugabe überwiegend vor der Polymerisation erfolgt, führt dies in der Regel zu höheren mittleren Molekulargewichten des gebildeten Polymers. Werden hingegen größere Menge Regler verwendet und/oder findet die Zugabe des Reglers größtenteils während der Polymerisation statt (Schritt b2)), führt dies in der Regel zu einem kleineren mittleren Molekulargewicht.

### 1. Variante: Herstellung einer Polymerzusammensetzung für Folien

Das erfindungsgemäße Verfahren dient in einer ersten bevorzugten Ausführungsform zur Herstellung einer Polymerzusammensetzung, die sich zur Bereitstellung von Folien, insbesondere von transparenten Folien eignet.

In der 1. Variante beträgt die Menge an Polyetherkomponente PE) in der Vorlage (Schritt b1)) vorzugsweise 30 bis 100 Gew.-%, besonders bevorzugt 65 bis 100 Gew.-% und insbesondere 80 bis 100 Gew.-% bezogen auf das Gesamtgewicht der zur Polymerisation eingesetzten Polyetherkomponente PE).

In der 1. Variante liegt das Mengenverhältnis der zur Herstellung der Polymerzusammensetzung verwendeten Polyetherkomponente PE) zur eingesetzten Monomerzusammensetzung M) üblicherweise im Bereich von 1,0 : 0,8 bis 1,0 : 5, bevorzugt im Bereich von 1,0 : 1,0 bis 1,0 : 2,5.

In dieser Ausführungsform liegt der Gehalt an Lösungsmittel im Vorlauf üblicherweise bei maximal 70 Gew.-%, bezogen auf das Gesamtgewicht der im Vorlauf befindlichen Einsatzstoffe. Bevorzugt liegt der Gehalt an Lösungsmittel im Vorlauf bei maximal 40 Gew.-%, insbesondere bei maximal 35 Gew.-%, bezogen auf das Gesamtgewicht der im Vorlauf befindlichen Einsatzstoffe. Die Lösungsmittelmenge ändert sich über den gesamten Verlauf des Verfahrens in der Regel nur um wenige Gewichtsprozente. Üblicherweise werden Lösungsmittel LM) verwendet, die einen Siedepunkt bei Normaldruck (1 bar) von unter 240°C aufweisen. In der Regel wird als Lösungsmittel LM in dieser ersten bevorzugten Ausführungsform Wasser, Ethanol, n-Propanol, Isopropanol, n-Butanol, Ethylenglycol, Ethylenglycolmono(C₁-C₄-alkyl)ethern, Ethylenglycoldi(C₁-C₄-alkyl)ethern, 1,2-Propylenglycol, 1,2-Propylenglycolmono(C₁-C₄-alkyl)ethern, 1,2-Propylenglycoldi(C₁-C₄-alkyl)ethern, Dipropylenglycoleoder Mischungen davon verwendet. Bevorzugt wird als Lösungsmittel LM) in dieser ersten bevorzugten Ausführungsform Wasser, Ethanol, n-Propanol, Isopropanol, n-Butanol oder Mischungen davon verwendet.

In einer speziellen Variante dieser Ausführungsform enthält die Vorlage kein Lösungsmittel. Dieses wird erst in Schritt b2) über wenigstens einen der Zuläufe zugegeben. In einer ganz speziellen Variante dieser Ausführungsform wird kein Lösungsmittel vorgelegt sowie über den gesamten Verlauf des Verfahrens kein Lösungsmittel zugegeben.

In dieser ersten bevorzugten Ausführungsform werden die nach Beendigung der Polymerisation (Schritt b3)) erhaltenen Polymerzusammensetzungen in ein geeignetes Gefäß transferiert und gegebenenfalls direkt auf Umgebungstemperatur (20 °C) abgekühlt.

Die auf diese Weise erhaltenen Polymerzusammensetzungen besitzen eine feste Konsistenz und sind transparent, d.h. sie sind lichtdurchlässig und weisen eine Lichtdurchlässigkeit (T_{L}-Wert) von mindestens 85 %, insbesondere von mindestens 90 %, bezogen auf die Lichtdurchlässigkeit von Wasser, auf. Sie eignen sich zur Herstellung von Folien, z.B. als Umhüllung eines flüssigen Spül-, Wasch- oder Reinigungsmittels. Die Herstellung von Folien und von Umhüllungen auf deren Basis wird im Folgenden noch näher beschrieben.

### 2. Variante: Herstellung einer Polymerzusammensetzung für Beschichtungen

Das erfindungsgemäße Verfahren dient in einer zweiten bevorzugten Ausführungsform zur Herstellung einer Polymerzusammensetzung, die sich zur Bereitstellung_von festen Beschichtungen auf einem Substrat eignen.

In der 2. Variante beträgt die Menge an Polyetherkomponente PE) in der Vorlage (Schritt b1)) vorzugsweise 30 bis 100 Gew.-%, besonders bevorzugt 65 bis 100 Gew.-% und insbesondere 80 bis 100 Gew.-% bezogen auf das Gesamtgewicht der zur Polymerisation eingesetzten Polyetherkomponente PE).

In der 2. Variante liegt das Mengenverhältnis der zur Herstellung der Polymerzusammensetzung verwendeten Polyetherkomponente PE) zur eingesetzten Monomerzusammensetzung M) üblicherweise im Bereich von 1,0 : 0,8 bis 1,0 : 5, bevorzugt im Bereich von 1,0 : 1,0 bis 1,0 : 2,5.

In dieser Ausführungsform liegt der Gehalt an Lösungsmittel im Vorlauf üblicherweise bei maximal 70 Gew.-%, bezogen auf das Gesamtgewicht der im Vorlauf befindlichen Einsatzstoffe. Bevorzugt liegt der Gehalt an Lösungsmittel im Vorlauf bei maximal 40 Gew.-%, insbesondere bei maximal 35 Gew.-%, bezogen auf das Gesamtgewicht der im Vorlauf befindlichen Einsatzstoffe. Die Lösungsmittelmenge ändert sich über den gesamten Verlauf des Verfahrens in der Regel nur um wenige Gewichtsprozente. Üblicherweise werden Lösungsmittel LM) verwendet, die einen Siedepunkt bei Normaldruck (1 bar) von unter 240°C aufweisen. In der Regel wird als Lösungsmittel LM in dieser ersten bevorzugten Ausführungsform Wasser, Ethanol, n-Propanol, Isopropanol, n-Butanol, Ethylenglycol, Ethylenglycolmono(C₁-C₄-alkyl)ethern, Ethylenglycoldi(C₁-C₄-alkyl)ethern, 1,2-Propylenglycol, 1,2-Propylenglycolmono(C₁-C₄-alkyl)ethern, 1,2-Propylenglycoldi(C₁-C₄-alkyl)ethern, Dipropylenglycoleoder Mischungen davon verwendet. Bevorzugt wird als Lösungsmittel LM) in dieser ersten bevorzugten Ausführungsform Wasser, Ethanol, n-Propanol, Isopropanol, n-Butanol oder Mischungen davon verwendet.

In einer speziellen Variante dieser Ausführungsform enthält die Vorlage kein Lösungsmittel. Dieses wird erst in Schritt b2) über wenigstens einen der Zuläufe zugegeben. In einer ganz speziellen Variante dieser Ausführungsform wird kein Lösungsmittel vorgelegt sowie über den gesamten Verlauf des Verfahrens kein Lösungsmittel zugegeben.

In dieser ersten bevorzugten Ausführungsform werden die nach Beendigung der Polymerisation (Schritt b3)) erhaltenen Polymerzusammensetzungen in ein geeignetes Gefäß transferiert und gegebenenfalls direkt auf Umgebungstemperatur (20 °C) abgekühlt.

Die auf diese Weise erhaltenen Polymerzusammensetzungen eignen sich zur Herstellung von festen Beschichtungen auf einem Substrat, z.B. auf einer Spül-, Wasch- oder Reinigungsmitteltablette. Die Herstellung von solchen Beschichtungen wird im Folgenden noch näher beschrieben.

### 3. Variante: Herstellung fester Zusammensetzungen

Das erfindungsgemäße Verfahren dient in einer dritten bevorzugten Ausführungsform zur Bereitstellung fester, speziell teilchenförmiger Polymerzusammensetzungen.

In dieser dritten bevorzugten Ausführungsform des erfindungsgemäßen Verfahrens beträgt die Menge an Polyetherkomponente PE) in der Vorlage (Schritt b1)) üblicherweise 40 bis 100 Gew.-%, bevorzugt 90 bis 100 Gew.-% und insbesondere 95 bis 100 Gew.-% bezogen auf das Gesamtgewicht der zur Polymerisation eingesetzten Polyetherkomponente PE). Speziell wird die Polyetherkomponente PE) vor der Polymerisation vollständig vorgelegt.

In dieser dritten bevorzugten Ausführungsform liegt der Gehalt an Lösungsmittel in der Vorlage bei 25 bis 70 Gew.-%, bezogen auf das Gesamtgewicht aller im Vorlauf befindlichen Einsatzstoffe. Bevorzugt liegt der Gehalt an Lösungsmittel im Vorlauf bei 30 bis 65 Gew.-%, insbesondere bei 35 bis 60 Gew.-%, bezogen auf das Gesamtgewicht aller im Vorlauf befindlichen Einsatzstoffe. Bevorzugt wird als Lösungsmittel LM) Wasser oder C₁-C₆-Alkanole verwendet, besonders bevorzugt sind Wasser, Methanol, Ethanol, n-Propanol, Isopropanol, n-Butanol, insbesondere Wasser oder Isopropanol sowie Mischungen davon.

Das Mengenverhältnis der zur Herstellung der Polymerzusammensetzung verwendeten Polyetherkomponente PE) zur eingesetzten Monomerzusammensetzung M) liegt in dieser zweiten Ausführungsform üblicherweise im Bereich von 1 : 1 bis 1 : 10.

In dieser dritten bevorzugten Ausführungsform werden die nach Beendigung der Polymerisation (Schritt b3)) erhaltenen Polymerzusammensetzungen in ein geeignetes Gefäß transferiert und gegebenenfalls direkt auf Umgebungstemperatur (20 °C) abgekühlt. In der Regel werden die in der zweiten bevorzugten Ausführungsform erhaltenen Polymerzusammensetzungen umgehend einem Trocknungs- und/oder Zerkleinerungsverfahren, wie nachfolgend beschrieben, zugeführt.

Die nach dem erfindungsgemäßen Verfahren hergestellten Polymerzusammensetzungen können nach der Polymerisation weiteren Nachbearbeitungsschritten unterzogen werden. Dazu zählen beispielsweise Schmelzverfahren, Formgebungsverfahren, Trocknungsverfahren, Zerkleinerungsverfahren, Reinigungsverfahren oder Kombinationen davon.

In einer geeigneten Ausführungsform wird das in Schritt b) erhaltene Reaktionsprodukt einer Formgebung unterzogen. Bevorzugt wird dabei ein teilchenförmiges Produkt erhalten. Die Polymerzusammensetzung kann in einer Teilchengröße bereitgestellt werden, die von pulverförmigen Partikeln bis Granulatgröße reicht. Eine bevorzugte Ausführungsform ist eine teilchenförmige Polymerzusammensetzung mit einer Teilchengröße in einem Bereich von etwa 100 µm bis 10 mm.

Vorzugsweise umfasst die Formgebung des Präpolymers eine Plastifizierung, z.B. durch Extrusion, und eine Granulierung, Walzenformung und/oder eine Mahlung. Geeignete Verfahren zur Granulierung, Walzenformung und Mahlung von Polymeren sind dem Fachmann bekannt. In einer geeigneten Ausführung wird die Polymerzusammensetzung zur Formgebung zuerst zu einem oder mehreren Strängen geformt. Dazu können dem Fachmann bekannte Vorrichtungen verwendet werden. Geeignete Vorrichtungen sind z. B. Lochplatten, Düsen oder Düsenplatten. In einer geeigneten Ausführungsform wird die zu Strängen geformte Polymerzusammensetzung in fließfähiger Form einer Zerkleinerung zu Polymerteilchen unterzogen. In einer alternativen Ausführungsform wird die zu Strängen geformte Polymerzusammensetzung verfestigt und anschließend einer Zerkleinerung zu Polymerteilchen unterzogen. Geeignete Mühlen zur Mahlung der Präpolymere sind z. B. Hammermühlen, Walzenstühle, Kugelmühlen, etc.

In einer ersten bevorzugen Nachbearbeitungsvariante werden die erfindungsgemäßen transparenten Polymerzusammensetzungen zunächst durch Erwärmen in einen fließfähigen Zustand versetzt. Diese können dann in einem weiteren Schritt verschiedensten Formgebungsverfahren unterzogen werden, wie beispielsweise das Ausstreichen auf einem festes Trägermaterial zur Herstellung transparenter Folien oder das Gießen von Hüllen, Hülsen oder anderer zur Befüllung geeigneter Hohlkörper. Nach der Formgebung werden die Folien oder Hohlkörper üblicherweise bei einer Temperatur von 50 bis 110 °C über einen Zeitraum von 12 bis 72 Stunden getrocknet.

In einer zweiten bevorzugten Nachbearbeitungsvariante wird das in den erfindungsgemäßen Polymerzusammensetzungen optional enthaltene Lösungsmittel unter reduziertem Druck abdestilliert. Der so erhaltene polymere Feststoff wird anschließend zu einem Granulat zerkleinert oder durch Mörsern in ein Pulver überführt. In dieser Nachbearbeitungsvariante können die erfindungsgemäßen Polymerzusammensetzungen auch direkt im Anschluss an die Polymerisation und Abkühlung auf Raumtemperatur zu einem Granulat oder Pulver verarbeitet werden, falls diese eine feste Konsistenz aufweisen. In der Regel eignen sich die erfindungsgemäßen Polymerzusammensetzungen der zweiten bevorzugten Ausführungsform des erfindungsgemäßen Verfahrens in vorteilhafter Weise für diese Nachbearbeitungsvariante. Aus ihnen lassen sich in der Regel feste, rieselfähige Pulver und/oder Granulate gewinnen.

Die Säuregruppen der erfindungsgemäßen Polymerzusammensetzung können nicht, teilweise oder vollständig neutralisiert werden. Bevorzugt werden die Säuregruppen der erfindungsgemäßen Polymerzusammensetzung nicht oder nur teilweise neutralisiert.

Als Base für die Neutralisation können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Ammoniak oder Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin. Bevorzugt sind Aminoalkohole, z. B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Die Neutralisation der Säuregruppen kann auch mit Hilfe von Mischungen mehrerer Basen vorgenommen werden. Besonders bevorzugt ist die Base ausgewählt unter NaOH, KOH, 2-Amino-2-methyl-1-propanol, Triethylamin, Diethylaminopropylamin, Diethanolamin, Triethanolamin, Triisopropanolamin und Mischungen davon.

### Charakterisierung der Polymerzusammensetzung

Die erfindungsgemäße Polymerzusammensetzung weist vorzugsweise einen Gehalt an Säuregruppen von mehr als 1 mmol/g, besonders bevorzugt von mehr als 1.3 mmol/g auf. Die erfindungsgemäße Polymerzusammensetzung weist vorzugsweise einen Gehalt an Säuregruppen von höchstens 15 mmol/g auf. Die erfindungsgemäße Polymerzusammensetzung weist insbesondere einen Gehalt an Säuregruppen von 1.5 mmol/g bis 15 mmol/g auf.

Eine Polymerzusammensetzung in Form einer Festsubstanz weist vorzugsweise einen Gehalt an Säuregruppen von 1,5 mmol/g bis 15 mmol/g auf.

In einer ersten bevorzugten Ausführungsform liegen bis zu 80 Mol-% der Säuregruppen, besonders bevorzugt bis zu 70 Mol-% der Säuregruppen, insbesondere bis zu 20 Mol-% der Säuregruppen der erfindungsgemäßen Polymerzusammensetzung neutralisiert vor.

In einer zweiten bevorzugten Ausführungsform liegen die Säuregruppen der erfindungsgemäßen Polymerzusammensetzung in nicht neutralisierter Form vor.

Vorzugsweise weist die Polymerzusammensetzung eine Löslichkeit in Wasser bei 40 °C und einem pH-Wert von 8 von mindesten 5 g/L auf.

Das gewichtsmittlere Molekulargewicht M_{w} der erfindungsgemäßen Polymerzusammensetzung, bestimmt mittels Gelpermeationschromatographie (GPC) unter Verwendung von neutralisierter Polyacrylsäure als Polymerstandard beträgt vorzugsweise 1000 bis 150000 Dalton.

### Tensidhaltige Zusammensetzung

Die erfindungsgemäße Polymerzusammensetzung eignet sich besonders vorteilhaft zur Formulierung tensidhaltiger Zusammensetzungen. Insbesondere handelt es sich dabei um wässrige tensidhaltige Zusammensetzungen. Die erfindungsgemäße Polymerzusammensetzung zeichnet sich durch ihre gute Verträglichkeit mit einer Vielzahl Tensiden aus.

Tensidhaltigen Zusammensetzungen, die die erfindungsgemäße Polymerzusammensetzung enthalten, weisen vorzugsweise einen Gesamttensidgehalt von 0,1 bis 75 Gew.-%, besonders bevorzugt von 0,5 bis 60 Gew.-%, insbesondere von 1 bis 50 Gew.-%, bezogen auf das Gesamtgewicht der tensidhaltigen Zusammensetzung, auf.

Geeignete zusätzliche Tenside sind anionische Tenside, nichtionische Tenside, kationische Tenside, amphotere Tenside und Mischungen davon.

Typische Beispiele für anionische Tenside sind Seifen, Alkylsulfonate, Alkylbenzolsulfonate, Olefinsulfonate, Methylestersulfonate, Sulfofettsäuren, Alkylsulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkylglucosecarboxylate, Proteinfettsäurekondensate und Alkyl(ether)phosphate.

Geeignete Seifen sind z. B. Alkali-, Erdalkali- und Ammoniumsalze von Fettsäuren, wie Kaliumstearat.

Geeignete Olefinsulfonate werden z. B. durch Anlagerung von SO₃ an Olefine der Formel R¹⁵-CH=CH-R¹⁶ und nachfolgende Hydrolyse und Neutralisation erhalten, wobei R¹⁵ und R¹⁶ unabhängig voneinander für H oder Alkylreste mit 1 bis 20 Kohlenstoffatomen stehen, mit der Maßgabe, dass R¹⁵ und R¹⁶ zusammen mindestens 6 und vorzugsweise 8 bis 20, speziell 10 bis 16, Kohlenstoffatome aufweisen. Hinsichtlich Herstellung und Verwendung sei auf den Übersichtsartikel "J. Am. Oil. Chem. Soc.", 55, 70 (1978) verwiesen. Die Olefinsulfonate können als Alkali-, Erdalkali-, Ammonium-, Alkylammonium-, Alkanolammonium- oder Glucammoniumsalze vorliegen. Vorzugsweise liegen die Olefinsulfonate als Natriumsalze vor. Das hydrolysierte alpha-Olefinsulfonierungsprodukt, d. h. die alpha-Olefinsulfonate, setzen sich aus ca. 60 Gew.-% Alkansulfonaten und ca. 40 Gew.-% Hydroxyalkansulfonaten zusammen; hiervon sind etwa 80 bis 85 Gew.-% Mono- und 15 bis 20 Gew.-% Disulfonate.

Bevorzugte Methylestersulfonate (MES) werden durch Sulfonierung der Fettsäuremethylester von pflanzlichen oder tierischen Fetten oder Ölen gewonnen. Bevorzugt sind Methylestersulfonate aus pflanzlichen Fetten und Ölen, z. B. aus Rapsöl, Sonnenblumenöl, Sojaöl, Palmöl, Kokosfett, etc.

Bevorzugte Alkylsulfate sind Sulfate von Fettalkoholen der allgemeinen Formel R¹⁷-O-SO₃Y, worin R¹⁷ für einen linearen oder verzweigten, gesättigten oder ungesättigten Kohlenwasserstoffrest mit 6 bis 22 Kohlenstoffatomen und Y für ein Alkalimetall, das einwertige Ladungsäquivalent eines Erdalkalimetalls, Ammonium-, Mono-, Di-, Tri- oder Tetraalkylammonium, Alkanolammonium oder Glucammonium, steht. Geeignete Fettalkoholsulfate werden vorzugsweise durch Sulfatierung von nativen Fettalkoholen oder synthetischen Oxoalkoholen und nachfolgender Neutralisation erhalten werden. Typische Beispiele für Fettalkoholsulfate sind die Sulfatierungsprodukte des Capronalkohols, Caprylalkohols, 2-Ethylhexylalkohols, Caprinalkohols, Laurylalkohols, Isotridecylalkohols, Myristylalkohols, Cetylalkohols, Palmoleylalkohols, Stearylalkohols, Isostearylalkohols, Oleylalkohols, Elaidylalkohols, Petroselinylalkohols, Linolylalkohols, Linolenylalkohols, Behenylalkohols und Elaeostearylalkohols sowie die Salze und Mischungen davon. Bevorzugte Salze der Fettalkoholsulfate sind die Natrium- und Kaliumsalze, insbesondere die Natriumsalze. Bevorzugte Mischungen der Fettalkoholsulfate basieren auf technischen Alkoholmischungen, die z. B. bei der Hochdruckhydrierung von technischen Methylestern auf Basis von Fetten und Ölen oder bei der Hydrierung von Aldehyden aus der Oxosynthese oder bei der Dimerisierung von ungesättigten Fettalkoholen anfallen. Vorzugsweise werden zur Herstellung von Alkylsulfaten Fettalkohole und Fettalkoholgemische mit 12 bis 18 Kohlenstoffatomen und insbesondere 16 bis 18 Kohlenstoffatomen eingesetzt. Typische Beispiele hierfür sind technische Alkoholsulfate auf Basis von pflanzlichen Rohstoffen.

Bevorzugte Sarkosinate sind Natriumlauroylsarkosinat oder Natriumstearoylsarkosinat.

Bevorzugte Proteinfettsäurekondensate sind pflanzliche Produkte auf Weizenbasis.

Bevorzugte Alkylphosphate sind Mono- und Diphosphorsäurealkylester.

Geeignete Acylglutamate sind Verbindungen der Formel (I) 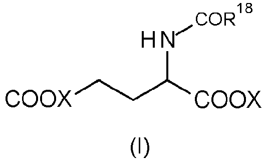 worin COR¹⁸ für einen linearen oder verzweigten Acylrest mit 6 bis 22 Kohlenstoffatomen und 0, 1, 2 oder 3 Doppelbindungen und X für Wasserstoff, ein Alkalimetall, das einwertige Ladungsäquivalent eines Erdalkalimetalls, Ammonium, Alkylammonium, Alkanolammonium oder Glucammonium steht. Die Herstellung von Acylglutamaten erfolgt beispielsweise durch Schotten-Baumann-Acylierung von Glutaminsäure mit Fettsäuren, Fettsäureestern oder Fettsäurehalogeniden.

Acylglutamate sind kommerziell beispielsweise von der Clariant AG, Frankfurt/DE, oder der Ajinomoto Co. Inc., Tokio/JP, erhältlich. Eine Übersicht zu Herstellung und Eigenschaften der Acylglutamate findet sich von M. Takehara et al. in J. Am. Oil Chem. Soc. 49 (1972) 143. Typische als Komponente b) geeignete Acylglutamate leiten sich bevorzugt von Fettsäuren mit 6 bis 22 und besonders bevorzugt 12 bis 18 Kohlenstoffatomen ab. Es werden insbesondere die Mono- oder Dialkalimetallsalze des Acylglutamats eingesetzt. Dazu zählen z. B. (Handelsnamen der Fa. Ajinomoto, USA in Klammern): Natriumcocoylglutamat (Amisoft CS-11), Dinatriumcocoylglutamat (Amisoft ECS-22SB), Triethanolammoniumcocoylglutamat (Amisoft CT-12), Triethanolammoniumlauroylglutamat (Amisoft LT-12), Natriummyristoylglutamat (Amisoft MS-11), Natriumstearoylglutamat (Amisoft HS-11 P) und Mischungen davon.

Zu den zusätzlichen nichtionischen Tensiden gehören beispielsweise:
- Glycerinester, wie beispielsweise Glycerinmonostearat,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate. Beispielsweise können Cocodimethylsulfopropylbetain, Laurylbetain, Cocamidopropylbetain, Natriumcocamphopropionat oder Tetradecyldimethylaminoxid eingesetzt werden.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide. Beispielsweise können Behenyl- oder Cetyltrimethylammoniumchlorid eingesetzt werden.

### Wasch- Reinigungs- und Geschirrspülmittel

Die erfindungsgemäße Polymerzusammensetzung eignet sich vorteilhaft für die Verwendung in Wasch- und Reinigungsmitteln, in Geschirrspülmittel und in Klarspülern.

Unter Waschmitteln versteht man dabei im Rahmen der vorliegenden Erfindung solche Mittel, die zum Reinigen von flexiblen Materialien mit hoher Saugfähigkeit verwendet werden, z.B. von Materialien mit einem textilen Charakter, während man unter Reinigungsmitteln im Rahmen der vorliegenden Erfindung solche Mittel versteht, die zum Reinigen von Materialien mit geschlossener Oberfläche, d.h. mit einer Oberfläche, die keine oder nur wenige und kleine Poren hat und infolgedessen keine oder nur eine geringe Saugfähigkeit aufweist, eingesetzt werden.

Beispiele für flexible Materialien mit hoher Saugfähigkeit sind solche, die natürliche, synthetische oder halbsynthetische Fasermaterialien enthalten oder daraus bestehen und die demzufolge in der Regel zumindest teilweise einen textilen Charakter aufweisen. Die faserhaltigen oder aus Fasern bestehenden Materialien können prinzipiell in jeder im Gebrauch oder der Herstellung und Verarbeitung vorkommenden Form vorliegen. Beispielsweise können Fasern ungeordnet in Form von Flocke oder Haufwerk, geordnet in Form von Fäden, Garnen, Zwirnen, oder in Form von Flächengebilden wie Vliesen, Lodenstoffen oder Filz, Geweben, Gewirken in allen denkbaren Bindungsarten vorliegen. Bei den Fasern kann es sich um Rohfasern oder um Fasern in beliebigen Verarbeitungsstadien handeln. Beispiele sind natürliche Eiweiß- oder Zellulosefasern, wie Wolle, Seide, Baumwolle, Sisal, Hanf oder Kokosfasern, oder synthetische Fasern, wie beispielsweise Polyester-, Polyamid- oder Polyacrylnitrilfasern.

Beispiele für Materialien, die keine oder nur wenige und kleine Poren haben und keine oder nur eine geringe Saugfähigkeit aufweisen, sind Metall, Glas, Email oder Keramik. Typische Objekte aus diesen Materialien sind z.B. metallene Spülbecken, Bestecke, Glas- und Porzellangeschirr, Badewannen, Waschbecken, Kacheln, Fliesen, ausgehärtete Kunstharze, wie z.B. dekorative Melaminharzoberflächen auf Küchenmöbeln oder lackierte Metallflächen wie z.B. Kühlschränke und Autokarosserien, gedruckten Schaltungen (printed circuit boards), Mikrochips, versiegelte oder lackierte Hölzer, z.B. Parkett oder Wandverkleidungen, Fensterrahmen, Türen, Kunststoffbeläge wie Fußbodenbeläge aus PVC oder Hartgummi, oder Hart- oder Weichschaumstoffe mit weitgehend geschlossenen Oberflächen.

Beispiele für Reinigungsmittel, die die erfindungsgemäße Polymerzusammensetzung enthalten, umfassen Wasch- und Reinigungsmittel, Geschirrspülmittel, wie Handgeschirrspülmittel oder Maschinengeschirrspülmittel (= Geschirrspülmittel für die Geschirrspülmaschine), Metallentfetter, Glasreiniger, Fußbodenreiniger, Allzweckreiniger, Hochdruckreiniger, Neutralreiniger, alkalische Reiniger, saure Reiniger, Spritzentfetter, Molkereireiniger, Großküchenreiniger, Apparatereiniger in der Industrie, insbesondere der chemischen Industrie, Reiniger für der Autowäsche und auch Haushalts-Allzweckreiniger.

Eine erste Ausführungsform sind Wasch- und Reinigungs- und Geschirrspülmittel, die die erfindungsgemäße Polymerzusammensetzung enthalten und die bei Raumtemperatur (20 °C) fest sind. Eine zweite Ausführungsform sind Wasch- und Reinigungs- und Geschirrspülmittel, die eine nicht erfindungsgemäße Polymerzusammensetzung enthalten, die bei Raumtemperatur (20 °C) flüssig oder gelförmig ist und die eine erfindungsgemäße Polymerzusammensetzung in der Umhüllung oder Beschichtung der Wasch-, Reinigungs- oder Geschirrspülmittel oder wenigstens eine deren Komponenten enthalten. Eine speziellere Ausführung sind Wasch- und Reinigungs- und Geschirrspülmittel, die eine erfindungsgemäße Polymerzusammensetzung in der Umhüllung oder Beschichtung enthalten, wobei die Umhüllung oder Beschichtung zusätzlich wenigstens einen Wirkstoff enthält. Der wenigstens eine Wirkstoff wird dann beim Wasch- und Reinigungs- oder Geschirrspülvorgang kontrolliert freigesetzt.

Bei den festen Wasch- und Reinigungsmitteln kann es sich um pulverförmige oder tablettenförmige Produkte ("Tabs") handeln. In einer speziellen Ausführung handelt es sich um tablettenförmige Produkte ("Tabs"). In einer ersten besonders bevorzugten Ausführung handelt es sich dann um tablettenförmige Waschmittel. In einer weiteren besonders bevorzugten Ausführung handelt es sich dann um tablettenförmige Geschirrspülmittel, insbesondere um tablettenförmige Maschinengeschirrspülmittel.

Bei tablettenförmigen Geschirrspülmitteln kann es sich um einfache Tabs oder auch um sogenannte "2 in 1"-, "3 in 1"-, "5 in 1"- oder "7 in 1"-Produkte (multifunktionelle Produkte) handeln. Näheres zu diesen Formulierungen findet sich in Hermann G. Hauthal, G. Wagner (Hrsg.), Reinigungs- und Pflegemittel im Haushalt, Verlag für chemische Industrie, H. Ziolkowsky GmbH, Augsburg 2003, Kapitel 4.2, Seiten 161-184. "2 in 1"-Produkte enthalten neben den üblichen Bestandteilen von Maschinengeschirrspülmitteln zusätzlich einen Klarspüler. "3 in 1"-Produkte enthalten außerdem einen Wasserenthärter. "5 in 1"-Produkte enthalten in der Regel außerdem ein Glasschutzmittel und ein Spülkraftverstärkungsmittel. "7 in 1"-Produkte enthalten außerdem ein Edelstahlglanzmittel und ein Entkrustungsmittel. In diesen tablettenförmigen Geschirrspülmitteln kann die erfindungsgemäße Polymerzusammensetzung im Klarspülkern enthalten sein oder sie ist als Feststoff im tablettenförmigen Geschirrspülmittel enthalten.

Die erfindungsgemäße Polymerzusammensetzung eignet sich weiterhin zur Herstellung einer Umhüllung, die feste oder flüssige oder gelförmige Wasch-, Reinigungs- oder Geschirrspülmittel oder wenigstens eine deren Komponenten enthält. Die erfindungsgemäße Polymerzusammensetzung eignet sich weiterhin zur Herstellung einer Beschichtung auf einem festen Wasch-, Reinigungs- oder Geschirrspülmittel oder auf wenigstens einer festen Komponente davon.

Die erfindungsgemäße Polymerzusammensetzung zeichnet sich insbesondere durch eine hervorragende Wirkung als Cobuilder, Tensid oder soil release Polymer aus. Beim Einsatz im Klarspülgang des maschinellen Geschirrspülers wirkt sie auch als Inkrustierungsinhibitor. Die Bestandteile der Polymerzusammensetzung unterstützen weiterhin die Reinigungsleistung der Gesamtformulierung, indem sie den Schmutz ablösen und dispergieren.

Das erfindungsgemäße Wasch-, Reinigungs- und Geschirrspülmittel umfasst vorzugsweise folgende Bestandteile:
a) wenigstens eine erfindungsgemäße Polymerzusammensetzung;
b) wenigstens einen Builder (auch als Sequestrierungsmittel, Gerüststoff, Komplexbildner, Chelator, Chelatisierungsmittel oder Enthärter bezeichnet);
c) gegebenenfalls wenigstens ein Enzym;
d) gegebenenfalls wenigstens ein Bleichmittel; und
e) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Tablettierhilfsmitteln, Desintegrationsmiteln, Verdickern, Löslichkeitsvermittlern, organischen Lösemitteln, Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln, UV-Absorbern und Wasser.

### Wasch- und Reinigungsmittel

Vorzugsweise enthalten die erfindungsgemäßen Wasch- und Reinigungsmittel:
a) wenigstens eine erfindungsgemäße Polymerzusammensetzung: von 0,1 bis 20 Gew.-%;
b) wenigstens ein Builder: von 5 bis 80 Gew.-%;
c) wenigstens ein Enzym: von 0 bis 8 Gew.-%;
d) wenigstens ein Bleichmittel: von 0 bis 30 Gew.-%; und
e) wenigstens ein weiterer Zusatzstoff: 0 bis 50 Gew.-%.

Die Gew.-%-Angaben beziehen sich dabei auf das Gesamtgewicht des Wasch- und Reinigungsmittels. Die Gewichtsmengen von a) bis e) ergänzen sich zu 100 Gew.-%.

Besonders bevorzugt umfasst das erfindungsgemäße Wasch- und Reinigungsmittel wenigstens ein Enzym.

Besonders bevorzugt sind die oben genannten Bestandteile in folgenden Mengenverhältnissen im erfindungsgemäßen Wasch- und Reinigungsmittel enthalten:
a) wenigstens eine erfindungsgemäße Polymerzusammensetzung: von 0,1 bis 10 Gew.-%;
b) wenigstens einen Builder: von 20 bis 80 Gew.-%;
c) wenigstens ein Enzym: von 0,1 bis 6 Gew.-%;
d) wenigstens ein Bleichmittel: von 0 bis 30 Gew.-%; und
e) wenigstens ein weiterer Zusatzstoff: 0 bis 50 Gew.-%.

Die Gew.-%-Angaben beziehen sich dabei auf das Gesamtgewicht des Wasch- und Reinigungsmittels. Die Gewichtsmengen von a) bis e) ergänzen sich zu 100 Gew.-%.

Stärker bevorzugt umfasst das erfindungsgemäße Wasch- und Reinigungsmittel außerdem wenigstens ein Bleichmittel.

Stärker bevorzugt sind die oben genannten Bestandteile in folgenden Mengenverhältnissen im erfindungsgemäßen Wasch- und Reinigungsmittel enthalten:
a) wenigstens eine erfindungsgemäße Polymerzusammensetzung: von 0,1 bis 10 Gew.-%;
b) wenigstens ein Builder: von 20 bis 80 Gew.-%;
c) wenigstens ein Enzym: von 0,1 bis 6 Gew.-%;
d) wenigstens ein Bleichmittel: von 5 bis 25 Gew.-%; und
e) wenigstens ein weiterer Zusatzstoff: 0 bis 50 Gew.-%.

Die Gew.-%-Angaben beziehen sich dabei auf das Gesamtgewicht des Wasch- und Reinigungsmittels. Die Gewichtsmengen von a) bis e) ergänzen sich zu 100 Gew.-%.

Bezüglich geeigneter und bevorzugter erfindungsgemäßer Polymerzusammensetzungen wird auf die vorstehenden Ausführungen verwiesen.

### Geschirrspülmittel

Die zuvor beschriebenen erfindungsgemäßen Polymerzusammensetzungen eignen sich besonders vorteilhaft zum Einsatz in Geschirrreinigungsmitteln, insbesondere für maschinelle Geschirrreinigungsverfahren(automatic dish washing, ADW). Die erfindungsgemäße Polymerzusammensetzung hat sowohl eine Wirkung als Tensid als auch als Cobuilder. Beispiele für erfindungsgemäße Formulierungen, die die Polymerzusammensetzung enthalten, für die Geschirrreinigung, umfassen somit Maschinengeschirrspülmittel, Klarspülmittel und Maschinengeschirrspülmittel mit Klarspülfunktion. Die Formulierungen liegen insbesondere bei Raumtemperatur (20 °C) vor.
Maschinelle Geschirrreinigungsverfahren im häuslichen und im gewerblichen Bereich umfassen mehrere aufeinanderfolgende Schritte, wobei der erste das mechanische Abtragen lose anhaftender Speisereste umfasst, der zweite den eigentlichen Reinigungsvorgang mit Hilfe einer Geschirrspülmaschine darstellt und der dritte in der Regel aus einem Klarspülschritt besteht, an den sich das Trocknen des gereinigten Geschirrs anschließt. Diese Vorgänge werden mehr oder weniger automatisiert durchgeführt, wobei als zentrale Einheit eine Geschirrspülmaschine zum Einsatz kommt, in der zumindest der Reinigungsschritt und in der Regel auch der anschließende Klarspülschritt und/oder der Trocknungsschritt durchgeführt werden.

In Geschirrspülmaschinen für den häuslichen Bereich erfolgt die Reinigung des verschmutzten Geschirrs in der Regel in einer einzelnen Kammer, wobei die zuvor genannten Behandlungsschritte programmgesteuert nacheinander ablaufen. Frischwasser gelangt über die Enthärtungseinheit zum Pumpensumpf und wird mittels bewegter Sprüharme über dem zu spülenden Gut versprüht. Abgespülte wasserunlösliche Stoffe werden am Pumpensumpf herausgefiltert. Beim zweiten Spülgang wird ein in der Regel alkalisches Reinigungsmittel dem Spülwasser zugesetzt, auf die eingestellte Temperatur erhitzt und auf dem zu spülenden Gut verteilt. Beim letzten Spülgang wird der Behandlungsflüssigkeit ein Klarspülmittel zugesetzt, das die Oberflächenspannung reduziert, wodurch die Behandlungsflüssigkeit leichter vom Spülgut abläuft. Nach dem letzten Spülgang wird der Inhalt getrocknet. Die im Spülvorgang eingesetzten Komponenten, wie Wasseraufbereiter, Reinigungsmittel, Klarspülmittel, etc., können entweder in Form von Einzelkomponenten oder in Mehrkomponentenformulierungen eingesetzt werden. Solche multifunktionalen Reiniger enthalten Tenside für das Klarspülen und ein Polymer für die Wasserenthärtung. In die Geschirrspülmaschine muss dann nicht separat ein Klarspülmittel und ein Salz für die Wasserenthärtung gegeben werden.

Gewerbliche Geschirrspülmaschinen bestehen im Prinzip aus stationären Bädertanks, aus denen eine im Wesentlichen wässrige Reinigungslösung auf das Geschirr aufgedüst oder -gesprüht wird, das sich auf einem Laufband über diesen Bädern vorbeibewegt, so dass die gebrauchte Lösung wieder in die Bädertanks zurück gelangt. Dabei tritt Wasser in den letzten Bädertank ein, fließt durch Überläufe kaskadenartig durch alle anderen Tanks und verlässt die Maschine durch den Überlauf des ersten Tanks. Das Aufbringen einer in der Regel hochalkalischen Reinigungslösung findet in der Regel mit Hilfe dafür vorgesehener Düsen oder einer speziellen Sprühanlage statt, die normalerweise im mittleren Bereich der Maschine angeordnet ist.

Die erfindungsgemäßen Formulierungen für die maschinelle Geschirrreinigung zeichnen sich insbesondere durch eine hervorragende belagsinhibierende Wirkung beim Einsatz im Klarspülgang des maschinellen Geschirrspülers aus (d. h. sie wirkt als Inkrustierungsinhibitor). Sie wirken dabei sowohl gegenüber anorganischen als auch gegenüber organischen Belägen inhibierend. Bei den anorganischen Belägen handelt es sich insbesondere um Calcium- und Magnesiumphosphat, -carbonat, -silikat und/oder -phosphonat, die aus den im Wasser enthaltenen Calcium- und Magnesiumsalzen und den in üblichen Geschirrspülmitteln enthaltenen Buildern entstehen. Bei den organischen Belägen handelt es sich insbesondere um Schmutzbestandteile aus der Spülflotte, wie z. B. Eiweiß-, Stärke- und Fettbeläge. Die erfindungsgemäßen Formulierungen für die maschinelle Geschirrreinigung sind auch gegenüber sogenannten Carry-Over-Belägen wirksam, die aus der Restwassermenge im Sumpf der Spülmaschine stammen und u. a. Geschirrspülmittelreste und eventuell auch noch Schmutzreste aus dem vorhergehenden Waschgang der Geschirrspülmaschine enthalten.

Die erfindungsgemäße Formulierung für die maschinelle Geschirrreinigung umfasst vorzugsweise folgende Bestandteile:
a) wenigstens eine erfindungsgemäße Polymerzusammensetzung,
b) wenigstens einen Builder (auch als Sequestrierungsmittel, Gerüststoff, Komplexbildner, Chelator, Chelatisierungsmittel oder Enthärter bezeichnet),
c) gegebenenfalls wenigstens ein Enzym,
d) gegebenenfalls wenigstens ein Bleichmittel,
e1) Wasser,
e2) gegebenenfalls wenigstens einen Verdicker, und
e3) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Löslichkeitsvermittlern und organischen Lösungsmitteln.

Die erfindungsgemäße Formulierung für die maschinelle Geschirrreinigung enthält, bezogen auf das Gesamtgewicht der Formulierung, vorzugsweise:
a) 0,1 bis 50 Gew.-% wenigstens einer erfindungsgemäßen Polymerzusammensetzung,
b) 5 bis 90 Gew.-% wenigstens eines Builders und/oder Cobuilders,
c) 0 bis 8 Gew.-% wenigstens eines Enzyms,
d) 0 bis 30 Gew.-% wenigstens eines Bleichmittels,
e1) 0,1 bis 90 Gew.-% Wasser,
e2) 0 bis 8 Gew.-% wenigstens eines Verdickers
e3) 0 bis 25 Gew.-% wenigstens eines weiteren Zusatzstoffs,
mit der Maßgabe, dass sich die Gewichtsmengen der Komponenten a) bis e) zu 100 Gew.-% ergänzen.

In einer bevorzugten Ausführungsform enthält die erfindungsgemäße Formulierung für die maschinelle Geschirrreinigung wenigstens ein Enzym.

Die erfindungsgemäße Formulierung für die maschinelle Geschirrreinigung enthält, bezogen auf das Gesamtgewicht der Formulierung, vorzugsweise:
a) 2 bis 40 Gew.-% wenigstens einer erfindungsgemäßen Polymerzusammensetzung,
b) 5 bis 80 Gew.-% wenigstens eines Builders und/oder Cobuilders,
c) 0,1 bis 6 Gew.-% wenigstens eines Enzyms,
d) 0 bis 30 Gew.-% wenigstens eines Bleichmittels,
e1) 0,1 bis 80 Gew.-% Wasser,
e2) 0 bis 6 Gew.-% wenigstens eines Verdickers
e3) 0 bis 25 Gew.-% wenigstens eines weiteren Zusatzstoffs,
mit der Maßgabe, dass sich die Gewichtsmengen der Komponenten a) bis e) zu 100 Gew.-% ergänzen.

Besonders bevorzugt enthält die erfindungsgemäße Formulierung für die maschinelle Geschirrreinigung, bezogen auf das Gesamtgewicht der Formulierung:
a) 0,12 bis 30 Gew.-% wenigstens einer erfindungsgemäßen Polymerzusammensetzung,
b) 5 bis 75 Gew.-% wenigstens eines Builders und/oder Cobuilders,
c) 0,1 bis 6 Gew.-% wenigstens eines Enzyms,
d) 0 bis 25 Gew.-% wenigstens eines Bleichmittels,
e1) 0,1 bis 80 Gew.-% Wasser,
e2) 0,1 bis 5 Gew.-% wenigstens eines Verdickers
e3) 0 bis 25 Gew.-% wenigstens eines weiteren Zusatzstoffs,
mit der Maßgabe, dass sich die Gewichtsmengen der Komponenten a) bis e) zu 100 Gew.-% ergänzen.

Die Polymerzusammensetzung a) kann in die Formulierung eingearbeitet werden oder in der Gesamtformulierung in einem separaten Bereich als klare Gelkomponente von den übrigen Komponenten separat vorliegen (z. B. abgetrennt durch eine Folie, beispielsweise aus einer erfindungsgemäßen Polymerzusammensetzung oder aus Polyvinylalkohol).

Die Geschirreinigerformulierungen können als Tabletten oder vollständig flüssige Formulierungen vorliegen. Es können aber auch mit Folie oder Formkörper hergestellte Behälter mit 1 bis 6 gleich großen oder unterschiedlich großen Einzelabschnitten vorliegen. Diese können unabhängig voneinander mit Pulver, Granulat, Feststoff oder Flüssigkeit gefüllt sein. Die Polymerzusammensetzung a) wird bevorzugt in einem separaten Kompartiment abgefüllt. Die Polymerzusammensetzung a) kann zusätzlich verdickt oder eingefärbt werden.

### Komponente a)

Bezüglich als Komponente a) geeignete und bevorzugte erfindungsgemäße Polymerzusammensetzungen wird auf die vorstehenden Ausführungen verwiesen.

### Komponente b)

Die erfindungsgemäßen Wasch- und Reinigungs- und Geschirrspülmittel enthalten vorzugsweise wenigstens einen Builder.

Builder, die teilweise auch auch als Sequestrierungsmittel, Gerüststoff, Komplexbildner, Chelator, Chelatisierungsmittel oder Enthärter bezeichnet werden, binden Erdalkalimetalle und andere wasserlösliche Metallsalze, ohne zu präzipitieren. Sie helfen Schmutz aufzubrechen, dispergieren Schmutzteilen, helfen Schmutz abzulösen und haben teilweise eine eigene Waschwirkung. Außerdem halten sie, wenn sie fest sind und in pulverförmigen Formulierungen eingesetzt werden, das Pulver rieselfähig.

Geeignete Builder können sowohl organischer als auch anorganischer Natur sein. Beispiele sind Alumosilikate, Carbonate, Phosphate und Polyphosphate, Polycarbonsäuren, Polycarboxylate, Hydroxycarbonsäuren, Phosphonsäuren, z.B. Hydroxyalkylphosphonsäuren, Phosphonate, Aminopolycarbonsäuren und deren Salze und polymere carbonsäuregruppenhaltige Verbindungen und deren Salze.

Geeignete anorganische Builder sind beispielsweise kristalline oder amorphe Alumosilikate mit ionenaustauschenden Eigenschaften, wie Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise in der US-A-4604224 beschrieben. Als Builder geeignete kristalline Silikate sind beispielsweise Disilikate oder Schichtsilikate, z.B. 5-Na₂Si₂O₅ oder B-Na₂Si₂O₅ (SKS 6 bzw. SKS 7). Die Silikate können in Form ihrer Alkalimetall-, Erdalkalimetall- oder Ammoniumsalze eingesetzt werden, vorzugsweise als Na-, Li- und Mg-Silikate. Amorphe Silikate wie beispielsweise Natriummetasilikat, welches eine polymere Struktur aufweist, oder amorphes Disilikat (Britesil® H 20 Hersteller: Akzo) sind ebenfalls verwendbar. Bevorzugt ist hierunter Natriumdisilikat.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. -Hydrogencarbonate, insbesondere Natriumcarbonat und/oder Natriumhydrogencarbonat, eingesetzt.

Übliche, als anorganische Builder eingesetzte Phosphate sind Alkalimetallorthophosphate und/oder -polyphosphate wie z.B. Pentanatriumtriphosphat.

Geeignete organische Builder sind beispielsweise C₄-C₃₀-Di-, -Tri- und -Tetracarbonsäuren, wie z.B. Bernsteinsäure, Propantricarbonsäure, Butantetracarbonsäure, Cyclopentantetracarbonsäure und Alkyl- und Alkenylbernsteinsäuren mit C₂-C₂₀-Alkyl- bzw. -Alkenyl-Resten.

Geeignete organische Builder sind weiterhin Hydroxycarbonsäuren und Polyhydroxycarbonsäuren (Zuckersäuren). Dazu zählen C₄-C₂₀-Hydroxycarbonsäuren wie z.B. Äpfelsäure, Weinsäure, Gluconsäure, Schleimsäure, Milchsäure, Glutarsäure, Zitronensäure, Tartronsäure, Glucoheptonsäure, Lactobionsäure und Saccharosemono-, -di- und -tricarbonsäure. Bevorzugt sind hierunter Zitronensäure und ihre Salze.

Geeignete organische Builder sind weiterhin Phosphonsäuren, wie z.B. Hydroxyalkylphosphonsäuren, Aminophosphonsäuren und die Salze davon. Dazu zählen z.B. Phosphonobutantricarbonsäure, Aminotris-methylenphosphonsäure, Ethylendiamintetraethylenphosphonsäure, Hexamethylendiamintetramethylenphosphonsäure, Diethylentriaminpentamethylenphosphonsäure, Morpholino-methandiphosphonsäure, 1-Hydroxy-C₁-bis C₁₀-alkyl-1,1-diphosphonsäuren wie 1-Hydroxyethan-1,1-diphosphonsäure. Bevorzugt ist hierunter 1-Hydroxyethan-1,1-diphosphonsäure und deren Salze.

Geeignete organische Builder sind weiterhin Aminopolycarbonsäuren, wie Nitrilotriessigsäure (NTA), Nitrilomonoessigdipropionsäure, Nitrilotripropionsäure, β-Alanindiessigsäure (β-ADA), Ethylendiamintetraessigsäure (EDTA), Diethylentriaminpentaessigsäure, 1,3-Propylendiamintetraessigsäure, 1,2-Propylendiamintetraessigsäure, N-(Alkyl)-ethylendiamintriessigsäure, N-(Hydroxyalkyl)-ethylendiamintriessigsäure, Ethylendiamintriessigsäure, Cyclohexylen-1,2-diamintetraessigsäure, Iminodibernsteinsäure, Ethylendiamindibernsteinsäure, Serindiessigsäure, Isoserindiessigsäure, L-Asparagindiessigsäure, L-Glutamindiessigsäure, Methylglycindiessigsäure (MGDA) und die Salze der zuvorgenannten Aminopolycarbonsäuren. Bevorzugt sind hierunter L-Glutamindiessigsäure, Methylglycindiessigsäure und deren Salze.

Geeignete organische Builder sind weiterhin polymere carbonsäuregruppenhaltige Verbindungen, wie Acrylsäure-Homopolymere. Diese weisen vorzugsweise ein zahlenmittleres Molekulargewicht im Bereich von 800 bis 70000 g/mol, besonders bevorzugt von 900 bis 50000 g/mol, insbesondere von 1000 bis 20000 g/mol, speziell 1000 bis 10000 g/mol, auf. Der Begriff Acrylsäure-Homopolymer umfasst dabei auch Polymere, in denen die Carbonsäuregruppen teilweise oder vollständig neutralisiert vorliegen. Dazu zählen Acrylsäure-Homopolymere, in denen die Carbonsäuregruppen teilweise oder vollständig in Form von Alkalimetallsalzen oder Ammoniumsalzen vorliegen. Bevorzugt sind Acrylsäure-Homopolymere, in denen die Carbonsäuregruppen protoniert sind oder in denen die Carbonsäuregruppen teilweise oder vollständig in Form von Natriumsalzen vorliegen.

Geeignete polymere carbonsäuregruppenhaltige Verbindungen sind auch Oligomaleinsäuren, wie sie beispielsweise in EP-A 451 508 und EP-A 396 303 beschrieben sind.

Geeignete polymere carbonsäuregruppenhaltige Verbindungen sind auch Terpolymere ungesättigter C₄-C₈-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können. Als ungesättigte C₄-C₈-Dicarbonsäuren sind hierbei beispielsweise Maleinsäure, Fumarsäure, Itaconsäure und Citraconsäure geeignet. Bevorzugt wird Maleinsäure. Die Gruppe (i) umfasst monoethylenisch ungesättigte C₃-C₈-Monocarbonsäuren wie z.B. Acrylsäure, Methacrylsäure, Crotonsäure und Vinylessigsäure. Bevorzugt werden aus der Gruppe (i) Acrylsäure und Methacrylsäure eingesetzt. Die Gruppe (ii) umfasst monoethylenisch ungesättigte C₂-C₂₂-Olefine, Vinylalkylether mit C₁-C₈-Alkylgruppen, Styrol, Vinylester von C₁-C₈-Carbonsäuren, (Meth)acrylamid und Vinylpyrrolidon. Bevorzugt werden aus der Gruppe (ii) C₂-C₆-Olefine, Vinylalkylether mit C₁-C₄-Alkylgruppen, Vinylacetat und Vinylpropionat eingesetzt. Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt. Die Gruppe (iii) umfasst (Meth)acrylester von C₁-C₈-Alkoholen, (Meth)acrylnitril, (Meth)acrylamide von C₁-C₈-Aminen, N-Vinylformamid und N-Vinylimidazol.

Geeignete polymere carbonsäuregruppenhaltige Verbindungen sind auch Homopolymere der monoethylenisch ungesättigten C₃-C₈-Monocarbonsäuren wie z.B. Acrylsäure, Methacrylsäure, Crotonsäure und Vinylessigsäure, insbesondere der Acrylsäure und Methacrylsäure, Copolymere von Dicarbonsäuren, wie z.B. Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000; Terpolymere aus Maleinsäure, Acrylsäure und einem Vinylester einer C₁-C₃- Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann; Copolymere von Maleinsäure mit C₂-C₈-Olefinen im Molverhältnis 40:60 bis 80:20, wobei Copolymere von Maleinsäure mit Ethylen, Propylen oder Isobuten im Molverhältnis 50:50 besonders bevorzugt sind.

Geeignete polymere carbonsäuregruppenhaltige Verbindungen sind weiterhin Copolymere von 50 bis 98 Gew.-% ethylenisch ungesättigter schwacher Carbonsäuren mit 2 bis 50 Gew.-% ethylenisch ungesättigter Sulfonsäuren, wie sie beispielsweise in der EP-A-0877002 beschrieben sind. Geeignete schwache ethylenisch ungesättigte Carbonsäuren sind insbesondere C₃-C₆-Monocarbonsäuren, wie Acrylsäure und Methacrylsäure. Geeignete ethylenisch ungesättigte Sulfonsäuren sind 2-Acetylamidomethyl-1-propansulfonsäure, 2-Methacrylsäureamido-2-methyl-1-propansulfonsäure, 2-Methacrylamindo-2-hydroxypropansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen-1-sulfonsäure, Styrolsulfonsäure, Vinylsulfonsäure, 3-Sulfopropylacrylat, 3-Sulfopropylmethacrylat, Sulfomethylacrylamid, Sulfomethylmethacrylamid und Salze dieser Säuren. Die Copolymere können weiterhin 0 bis 30 Gew.-% ethylenisch ungesättigter C₄-C₈-Dicarbonsäuren, wie Maleinsäure, sowie 0 bis 30 Gew.-% wenigstens eines Monomers, das mit den zuvor genannten Monomeren copolymerisierbar ist, einpolymerisiert enthalten. Bei letzterem handelt es sich beispielsweise um C₁-C₄-Alkylester von (Meth)Acrylsäure, C₁-C₄-Hydroxyalkylester von (Meth)Acrylsäure, Acrylamid, Alkyl-substituiertes Acrylamid, N,N-Dialkyl-substituiertes Acrylamid, Vinylphosphonsäure, Vinylacetat, Allylalkohole, sulfonierte Allylalkohole, Styrol und andere Vinylaromaten, Acrylonitril, N-Vinylpyrrolidon, N-Vinylformamid, N-Vinylimidazol oder N-Vinylpyridin. Das gewichtsmittlere Molekulargewicht dieser Copolymere liegt im Bereich von 3000 bis 50000 Dalton. Besonders geeignet sind Copolymere mit etwa 77 Gew.-% wenigstens einer ethylenisch ungesättigten C₃-C₆-Monocarbonsäure und etwa 23 Gew.-% wenigstens einer ethylenisch ungesättigten Sulfonsäure.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls. Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden. Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii). Als Pfropfgrundlage sind abgebaute Polysaccharide wie z.B. sauer oder enzymatisch abgebaute Stärken, Inuline oder Zellulose, Eiweisshydrolysate und reduzierte (hydrierte oder hydrierend aminierte) abgebaute Polysaccharide wie z.B. Mannit, Sorbit, Aminosorbit und N-Alkylglucamin geeignet sowie auch Polyalkylenglykole mit Molmassen mit bis zu M_{w} = 5000 wie z. B. Polyethylenglykole, Ethylenoxid/Propylenoxidbzw. Ethylenoxid/Butylenoxid bzw. Ethylenoxid/Propylenoxid/Butylenoxid-Blockcopolymere und alkoxylierte ein- oder mehrwertige C₁-C₂₂-Alkohole. (vgl. US-A-5756456).

Ebenfalls geeignet sind Polyglyoxylsäuren, wie sie beispielsweise in EP-B-001004, USA-5399286, DE-A-4106355 und EP-A-656914 beschrieben sind. Die Endgruppen der Polyglyoxylsäuren können unterschiedliche Strukturen aufweisen.

Weiterhin sind Polyamidocarbonsäuren und modifizierte Polyamidocarbonsäuren geeignet; diese sind beispielsweise aus EP-A-454126, EP-B-511037, WO-A94/01486 und EP-A-581452 bekannt.

Auch Polyasparaginsäuren oder Cokondensate der Asparaginsäure mit weiteren Aminosäuren, C₄-C₂₅-Mono- oder -Dicarbonsäuren und/oder C₄-C₂₅-Mono- oder-Diaminen können als polymere carbonsäuregruppenhaltige Verbindungen eingesetzt werden. Besonders bevorzugt werden in phosphorhaltigen Säuren hergestellte, mit C₆-C₂₂-Mono- oder -Dicarbonsäuren bzw. mit C₆-C₂₂-Mono- oder -Diaminen modifizierte Polyasparaginsäuren eingesetzt.

Unter den polymeren carbonsäuregruppenhaltigen Verbindungen sind Polyacrylsäuren auch in teilweise oder vollständig neutralisierter Form, bevorzugt.

Als organische Builder eignen sich weiterhin Iminodibernsteinsäure, Oxydibernsteinsäure, Aminopolycarboxylate, Alkylpolyaminocarboxylate, Aminopolyalkylenphosphonate, Polyglutamate, hydrophob modifizierte Citronensäure wie z.B. Agaricinsäure, Poly-[alpha]-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Builder verwendet werden.

Bevorzugt wird als Komponente b) ein Gemisch verschiedener Builder eingesetzt.

Bevorzugt enthält das Gemisch verschiedener Builder wenigstens zwei der folgenden Bestandteile: wenigstens ein Carbonat (z.B. Natriumcarbonat), wenigstens ein Silikat (z.B. Natriumdisilkat), wenigstens eine polymere carbonsäuregruppenhaltige Verbindung oder wenigstens eine polymere carbonsäuregruppenhaltige Verbindung, in der die Carbonsäuregruppen teilweise oder vollständig neutralisiert vorliegen (z.B. Polyacrylsäure), wenigstens eine (Poly)Hydroxycarbonsäure oder ein Salz davon (z.B. Zitronensäure oder ein Citrat), wenigstens eine Aminopolycarbonsäure oder ein Salz davon (z.B. Methylglycindiessigsäure oder ein Salz davon, z.B. ein Natriumsalz davon), wenigstens eine Phosphonsäure (z.B. 1-Hydroxyethan1-(1,1-diphosphonsäure); HEDP), wenigstens ein Phosphat. Besonders bevorzugt enthält das Gemisch wenigstens ein Carbonat, wenigstens ein Silikat und wenigstens eine polymere gegebenenfalls (teil)neutralisierte carbonsäuregruppenhaltige Verbindung sowie optional wenigstens einen der folgenden Bestandteile: wenigstens eine (Poly)Hydroxycarbonsäure oder ein Salz davon, wenigstens eine Phosphonsäure, wenigstens ein Phosphat. Speziell enthält das Gemisch wenigstens ein Carbonat, wenigstens ein Silikat, wenigstens eine polymere gegebenenfalls (teil)neutralisierte carbonsäuregruppenhaltige Verbindung, wenigstens eine (Poly)Hydroxycarbonsäure oder ein Salz davon und wenigstens eine Phosphonsäure, sowie optional wenigstens ein Phosphat.

In einem solchen Gemisch sind die Bestandteile vorzugsweise in folgenden Mengen enthalten:
b1) wenigstens ein Carbonat: 10 bis 50 Gew.-%;
b2) wenigstens ein Silikat: 1 bis 10 Gew.-%;
b3) wenigstens eine polymere gegebenenfalls (teil)neutralisierte carbonsäuregruppenhaltige Verbindung: 5 bis 20 Gew.-%;
b4) wenigstens eine (Poly)Hydroxycarbonsäure oder ein Salz davon: 0 bis 50 Gew.-%;
b5) wenigstens eine Aminopolycarbonsäure oder ein Salz davon: 0 bis 60 Gew.-%;
b6) wenigstens eine Phosphonsäure: 0,2 bis 1 Gew.-%;
b7) wenigstens ein Phosphat: 0 bis 60 Gew.-%.

Die Gew.-%-Angaben beziehen sich dabei auf das Gesamtgewicht des Builders. Die Gewichtsmengen von b1) bis b7) ergänzen sich zu 100 Gew.-%.

### Komponente c)

Die erfindungsgemäßen Wasch-, Reinigungs- und Geschirrspülmittel enthalten vorzugsweise wenigstens ein Enzym.

Geeignete Enzyme sind solche, wie sie üblicherweise als industrielle Enzyme eingesetzt werden. Dazu zählen sowohl Enzyme mit optimaler Aktivität im neutralen bis alkalischen pH-Bereich, als auch Enzyme mit optimaler Aktivität im sauren pH-Bereich.

Die Enzyme sind vorzugsweise ausgewählt unter Aminopeptidasen, Amylasen, Arabinasen, Carbohydrasen, Carboxypeptidasen, Catalasen, Cellulasen, Chitinasen, Cutinasen, Cyclodextringlycosyltransferasen, Deoxyribonucleasen, Esterasen, Galactanasen, Alpha-Galactosidasen, Beta-Galactosidasen, Glucanasen, Glucoamylasen, Alpha-Glucosidasen, Beta-Glucosidasen, Haloperoxidasen, Hydrolaseinvertasen, Isomerasen, Keratinasen, Laccasen, Lipasen, Mannanasen, Mannosidasen, Oxidasen, pectinolytischen Enzymen, Peptidoglutaminasen, Peroxidasen, Peroxygenasen, Phytasen, Polyphenoloxidasen, proteolytischen Enzymen, Ribonucleasen, Transglutaminasen, Transferasen, Xylanasen und Mischungen davon

Die Enzyme sind speziell ausgewählt unter Hydrolasen, wie Proteasen, Esterasen, Glucosidasen, Lipasen, Amylasen, Cellulasen, Mannanasen, anderen Glykosylhydrolasen und Gemischen der zuvor genannten Enzyme. Alle diese Hydrolasen tragen zur Schmutzauflösung und -entfernung von protein-, fett- oder stärkehaltigen Verschmutzungen bei. Zur Bleiche können auch Oxireduktasen eingesetzt werden. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe.

Bevorzugte Enzyme werden im Folgenden genauer beschrieben:

### Proteasen:

Geeignete proteolytische Enzyme (Proteasen) können grundsätzlich tierischen, pflanzlichen oder mikrobiellen Ursprungs sein. Bevorzugt sind proteolytische Enzyme mikrobiellen Ursprungs. Dazu zählen auch chemisch oder genetisch modifizierte Mutanten.

Bevorzugte Proteasen sind Serinproteasen, Metalloproteasen oder trypsinartige Proteasen. Vorzugsweise wird eine alkalische mikrobielle Protease eingesetzt. Beispiele für alkalische Proteasen sind Subtilisine, speziell solche die von *Bacillus* abgeleitet sind, z.B. Subtilisin Novo, Subtilisin Carlsberg, Subtilisin 309, Subtilisin 147 und Subtilisin 168 (beschrieben in WO 89/06279). Beispiele für trypsinartige Proteasen sind Trypsin (z.B. aus Schwein oder Rind) und die z.B. in der WO 89/06270 beschriebene *Fusarium* Protease.

Bevorzugte kommerziell erhältliche Proteasen umfassen die unter folgenden Markenbezeichnungen erhältlichen Proteasen: Alcalase™, Savinase™, Primase™, Durazym™, Esperase™, Neutrase™ von Novozymes A/S (Dänemark), die von DuPont/Genencor unter den Markennamen Maxatase™, Maxacal™, Maxapem™,, PREFERENZ™ P, EXCELLENZ™ P Properase™, Purafect™ und Purafect™ OXP vertriebenen Produkte, und die von Solvay Enzymes unter den Markennamen Opticlean™ und Optimase™ vertriebenen Produkte.

Die erfindungsgemäßen Zusammensetzungen enthalten vorzugsweise wenigstens eine Protease in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Insbesondere liegt die erfindungsgemäße Zusammensetzung in Form einer enzymhaltigen Folie vor. Dann enthält die erfindungsgemäßen Zusammensetzung vorzugsweise wenigstens eine Protease in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der enzymhaltigen Folie.

### Lipasen:

Geeignete Lipasen können grundsätzlich von Bakterien oder Pilzen stammen. Dazu zählen auch chemisch oder genetisch modifizierte Mutanten.

Beispiele für geeignete Lipasen sind *Humicola lanuginosa* Lipase, wie z.B. beschrieben in EP 258 068 und EP 305 216, *Rhizomucor miehei* Lipase, wie z.B. beschrieben in EP 238 023, *Candida* Lipase, wie C. *antarctica* Lipase, z.B. die C. *antarctica* Lipasen A or B wie beschrieben in EP 214 761, *Pseudomonas Lipase, wie* P. *alcaligenes* und P. *pseudoal- caligenes* Lipase, wie z.B. beschrieben in EP 218 272, P. *cepacia* Lipase, wie z.B. beschrieben in EP 331 376, *P. stutzeri* Lipase, wie z.B. beschrieben in GB 1,372,034, *P. fluorescens* Lipase, *Bacillus Lipase,* z.B. eine *B. subtilis* Lipase (Dartois et al., (1993), Biochemica et Biophysica acta 1131, 253-260), *B. stearo- thermophilus* Lipase (JP 64/744992) und *B. pumilus* Lipase (WO 91/16422).

Weiterhin ist eine Anzahl geklonter Lipasen geeignet, umfassend *Penicillium camembertii* Lipase beschrieben von Yamaguchi et al., (1991), Gene 103, 61-67), *Geotricum candidum* Lipase (Schimada, Y. et al., (1989), J. Biochem., 106, 383-388), und verschiedene *Rhizopus* Lipasen, wie *R. delemar* Lipase (Hass, M. J et al., (1991), Gene 109, 117-113), *R. niveus* Lipase (Kugimiya et al., (1992), Biosci. Biotech. Biochem. 56, 716-719) und *R. oryzae* Lipase.

Weiterhin können andere Typen lipolytischer Enzyme eingesetzt werden, wie Cutinasen, z.B. eine von *Pseudomonas mendocina* abgeleitete Cutinase wie z.B. beschrieben in WO 88/09367, oder eine von *Fusarium solani pisi* abgeleitete cutinase (wie z.B. beschrieben in WO 90/09446).

Speziell geeignete Lipasen sind MI Lipase™, Luma Fast™ und Lipo-max™ (Genencor), Lipoclean™, Lipex™, Lipolex™ Lipolase™ und Lipolase Ultra™ (Novozymes A/S), und Lipase P "Amano" (Amano Pharmaceutical Co. Ltd.).

Die erfindungsgemäßen Zusammensetzungen enthalten vorzugsweise wenigstens eine Lipase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Insbesondere liegt die erfindungsgemäße Zusammensetzung in Form einer enzymhaltigen Folie vor. Dann enthält die erfindungsgemäßen Zusammensetzung vorzugsweise wenigstens eine Lipase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der enzymhaltigen Folie.

### Amylasen:

Grundsätzlich sind alle α- und/oder β-Amylasen geeignet. Geeignete Amylasen können grundsätzlich von Bakterien oder Pilzen stammen. Dazu zählen auch chemisch oder genetisch modifizierte Mutanten.

Beispiele für geeignete Amylasen sind α-Amylasen erhalten aus einem speziellen Stamm von B. amyloliquefaciens oder B. *licheniformis,* detailliert beschrieben in GB 1,296,839. Weitere geeignete Amylasen umfassen Rückgrate, bie bekannt sind als SP707 aus *Bacillus* sp, AP1378, BSG *B. stearothermophilus* alpha-Amylase, SP690, SP722, und AA560 aus *Bacillus* sp. Geeignete kommerziell erhältliche Amylasen sind Stainzyme™, Stainzyme Plus™, Natalase™, Duramyl™, Termamyl™, Fungamyl™ and BAN™ (erhältlich von Novozymes A/S), undRapidase™, PREFERENZ™ S, EXCELLENZ™ S und Maxamyl P™ (erhältlich von Genencor).

Die erfindungsgemäßen Zusammensetzungen enthalten vorzugsweise wenigstens eine Amylase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Insbesondere liegt die erfindungsgemäße Zusammensetzung in Form einer enzymhaltigen Folie vor. Dann enthält die erfindungsgemäßen Zusammensetzung vorzugsweise wenigstens eine Amylase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der enzymhaltigen Folie.

### Cellulasen:

Grundsätzlich sind alle Cellullasen geeignet. Geeignete Cellulasen können grundsätzlich von Bakterien oder Pilzen stammen. Dazu zählen auch chemisch oder genetisch modifizierte Mutanten.

Geeignete Cellulasen sind in der in US 4,435,307 beschrieben. Dabei handelt es sich um pilzliche Cellulasen, hergestellt aus *Humicola insolens.* Speziell geeignet sind Cellulasen mit farbpflegenden Eigenschaften. Beispiele solcher Cellulasen sind in der EP 0 495 257 beschrieben.

Geeignete kommerziell erhältliche Cellulasen umfassen Celluzyme™ hergestellt aus einem Stamm von *Humicola insolens,* Carezyme™, Celluclean™, Endolase™, Whitezyme™ (Novozymes A/S), REVITALENZ™ (Dupont), Biotouch C™ (AB Enzymes) und KAC-500(B)™ (Kao Corporation).

Die erfindungsgemäßen Zusammensetzungen enthalten vorzugsweise wenigstens eine Cellulase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Insbesondere liegt die erfindungsgemäße Zusammensetzung in Form einer enzymhaltigen Folie vor. Dann enthält die erfindungsgemäßen Zusammensetzung vorzugsweise wenigstens eine Cellulase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der enzymhaltigen Folie.

### Peroxidasen / Oxidasen:

Geeignete Peroxidasen / Oxidasen können grundsätzlich von Pflanzen, Bakterien oder Pilzen stammen. Dazu zählen auch chemisch oder genetisch modifizierte Mutanten.

Peroxidase-Enzyme werden in Kombination mit Wasserstoffperoxid oder einer Wasserstoffperoxidquelle (z.B. einem Percarbonat, Perborat oder Persulfat) eingesetzt. Oxidase-Enzyme werden in Kombination mit Sauerstoff eingesetzt. Beide Enzymtypen werden zur "Lösungsbleiche" eingesetzt, um die Farbstoffübertragung von einem gefärbten Gewebe auf ein anderes Gewebe zu vermeiden, wenn diese gemeinsam in einer Flotte gewaschen werden. Der Einsatz kann vorzugsweise mit Wirkverbesserern erfolgen, die z.B. in der WO 94/12621 und WO 95/01426 beschrieben sind.

Die erfindungsgemäßen Zusammensetzungen enthalten vorzugsweise wenigstens eine Peroxidase oder Oxidase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Insbesondere liegt die erfindungsgemäße Zusammensetzung in Form einer enzymhaltigen Folie vor. Dann enthält die erfindungsgemäßen Zusammensetzung vorzugsweise wenigstens eine Peroxidase oder Oxidase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der enzymhaltigen Folie.

### Lyasen:

Grundsätzlich sind alle Lyasen geeignet. Geeignete Lyasen können grundsätzlich von Bakterien oder Pilzen stammen. Dazu zählen auch chemisch oder genetisch modifizierte Mutanten.

Geeignete Lyasen sind Pectatlyasen oder Pectinlyasen. Geeignete kommerziell erhältliche Lyasen umfassen XPect™ (Novozymes A/S) und PREFERENZ™ F (Dupont).

Die erfindungsgemäßen Zusammensetzungen enthalten vorzugsweise wenigstens eine Lyase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Insbesondere liegt die erfindungsgemäße Zusammensetzung in Form einer enzymhaltigen Folie vor. Dann enthält die erfindungsgemäßen Zusammensetzung vorzugsweise wenigstens eine Lyase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der enzymhaltigen Folie.

Erfindungsgemäße Mittel können weitere Enzyme enthalten, die unter dem Begriff Hemicellulasen zusammengefasst werden. Hierzu gehören beispielsweise Mannanasen, Xanthanlyasen, Pektinylasen (= Pektinasen), Pektinesterasen, Xyloglucanasen (=Xylanasen), Pullulanasen und β-Glucanasen.

Die erfindungsgemäßen Zusammensetzungen enthalten vorzugsweise wenigstens eine Hemicellulase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der erfindungsgemäßen Polymerzusammensetzung.

Insbesondere liegt die erfindungsgemäße Zusammensetzung in Form einer enzymhaltigen Folie vor. Dann enthält die erfindungsgemäßen Zusammensetzung vorzugsweise wenigstens eine Hemicellulase in einer Menge von 0.00001 Gew.-% bis 30 Gew.-% Enzymprotein, besonders bevorzugt von 0.0001 Gew.-% bis 15 Gew.-% Enzymprotein, insbesondere 0.001 Gew.-% bis 10 Gew.-% Enzymprotein, spezieller 0.001 Gew.-% bis 5 Gew.-% Enzymprotein, bezogen auf das Gesamtgewicht der enzymhaltigen Folie.

Vorzugsweise enthält das erfindungsgemäße Wasch-, Reinigungs- und Geschirrspülmittel wenigstens ein Enzym, das ausgewählt ist unter Proteasen, Amylasen, Mannanasen, Cellulasen, Lipasen, Pektinlyasen und Mischungen davon.

Vorzugsweise enthält das erfindungsgemäße Wasch-, Reinigungs- und Geschirrspülmittel wenigstens eine Protease und/oder Amylase.

Vorzugsweise enthält das erfindungsgemäße Wasch-, Reinigungs- und Geschirrspülmittel eine Enzymmischung. Bevorzugt sind beispielsweise Enzymmischungen, die folgende Enzyme enthalten oder aus ihnen bestehen:
- Protease und Amylase,
- Protease und Lipase (bzw. lipolytisch wirkenden Enzymen),
- Protease und Cellulase,
- Amylase, Cellulase und Lipase (bzw. lipolytisch wirkenden Enzymen),
- Protease, Amylase und Lipase (bzw. lipolytisch wirkenden Enzymen)
- Protease, Lipase (bzw. lipolytisch wirkenden Enzymen) und Cellulase.

Die Enzyme können an Trägerstoffe adsorbiert sein, um sie gegen vorzeitige Zersetzung zu schützen.

Gegebenenfalls kann das erfindungsgemäße Wasch-, Reinigungs- und Geschirrspülmittel noch Enzymstabilisatoren enthalten. Dazu zählen z.B. Calciumpropionat, Natriumformiat, Borsäuren, Boronsäuren und deren Salze, wie 4-Formylphenylboronsäure, Peptide und Peptidderivate, wie z.B. Peptidaldehyde, Polyole, wie 1,2-Propandiol, und Mischungen davon.

### Komponente d)

Bei den Bleichmitteln d) handelt es sich vorzugsweise um Bleichsysteme, die neben Bleichmitteln gegebenenfalls noch Bleichaktivatoren, Bleichkatalysatoren und/oder Bleichstabilisatoren enthalten.

Geeignete Bleichmittel sind beispielsweise Percarbonsäuren, z.B. Diperoxododecandicarbonsäure, Phthalimidopercapronsäure oder Monoperoxophthalsäure oder-terephthalsäure, Salze der Percarbonsäuren, z.B. Natriumpercarbonat, Addukte von Wasserstoffperoxid an anorganische Salze, z.B. Natriumperborat-Monohydrat, Natriumperborat-Tetrahydrat, Natriumcarbonat-Perhydrat oder Natriumphosphat-Perhydrat, Addukte von Wasserstoffperoxid an organische Verbindungen, z.B. Harnstoff-Perhydrat, oder von anorganischen Peroxosalzen, z.B. Alkalimetallpersulfaten, oder -peroxodisulfaten.

Als Bleichaktivatoren eignen sich beispielsweise polyacylierte Zucker, z.B. Pentaacetylglucose; Acyloxybenzolsulfonsäuren und deren Alkali- und Erdalkaiimetallsalze, z.B. Natrium-p-nonanoyloxybenzolsulfonat oder Natrium-p-benzoyloxybenzolsulfonat; - N,N-diacylierte und N,N,N',N'-tetraacylierte Amine, z.B. N,N,N',N'-Tetraacetylmethylendiamin und -ethylendiamin (TAED), N,N-Diacetylanilin, N,N-Diacetyl-ptoluidin oder 1,3-diacylierte Hydantoine wie 1,3-Diacetyl-5,5-dimethylhydantoin; N-Alkyl-N-sulfonylcarbonamide, z.B. N-Methyl-N-mesylacetamid oder N-MethylN-mesylbenzamid; N-acylierte cyclische Hydrazide, acylierte Triazole oder Urazole, z.B. Monoacetylmaleinsäurehydrazid; O,N,N-trisubstituierte Hydroxylamine, z.B. O-Benzoyl-N,N-succinylhydroxylamin, O-Acetyl-N,N-succinylhydroxylamin oder O,N,N-Triacetylhydroxylamin; N,N'-Diacylsulfurylamide, z.B. N,N'-Dimethyl-N,N'-diacetylsulfurylamid oder N,N'- Diethyl-N,N'-dipropionylsulfurylamid; acylierte Lactame wie beispielsweise Acetylcaprolactam, Octanoylcaprolactam, Benzoylcaprolactam oder Carbonylbiscaprolactam; Anthranilderivate wie z.B. 2-Methylanthranil oder 2-Phenylanthranil; Triacylcyanurate, z.B. Triacetylcyanurat oder Tribenzoylcyanurat; Oximester und Bisoximester wie z.B. O-Acetylacetonoxim oder Bisisopropyliminocarbonat; Carbonsäureanhydride, z.B. Essigsäureanhydrid, Benzoesäureanhydrid, m-Chlorbenzoesäureanhydrid oder Phthalsäureanhydrid; Enolester wie z.B. Isopropenylacetat; 1,3-Diacyl-4,5-diacyloxy-imidazoline, z.B. 1,3-Diacetyl-4,5-diacetoxyimidazolin; Tetraacetylglycoluril und Tetrapropionylglycoluril; diacylierte 2,5-Diketopiperazine, z.B. 1,4-Diacetyl-2,5-diketopiperazin; ammoniumsubstituierte Nitrile wie z.B. N-Methylmorpholiniumacetonitrilmethylsulfat; Acylierungsprodukte von Propylendiharnstoff und 2,2-Dimethyl-propylendihamstoff, z.B. Tetraacetyipropylendihamstoff; α-Acyloxypolyacylmalonamide, z.B. α-Acetoxy-N,N'-diacetylmalonamid; Diacyl-dioxohexahydro-1 ,3,5-triazine, z.B. 1,5-Diacetyl-2,4-dioxohexahydro1,3,5-triazin; Benz-(4H)1,3-oxazin-4-one mit Alkylresten, z.B. Methyl, oder aromatischen Resten z.B. Phenyl, in der 2-Position.

Ein Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise in US-A 5 360 569 und EP-A 453 003 beschrieben sind. Besonders wirksame Bleichkatalysatoren sind Mangankomplexe, die beispielsweise in der WO-A 94/21777 beschrieben sind. Solche Verbindungen werden im Falle ihres Einsatzes in den Wasch- und Reinigungsmitteln höchstens in Mengen bis 1 ,5 Gew.-%, insbesondere bis 0,5 Gew.-%, im Falle von sehr aktiven Mangankomplexen in Mengen bis zu 0,1 Gew.-%, eingearbeitet. Neben dem beschriebenen Bleichsystem aus Bleichmitteln, Bleichaktivatoren und gegebenenfalls Bleichkatalysatoren ist für die erfindungsgemäßen Wasch- und Reinigungsmittel auch die Verwendung von Systemen mit enzymatischer Peroxidfreisetzung oder von photoaktivierten Bleichsystemen möglich.

### Komponente e)

Die erfindungsgemäßen Wasch-, Reinigungs- und Geschirrspülmittel können als Zusatzstoff e) Wasser (= Komponente e1) enthalten. In einer speziellen Ausführungsform enthalten die erfindungsgemäßen Wasch-, Reinigungs- und Geschirrspülmittel 0,1 bis 80 Gew.-% Wasser.

Die erfindungsgemäßen Wasch-, Reinigungs- und Geschirrspülmittel weisen in der Regel bereits aufgrund der enthaltenen Polymerzusammensetzungen in Form von flüssigen und speziell wässrigen Zusammensetzung vorteilhafte rheologische Eigenschaften auf.

Um flüssigen und speziell wässrigen Zusammensetzungen der erfindungsgemäßen Wasch-, Reinigungs- und Geschirrspülmittel die erwünschte Viskosität zu verleihen, kann zusätzlich als Komponente e) wenigstens ein Verdicker (= Komponente e2) eingesetzt werden.

Geeignet sind grundsätzlich jedwede bekannten Verdicker (Rheologiemodifizierungsmittel), sofern sie keinen negativen Einfluss auf die Wirkung des Wasch- und Reinigungsmittels ausüben. Geeignete Verdicker können sowohl natürlichen Ursprungs als auch synthetischer Natur sein.

Beispiele für Verdicker natürlichen Ursprungs sind Xanthan, Johannisbrotkernmehl, Guarmehl, Carrageen, Agar, Tragant, Gummi arabicum, Alginate, modifizierte Stärken, wie Hydroxyethylstärke, Stärkephosphatester oder Stärkeacetate, Dextrine, Pektine und Cellulosederivate, wie Carboxymethylcellulose, Hydroxyethylcellulose, Hydroxypropylcellulose, Hydroxypropylmethylcellulose, Methylcellulose und dergleichen.

Verdicker natürlichen Ursprungs sind auch anorganische Verdicker, wie Polykieselsäuren und Tonmineralien, z.B. Schichtsilikate, wie auch die bei den Buildern genannten Silikate.

Beispiele für synthetische Verdicker sind Polyacryl- und Polymethacrylverbindungen, wie (teil)vernetzte Homopolymere der Acrylsäure, beispielsweise mit einem Allylether von Saccharose oder Pentaerythrit oder mit Propylen vernetzte Homopolymere der Acrylsäure (Carbomer), z.B. die Carbopol®-Marken von BF Goodridge (z.B. Carbopol® 676, 940, 941, 934 und dgl.) oder die Polygel®-Marken von 3V Sigma (z.B. Polygel® DA), Copolymere ethylenisch ungesättigter Mono- oder Dicarbonsäuren, beispielsweise Terpolymere von Acrylsäure, Methacrylsäure oder Maleinsäure mit Methyl- oder Ethylacrylat und einem (Meth)Acrylat, das sich von langkettigen ethoxylierten Alkoholen ableitet, beispielsweise die Acusol®-Marken von Rohm & Haas (z.B. Acusol® 820 oder 1206A), Copolymere von zwei oder mehr Monomeren, die ausgewählt sind unter Acrylsäure, Methacrylsäure und ihren C₁-C₄-Alkylestern, z.B. Copolymere von Methacrylsäure, Butylacrylat und Methylmethacrylat oder von Butylacrylat und Methylmethacrylat, z.B. die Aculyn®- und Acusol®-Marken von Rohm & Haas (z.B. Aculyn® 22, 28 oder 33 und Acusol® 810, 823 und 830), oder vernetzte hochmolekulare Acrylsäurecopolymere, beispielsweise mit einem Allylether von Saccharose oder Pentaerythrit vernetzte Copolymere von C₁₀-C₃₀-Alkylacrylaten mit einem oder mehreren Comonomeren, die ausgewählt sind unter Acrylsäure, Methacrylsäure und ihren C₁-C₄-Alkylestern (z.B. Carbopol® ETD 2623, Carbopol® 1382 oder Carbopol® AQUA 30 von Rohm & Haas).

Beispiele für synthetische Verdicker sind weiterhin Umsetzungsprodukte von Maleinsäurepolymeren mit ethoxylierten langkettigen Alkoholen, z.B. die Surfonic L-Serie von Texaco Chemical Co. oder Gantrez AN-119 von ISP; Polyethylenglykole, Polyamide, Polyimine und Polycarbonsäuren.

Geeignet sind auch Gemische der o.g. Verdicker.

Bevorzugte Verdicker sind Xanthane und die oben genannten Polyacryl- und Polymethacrylverbindungen

Die erfindungsgemäßen Wasch-, Reinigungs- und Geschirrspülmittel können als Zusatzstoff e) wenigstens einen weiteren Zusatzstoff enthalten.

Tenside aus der Gruppe e3), die von Komponente a) verschieden sind, können kationisch, anionisch, zwitterionisch oder nichtionisch sein. Geeignete Tenside sind die zuvor bei den Tensidsystemen genannte.

Geeignete organische Lösungsmittel e3) sind ausgewählt unter ein- oder mehrwertigen Alkoholen, Alkanolaminen oder Glykolethern. Vorzugsweise sind sie ausgewählt unter Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propylether, Dipropylenglykolmonomethyl- oder - ethylether, Di-isopropylenglykolmonomethyl- oder -ethylether, Methoxy-, Ethoxy- oder Butoxytriglykol, i-Butoxy-ethoxy-2-propanol, 3-Methyl-3-methoxybutanol, Propylen-glykol-t-butylether sowie Mischungen dieser Lösemittel.

Als Schauminhibitoren oder Entschäumer der Komponente e3) kommen beispielsweise Seifen, Paraffine oder Silikonöle in Betracht, die gegebenenfalls auf Trägermaterialien aufgebracht sein können.

Geeignete Basen der Komponente e3) sind Alkalimetallhydroxide, Erdalkalimetallhydroxide, Alkalimetallcarbonate, Erdalkalimetallcarbonate, Ammoniumcarbonat Alkalimetallhydrogencarbonate, Erdalkalimetallhydrogencarbonate, Ammoniumhydrogencarbonat und Mischungen davon. Vorzugsweise werden Na-, Li- und Mg-Carbonate bzw. -Hydrogencarbonate, insbesondere Natriumcarbonat und/oder Natriumhydrogencarbonat, eingesetzt.

Zusätzlich können die erfindungsgemäßen Wasch-, Reinigungs- oder Geschirrspülmittel weitere Zusatzstoffe e) enthalten, die die anwendungstechnischen und/oder ästhetischen Eigenschaften weiter verbessern. In der Regel enthalten bevorzugte Mittel zusätzlich zu den zuvor genannten Komponenten wenigstens einen weiteren Zusatzstoff, der ausgewählt ist unter Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln sowie UV-Absorbern.

Um den ästhetischen Eindruck der erfindungsgemäßen Wasch-, Reinigungs- oder Geschirrspülmittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

### I & I-Reiniger

Die erfindungsgemäßen Polymerzusammensetzungen eignen sich speziell für industrielle und institutionelle Reiniger (I & I-Reiniger). Industrielle und institutionelle Reiniger sind typischerweise Waschmittel, Allzweckreiniger, Schaumreiniger, Gelreiniger, CIP-Reiniger (cleaning in place-Reiniger) für professionelle und in der Regel automatisierte Reinigungsvorgänge, z. B. in Großwäschereien, Molkereien, Brauereien, der Nahrungsmittel- oder Getränkeindustrie, der Pharmaindustrie bzw. der Galenik oder Sanitärreiniger.

Die Reiniger können stark basisch mit hohem Elektrolytgehalt sein und bei Bedarf Bleichmittel (wie Wasserstoffperoxid, Natriumhypochlorit) oder Desinfektionsmittel sowie Entschäumer enthalten (z. B. in der Flaschenreinigung). Auch die gängigen zuvor genannten Enzyme können in den industriellen und institutionellen Reinigern enthalten sein. Hinsichtlich der Reinigungsarten, für die sich die erfindungsgemäßen Formulierungen eignen, herrscht große Vielfalt. Beispielhaft seien Reinigungsbäder (stationär oder bewegt), Sprühreinigung, Ultraschall-, Dampfstrahl- und Hochdruckreinigung erwähnt, gegebenenfalls in Kombination mit mechanischer Reinigung, z. B. durch rotierende Bürsten.

Die genannten Formulierungen für die Reinigung schließen solche für die Industrie, das Verkehrswesen, Handel und Gewerbe und für den privaten Sektor ein. Im einzelnen seien beispielhaft genannt: Professionelle Wäschereien, professionelles Reinigungsgewerbe, erzaufbereitende Industrie, Metall- und metallverarbeitende Industrie, Automobil- und Automobilzulieferindustrie, Elektroindustrie, Elektronikindustrie, Photoindustrie und -gewerbe, Freizeitindustrie und -gewerbe, Baustoffindustrie, Brauindustrie und -gewerbe; Nahrungsmittelindustrie (z. B. Verarbeitung oder Herstellung von Fleisch-, Geflügel-Milch- Fischprodukten) Tiernahrungsmittelindustrie, Kosmetika-Industrie, Pharmaindustrie, Agroindustrie, Gastronomie, Gesundheitswesen, Handwerksbetriebe, und öffentliches Verkehrswesen. Beispiele für zu reinigende Objekte sind Anstaltswäsche, Klinikwäsche, Wäsche aus Wäschereiannahmen, Gebäude mit Wohn-, Büro- oder Geschäftsräumen der verschiedensten Art sowie Sanitärräumen, Lagerhäuser, Brauereien, Einzelhandelsgeschäfte, wie Bäckereien, Metzgereien und Supermärkte; Krankenhäuser, Pflegeheime, Altersheime, Verwaltungsgebäude, Fabrikgebäude, Arztpraxen; weiterhin Kraftfahrzeuge (PKW und LKW), Autobusse, Straßentankfahrzeuge (innen und außen), Eisenbahnkesselwagen, Personen- und Güterwagen sowie Luftfahrzeuge und Schiffe; ferner Gebäudefassaden, gekachelte oder gestrichene Wände, Fußböden aus Holz (Parkett, Dielen) mit Estrich oder textilen oder Kunststoffbelägen, Signal- und Beleuchtungseinrichtungen, Möbel, Geländer, Schildbrücken, Schilder, Warnbaken, Begrenzungspfähle, Kessel, Geschirr, Glasscheiben, Straßen und Wege, Hofbefestigungen, Straßen- und Eisenbahntunnel.

### Folienanwendungen

Ein weiterer Gegenstand der Erfindung ist ein Verfahren zur Herstellung einer erfindungsgemäßen Polymerzusammensetzung in Form einer Folie, bei dem man ein nach dem erfindungsgemäßen Verfahren erhältliches Polymerisat, gegebenenfalls nach Zugabe wenigstens eines Wirkstoffs und/oder wenigstens eines Zusatzstoffes, einer Folienbildung unterzieht, vorzugsweise ausgewählt unter Blasformen, Thermoformen, Gießen und Kalandrieren.

Bezüglich geeigneter und bevorzugter Polymerzusammensetzungen für Folien wird auf die vorherigen Ausführungen in vollem Umfang Bezug genommen.

Wie zuvor ausgeführt, werden für den Einsatz in Form einer Folie vorzugsweise flexible erfindungsgemäße Polymerzusammensetzungen mit einer niedrigen Glasübergangstemperatur T_{G} eingesetzt. Vorzugsweise weisen die erfindungsgemäßen Polymerzusammensetzungen für den Einsatz in Form einer Folie eine Glasübergangstemperatur T_{G} im Bereich von 0 bis 50°C, vorzugsweise von 5 bis 20°C, auf.

Eine spezielle Ausführung ist ein Verfahren zur Herstellung einer erfindungsgemäßen Polymerzusammensetzung in Form einer Folie, die wenigstens einen Wirkstoff enthält. Geeignete Wirkstoffe sind die zuvor genannten als Koponenten b) bis e) genannten, worauf hier in vollem Umfang Bezug genommen wird. Bevorzugt ist der Wirkstoff ausgewählt unter Enzymen, Buildern, Bleichmitteln, Tensiden, Basen, Korrosionsinhibitoren, Entschäumern, Farbstoffen, Duftstoffen, Füllstoffen, Tablettierhilfsmitteln, Desintegrationsmiteln, Verdickern, Löslichkeitsvermittlern, organischen Lösemitteln, Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln, UV-Absorbern und Mischungen davon.

Eine ganz spezielle Ausführung ist ein Verfahren zur Herstellung einer erfindungsgemäßen Polymerzusammensetzung in Form einer Folie, die wenigstens ein Enzym enthält. Geeignete Enzyme sind die zuvor als Koponenten c) genannten, worauf hier in vollem Umfang Bezug genommen wird.

Zur Herstellung von Folien können der erfindungsgemäßen Polymerzusammensetzung Zusatzstoffe vor und/oder während der Folienherstellung zugesetzt werden. Die Zusatzstoffe zur Herstellung einer erfindungsgemäßen Folie sind vorzugsweise ausgewählt unter Weichmachern, Mitteln zur Modifikation der Gasdurchlässigkeit und Wasserdampf-Durchlässigkeit, Antistatikmitteln, Gleitmitteln, Slipmitteln, Auflösungshilfsmitteln, Farbstoffen, Pigmenten und Mischungen davon. In einer speziellen Ausführung wird dem Polymer-Material wenigstens ein Weichmacher zugesetzt, der der Folie und daraus gefertigten Umhüllungen dauerhafte Flexibilität verleiht. Geeignete Weichmacher sind Alkylenglycole, Oligoalkylenglycole, davon verschiedene Polyole, Alkylenimine, Oligoalkylenimine und Mischungen davon. Bevorzugt ist der Weichmacher ausgewählt unter Glycerin, Propylenglycol, Diethylenglycol, Triethylenglycol, Sorbitol, Diethylenimin, Triethylenimin, und Mischungen davon.

Grundsätzlich unterliegt das Folienherstellungsverfahren keinen besonderen Beschränkungen und der Fachmann kann unter Verwendung einer geeigneten erfindungsgemäßen Polymerzusammensetzung jedes beliebige Herstellungsverfahren anwenden, das im aufgrund seiner Fachkenntnis bekannt ist. Das gleiche gilt zur Herstellung von Umhüllungen auf Basis einer erfindungsgemäßen Folie. Besonders geeignet sind Extrusionsverfahren und Giessverfahren.

Bei der Herstellung der Folien durch Extrusion wird beispielsweise eine nach dem erfindungsgemäßen Verfahren erhältliche Polymerzusammensetzung, gegebenenfalls nach Zugabe wenigstens eines Wirkstoffs und/oder wenigstens eines Zusatzstoffes, extrudiert, in einem Blasverfahren zu einer Folie geblasen oder in Thermoformverfahren zu einer Folie geformt, und gegebenenfalls die so erhaltene Folie in eine für das Umhüllen von Waschmittel-, Spülmittel- oder Reinigungsmittel-Portionen geeignete Form gebracht.

Bei der Herstellung der Folien durch Gießen wird beispielsweise eine nach dem erfindungsgemäßen Verfahren erhältliche Polymerzusammensetzung, gegebenenfalls nach Zugabe wenigstens eines Zusatzstoffes, aufgeschmolzen oder in einem geeigneten Lösungsmittel oder Lösungsmittelgemisch gelöst, die so erhaltene fließfähige Polymerzusammensetzung zu einer Folie ausgegossen, und gegebenenfalls das Lösungsmittel oder Lösungsmittelgemisch durch Verdampfen entfernt.

Geeignete Lösungsmittel und Lösungsmittelgemische sind die zuvor als Komponente LM) beschriebenen, worauf hier in vollem Umfang Bezug genommen wird. Das Lösungsmittel ist besonders bevorzugt ausgewählt unter Wasser, Ethanol, n-Propanol, Isopropanol, Ethylenglycol, Diethylenglycol, 1,2-Propylenglycol, 1,2-Dipropylenglycol und Mischungen davon. In einer speziellen Ausführungsform wird als Lösungsmittel Wasser oder ein Gemisch aus Wasser und wenigstens einem von Wasser verschieden Lösungsmittel eingesetzt, ausgewählt unter Ethanol, n-Propanol, Isopropanol, Ethylenglycol, Diethylenglycol, 1,2-Propylenglycol, 1,2-Dipropylenglycol und Mischungen davon.

Die erfindungsgemäßen Polymerzusammensetzungen sind in der Regel thermoplastisch und können einer Umformung durch Thermoformen (d.h. Warmformen, Tiefziehen oder Vakuumtiefziehen) unterzogen werden. Ein Verfahren zur Herstellung von wasserlöslichen Folienverpackungen durch ein Thermoformverfahren, das einen Warmform- oder Tiefziehschritt umfasst, ist in der WO 00/55044 beschrieben.

Zur Herstellung von Folienportionen kann das Folienmaterial in geeigneter Weise konfektioniert werden, z.B. durch Schneiden in eine geeignete Größe und/oder Falten zur Bildung von Kompartimenten. Anschließend können die Kanten nach üblichen Versiegelungsverfahren, wie Heißsiegeln, Flüssigsiegeln oder Drucksiegeln verschlossen werden.

### Beschichtungen

Ein weiterer Gegenstand der Erfindung ist ein Verfahren zur Herstellung einer erfindungsgemäßen Polymerzusammensetzung in Form einer Beschichtung auf einem Substrat, bei dem man ein nach dem erfindungsgemäßen Verfahren erhältliches Polymerisat, gegebenenfalls nach Zugabe wenigstens eines Wirkstoffes und/oder wenigstens eines Zusatzstoffes und/oder wenigstens eines Lösungsmittels und gegebenenfalls unter Erwärmen, in eine fließfähige Form bringt, auf ein Substrat aufträgt und verfestigen lässt.

Bezüglich geeigneter und bevorzugter Polymerzusammensetzungen für Beschichtungen auf einem Substrat wird auf die vorherigen Ausführungen in vollem Umfang Bezug genommen.

Wie zuvor ausgeführt, werden für den Einsatz in Form einer Beschichtung vorzugsweise flexible erfindungsgemäße Polymerzusammensetzungen mit einer niedrigen Glasübergangstemperatur Tc eingesetzt. Vorzugsweise weisen die erfindungsgemäßen Polymerzusammensetzungen für den Einsatz in Form einer Beschichtung eine Glasübergangstemperatur T_{G} im Bereich von 0 bis 50°C, vorzugsweise von 5 bis 20 °C, auf.

Eine spezielle Ausführung ist ein Verfahren zur Herstellung einer erfindungsgemäßen Polymerzusammensetzung in Form einer Beschichtung auf einem Substrat, die wenigstens einen Wirkstoff enthält. Geeignete Wirkstoffe sind die zuvor genannten als Koponenten b) bis e) genannten, worauf hier in vollem Umfang Bezug genommen wird. Bevorzugt ist der Wirkstoff ausgewählt unter Enzymen, Buildern, Bleichmitteln, Tensiden, Basen, Korrosionsinhibitoren, Entschäumern, Farbstoffen, Duftstoffen, Füllstoffen, Tablettierhilfsmitteln, Desintegrationsmiteln, Verdickern, Löslichkeitsvermittlern, organischen Lösemitteln, Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln, UV-Absorbern und Mischungen davon.

Eine ganz spezielle Ausführung ist ein Verfahren zur Herstellung einer erfindungsgemäßen Polymerzusammensetzung in Form einer Beschichtung auf einem Substrat, die wenigstens ein Enzym enthält. Geeignete Enzyme sind die zuvor als Koponenten c) genannten, worauf hier in vollem Umfang Bezug genommen wird.

Zusatzstoffe zur Herstellung von Beschichtungen können der erfindungsgemäßen Polymerzusammensetzung vor und/oder während der Beschichtungsherstellung zugesetzt werden. Die Zusatzstoffe zur Herstellung einer erfindungsgemäßen Beschichtung sind vorzugsweise ausgewählt unter den zuvor zur Folienbildung genannten. Darauf wird in vollem Umfang Bezug genommen.

Geeignete Lösungsmittel und Lösungsmittelgemische zur Herstellung von Beschichtungen sind die zuvor als Komponente LM) beschriebenen, worauf hier in vollem Umfang Bezug genommen wird. Das Lösungsmittel ist besonders bevorzugt ausgewählt unter Wasser, Ethanol, n-Propanol, Isopropanol, Ethylenglycol, Diethylenglycol, 1,2-Propylenglycol, 1,2-Dipropylenglycol und Mischungen davon. In einer speziellen Ausführungsform wird als Lösungsmittel Wasser oder ein Gemisch aus Wasser und wenigstens einem von Wasser verschieden Lösungsmittel eingesetzt, ausgewählt unter Ethanol, n-Propanol, Isopropanol, Ethylenglycol, Diethylenglycol, 1,2-Propylenglycol, 1,2-Dipropylenglycol und Mischungen davon.

Die erfindungsgemäße Polymerzusammensetzung eignet sich zur Herstellung von Beschichtungen auf Substraten, wie Waschmittel-, Reinigungsmittel- oder Geschirrspülmitteltabletten. Die erhaltenen Beschichtungen sind unter den Anwendungsbedingungen wasserlöslich. Die Beschichtungen sind nicht brüchig und fühlen sich in der Regel angenehm glatt an.

Zum Auftragen der Beschichtung auf ein Substrat kann die erfindungsgemäße Polymerzusammensetzung in geschmolzener Form oder gelöst in einem Lösungsmittel oder Lösungsmittelgemisch eingesetzt werden. Geeignete Auftragverfahren sind dem Fachmann prinzipiell bekannt und umfassen übliche Sprüh- und Tauchauftragsverfahren. Bevorzugt ist der Sprühauftrag. Dazu kann die Polymerzusammensetzung in geschmolzener Form eingesetzt werden. Bevorzugt liegt die Polymerzusammensetzung für den Sprühauftrag gelöst in einem Lösungsmittel oder Lösungsmittelgemisch vor. Abschließend kann das Lösungsmittel oder Lösungsmittelgemisch durch Verdampfen entfernt werden.

### Klebstoffzusammensetzung

Die erfindungsgemäße Polymerzusammensetzung eingnet sich zur Herstellung von Klebstoffzusammensetzung die adhäsiv sind, d.h. sie sind in der Lage, Substrate zu verbinden, ohne dass die Substrate selbst merklich verändert werden, wobei der Zusammenhalt der verbundenen Substrate durch Adhäsionskräfte (Anziehungskräfte zwischen Adhäsiv und Substrat) und Kohäsion (innerer Zusammenhalt des Adhäsive) bestimmt wird.

Polymerzusammensetzungen zur Herstellung einer Klebstoffzusammensetzung werden erhalten durch radikalische Polymerisation einer Monomerzusammensetzung M), die wenigstens eine α,β-ethylenisch ungesättigte Carbonsäure A), wie zuvor definiert, enthält. Bevorzugte Monomere A) sind ausgewählt unter Acrylsäure, Methacrylsäure, Itaconsäure, Maleinsäure oder Fumarsäure. Die Säuregruppen können in protonierter Form oder in Form ihrer Salze vorliegen. Bevorzugt enthält die Monomerzusammensetzung 0,5 bis 15 Gew.-%, besonders bevorzugt 1 bis 10 Gew.-%, bezogen auf das Gesamtgewicht der Monomerzusammensetzung, wenigstens einer α,β-ethylenisch ungesättigte Carbonsäure A). Besonders bevorzugt ist Acrylsäure.

Geeignete Comonomere sind z.B. (Meth)acrylsäurealkylester mit einem C₁-C₁₀-Alkylrest, wie Methylmethacrylat, Methylacrylat, n-Butylacrylat, Ethylacrylat und Ethylhexylacrylat. Insbesondere sind auch Mischungen der (Meth)acrylsäurealkylester geeignet. Vinylester von Carbonsäuren mit 1 bis 20 C-Atomen sind z.B. Vinyllaurat, Vinylstearat, Vinylpropionat, Versaticsäurevinylester und Vinylacetat. Als vinylaromatische Verbindungen kommen Vinyltoluol, α- und p-Methylstyrol, α-Butylstyrol, 4-n-Butylstyrol, 4-n-Decylstyrol und vorzugsweise Styrol in Betracht. Beispiele für Nitrile sind Acrylnitril und Methacrylnitril. Die Vinylhalogenide sind mit Chlor, Fluor oder Brom substituierte ethylenisch ungesättigte Verbindungen, bevorzugt Vinylchlorid und Vinylidenchlorid. Als Vinylether zu nennen sind z.B. Vinylmethylether oder Vinylisobutylether. Bevorzugt sind Vinylether von 1 bis 4 C-Atome enthaltenden Alkoholen. Geeignete Kohlenwasserstoffe mit 4 bis 8 C- Atomen und zwei olefinischen Doppelbindungen sind zum Beispiel Butadien, Isopren und Chloropren. Weitere Monomere sind z.B. auch Hydroxylgruppen enthaltende Monomere, insbesondere C₁-C₁₀-Hydroxyalkyl(meth)acrylate oder (Meth)acrylamid. Als weitere Monomere seien darüber hinaus Phenyloxyethylglykolmono-(meth)acrylat, Glycidyl(meth)acrylat, Aminoalkyl(meth)acrylate wie z.B. 2-Aminoethyl-(meth)acrylat genannt. Alkylgruppen weisen vorzugsweise von 1 bis 20 C-Atome auf. Als weitere Monomere seien auch vernetzende Monomere genannt.

Bevorzugt als Polyetherkomponente PE) sind die zuvor genannten Polyetherole und speziell Polyethylenglycole.

Die Art und Menge der Monomere bzw. die Verhältnisse verschiedener Comonomere zueinander sind dabei derart, dass die Glasübergangstemperatur in dem zuvor genannten Bereich liegt.

Die erfindungsgemäßen Klebstoffzusammensetzungen können wenigstens ein Lösungs- bzw. Dispergiermittel enthalten. Das Lösungs- bzw. Dispergiermittel der Klebstoffzusammensetzung kann sowohl nur aus Wasser, als auch aus Mischungen aus Wasser und damit mischbaren Flüssigkeiten, wie Methanol oder Ethanol, bestehen. Vorzugsweise wird nur Wasser verwendet. Der pH-Wert der Klebstoffzusammensetzung wird vorzugsweise auf pH größer 4,5, insbesondere auf einen pH-Wert zwischen 5 und 9,5 eingestellt.

Die Klebstoffzusammensetzungen können allein aus dem Lösungsmittel und der Polymerzusammensetzung bestehen. Die Klebstoffzusammensetzung kann jedoch auch noch weitere Zusatzstoffe enthalten, z.B. Füllstoffe, Farbstoffe, Verlaufsmittel, Verdicker (vorzugsweise Assoziativverdicker), Entschäumer, Pigmente, Netzmittel oder Tackifier (klebrigmachende Harze). Für eine bessere Benetzung von Oberflächen können die Klebstoffe Benetzungshilfsmittel, z. B. Fettalkoholethoxylate, Alkylphenolethoxylate, Nonylphenolethoxylate, Polyoxyethylene, Polyoxypropylene oder Natriumdodecylsulfonate enthalten. Die Menge an Zusatzstoffen beträgt im allgemeinen 0,05 bis 5 Gew.-Teile, insbesondere 0,1 bis 3 Gew.-Teile auf 100 Gew.-Teile Polymerzusammensetzung (fest).

In einer Ausführungsform ist die Klebstoffzusammensetzung im Wesentlichen frei von Weichmachern. Weichmacher sind Zusätze, welche die Klebekraft herabsetzen. Im Wesentlichen frei von Weichmachern bedeutet, dass die Zusammensetzungen weniger als 1 Gew.%, vorzugsweise weniger als 0,5 Gew.%, bezogen auf die Gesamtzusammensetzung an Weichmachern, besonders bevorzugt keinen Weichmacher enthalten.

Die erfindungsgemäße Klebstoffzusammensetzung ist vorzugsweise ein Haftklebstoff. Ein Haftklebstoff ist ein viskoelastischer Klebstoff, dessen abgebundener Film bei Raumtemperatur (20°C) in trockenem Zustand permanent klebrig und klebfähig bleibt. Die Klebung auf Substraten erfolgt sofort durch leichten Anpressdruck.

Die erfindungsgemäße Klebstoffzusammensetzung kann zur Verbindung wenigstens zweier Substrate oder zur Herstellung von selbstklebenden Artikeln verwendet werden. Die zu verbindenden Substrate oder selbstklebenden Artikel sind zumindest teilweise mit dem Haftklebstoff beschichtet. Vorzugsweise sind die selbstklebenden Artikel nach der Verklebung wiederabziehbar. Bei den selbstklebenden Artikeln kann es sich z.B. um Folien, Bänder oder Etiketten handeln. Geeignete Trägermaterialien sind z.B. Papier, Kunststofffolien und Metallfolien. Bei erfindungsgemäßen selbstklebenden Bändern kann es sich um einseitig oder beidseitig beschichtete Bänder aus den obigen Substanzen handeln. Bei erfindungsgemäßen selbstklebenden Etiketten kann es sich um Etiketten aus Papier oder einer thermoplastischen Folie handeln. Als thermoplastische Folie kommen z.B. Folien aus Polyolefinen (z.B. Polyethylen, Polypropylen), Polyole- fincopolymeren, Folien aus Polyestern (z.B. Polyethylenterephtalat) oder Polyacetat in Betracht. Die Oberflächen der thermoplastischen Polymerfolien sind vorzugweise coronabehandelt. Die Etiketten sind einseitig mit Klebstoff beschichtet. Bevorzugte Substrate für die selbstklebenden Artikel sind Papier und Polymerfolien. Bevorzugte selbstklebende Artikel sind Papieretiketten Die Artikel sind auf mindestens einer Oberfläche zumindest teilweise mit einer erfindungsgemäßen Klebstoffzusammensetzung beschichtet. Der Klebstoff kann nach üblichen Methoden wie Rakeln oder Streichen auf die Artikel aufgetragen werden. Die Auftragsmenge beträgt bevorzugt 0,1 bis 20 g, besonders bevorzugt 2 bis 15 g Feststoff pro m². Nach dem Auftragen kann ein Trocknungsschritt zur Entfernung des Wasser bzw. der Lösungsmittel folgen. Bei den Substraten, die miteinander verbunden werden oder auf die die selbstklebenden Artikel vorteilhaft aufgebracht werden können, kann es sich z.B. um Metall, Holz, Glas, Papier oder Kunststoff handeln. Die selbstklebenden Artikel eignen sich insbesondere zum Verkleben auf Verpackungsoberflächen, Kartons, Kunststoffverpackungen, Bücher, Fenster, Kraftfahrzeugkarosserien oder Karosserieteilen. Die selbstklebenden Artikel spezieller Ausführungsformen können von den Gegenständen von Hand wieder abgezogen werden, ohne dass ein Klebstoffrückstand auf dem Gegenstand verbleibt. Die Haftung auf den Gegenständen ist gut, trotzdem lassen sich die Folien, Bänder und Etiketten leicht abziehen.

### Kosmetische und pharmazeutische Mittel

Die erfindungsgemäßen Polymerzusammensetzungen eignen sich vorzugsweise zur Formulierung von kosmetischen und pharmazeutischen Produkten, speziell von wässrigen kosmetischen und pharmazeutischen Produkten.

Ein weiterer Gegenstand der Erfindung ist eine kosmetische oder pharmazeutische Zusammensetzung, enthaltend
a) wenigstens eine erfindungsgemäße Polymerzusammensetzung, wie zuvor definiert,
b) wenigstens einen kosmetisch oder pharmazeutisch akzeptablen Wirkstoff, und
c) gegebenenfalls wenigstens einen von den Komponenten a) und b) verschiedenen kosmetisch oder pharmazeutisch akzeptablen Hilfsstoff.

Bevorzugt umfasst die Komponente c) wenigstens einen kosmetisch oder pharmazeutisch akzeptablen Träger.

Vorzugsweise ist die Träger-Komponente c) ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Geeignete hydrophile Komponenten c) sind die zuvor genannten organischen Lösungsmittel, Öle und Fette.

Speziell geeignete kosmetisch verträgliche Öl- bzw. Fettkomponenten c) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Bei den erfindungsgemäßen kosmetischen Mitteln kann es sich um hautkosmetische, haarkosmetische, dermatologische, hygienische oder pharmazeutische Mittel handeln. Die erfindungsgemäße Polymerzusammensetzung eignet sich speziell auch zur Formulierung von Gelen.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden. Die erfindungsgemäße Polymerzusammensetzung eignet sich speziell zur Formulierung von Gelen

Die erfindungsgemäßen kosmetischen Mittel können zusätzlich kosmetisch und/oder dermatologisch aktive Wirk- und Effektstoffe sowie Hilfsstoffe enthalten. Vorzugsweise enthalten die erfindungsgemäßen kosmetischen Mittel wenigstens eine erfindungsgemäße Polymerzusammensetzung, wie vorstehend definiert, wenigstens einen wie vorstehend definierten Träger C) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

Zusätzlich zu den erfindungsgemäßen Polymerzusammensetzungen können die kosmetischen Mittel wenigstens ein konventionelles Verdickungsmittel enthalten. Dazu zählen z.B. Polysaccharide und organische Schichtmineralien wie Xanthan Gum® (Kelzan® der Fa. Kelco), Rhodopol® 23 (Rhone Poulenc) oder Veegum® (Firma R. T. Vanderbilt) oder Attaclay® (Firma Engelhardt). Geeignete Verdickungsmittel sind auch organische natürliche Verdickungsmittel (Agar-Agar, Carrageen, Tragant, Gummi arabicum, Alginate, Pektine, Polyosen, Guar-Mehl, Johannisbrotbaumkernmehl, Stärke, Dextrine, Gelatine, Casein) und anorganische Verdickungsmittel (Polykieselsäuren, Tonmiheralien wie Montmorillonite, Zeolithe, Kieselsäuren). Weitere Verdickungsmittel sind Polysaccharidgummen, beispielsweise Gummi arabicum, Agar, Alginate, Carrageene und ihre Salze, Guar, Guaran, Traganth, Gellan, Ramsan, Dextran oder Xanthan und ihre Derivate, z. B. propoxyliertes Guar, sowie ihre Mischungen. Andere Polysaccharidverdicker sind beispielsweise Stärken verschiedensten Ursprungs und Stärkederivate, z. B. Hydroxyethylstärke, Stärkephosphat- ester oder Stärkeacetate, oder Carboxymethylcellulose bzw. ihr Natriumsalz, Methyl-, Ethyl-, Hydroxyethyl-, Hydroxypropyl-, Hydroxypropyl-methyl- oder Hydroxyethyl-methyl- cellulose oder Celluloseacetat. Als Verdickungsmittel können weiterhin Schichtsilikate eingesetzt werden. Hierzu zählen beispielsweise die unter dem Handelsnamen Laponite® erhältlichen Magnesium-oder Natrium-Magnesium-Schichtsilikate der Firma Solvay Alkali sowie die Magnesiumsilikate der Firma Süd-Chemie.

Geeignete kosmetisch und/oder dermatologisch aktive Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-Bund/oder UV-A-Bereich absorbieren. Geeignete UV-Filter sind die zuvor genannten. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeere, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen Wirkstoff (wie auch gegebenenfalls als Hilfsstoff) wenigstens ein kosmetisch oder pharmazeutisch akzeptables Polymer enthalten. Dazu zählen ganz allgemein anionische, kationische, amphotere und neutrale Polymere.

Beispiele für anionische Polymere sind Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der (Meth)acrylsäure, C₄-C₃₀-Alkylvinylester, C₄-C₃₀-Alkylvinylether und Hyaluronsäure. Ein Beispiel für ein anionisches Polymer ist weiterhin das unter der Bezeichnung Luviset® Shape (INCI Name: Polyacrylate-22) erhältliche Methylmethacrylat/Methacrylsäure/Acrylsäure/Urethanacrylat-Copolymer. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und -R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviset Clear®, Luviquat Supreme®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF SE), oder Polyvinylpyrrolidon und deren Copolymere, insbesondere mit Vinylestern, wie Vinylacetat, z. B. Luviskol® VA 37, VA 55, VA 64, VA 73 (BASF SE); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. - Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

In einer speziellen Ausführung enthalten die erfindungsgemäßen Zusammensetzungen wenigstens ein Polymer, das als Verdicker wirkt.

Geeignete polymere Verdicker sind beispielsweise gegebenenfalls modifizierte polymere Naturstoffe (Carboxymethylcellulose und andere Celluloseether, Hydroxyethyl- und -propylcellulose und dergleichen) sowie synthetische polymere Verdicker (Polyacryl- und Polymethacryl-Verbindungen, Vinylpolymere, Polycarbonsäuren, Polyether, Polyimine, Polyamide). Dazu zählen die zum Teil bereits zuvor genannten Polyacryl- und Polymethacryl-Verbindungen, beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung: Carbomer). Solche Polyacrylsäuren sind u. a. von der Fa. BF Goodrich unter dem Handelsnamen Carbopol® erhältlich, z. B. Carbopol 940 (Molekulargewicht ca. 4000000 Dalton), Carbopol 941 (Molekulargewicht ca. 1250000 Dalton) oder Carbopol 934 (Molekulargewicht ca. 3000000 Dalton). Weiterhin fallen darunter Acrylsäure-Copolymere, die beispielsweise von der Fa. Rohm & Haas unter den Handelsnamen Aculyn® und Acusol® erhältlich sind, z. B. die anionischen, nichtassoziativen Polymere Aculyn 22, Aculyne 28, Aculyn 33 (vernetzt), Acusol 810, Acusol 823 und Acusol 830 (CAS 25852-37-3). Speziell geeignet sind auch assoziative Verdicker, z.B. auf Basis modifizierter Polyurethane (HEUR) oder hydrophob modifizierter Acryl- oder Methacrylsäure-Copolymere (HASE-Verdicker, High Alkali Swellable Emulsion).

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die erfindungsgemäßen Polymerzusammensetzungen verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen erfindungsgemäßen Polymerzusammensetzungen zeigen vorteilhafte Wirkungen. Die Polymere können unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens eine erfindungsgemäße Polymerzusammensetzung in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den erfindungsgemäßen Polymerzusammensetzungen und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäße Polymerzusammensetzung auch mit herkömmlichen Polymeren, wie zuvor beschrieben, abmischen, falls spezielle Eigenschaften eingestellt werden sollen.

Zur Einstellung bestimmter Eigenschaften wie z. B. Verbesserung der Haptik, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser-(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einer erfindungsgemäßen Polymerzusammensetzung in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karite-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Es können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens eine erfindungsgemäße Polymerzusammensetzung sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete Tenside sind die zuvor genannten.

Weiterhin können die Duschgel-/Shampoo-Formulierungen zusätzliche Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens eine erfindungsgemäße Polymerzusammensetzung in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 5 Gew.-%, vorzugsweise 0,1 bis 3 Gew.-%, wenigstens einer erfindungsgemäßen Polymerzusammensetzung,
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0,05 bis 5 Gew.-% wenigstens eines kosmetisch aktiven Wirkstoffs, sowie
f) 0 bis 20 Gew.-%, vorzugsweise 0,1 bis 10 Gew.-%, wenigstens eines von a) bis e) und g) verschiedenen wasserlöslichen oder wasserdispergierbaren Polymers,
g) 0 bis 45 Gew.-%, vorzugsweise 0,05 bis 25 Gew.-%, weitere Bestandteile,
wobei sich die Komponenten a) bis g) zu 100 Gew.-% addieren.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerzusammensetzungen eignen sich insbesondere als Verdicker in Haarstyling-Zubereitungen, insbesondere Haarschäumen und Haargelen.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Die erfindungsgemäßen Polymerzusammensetzungen eignen sich auch für Styling-Gele. Als zusätzliche Gelbildner können alle in der Kosmetik üblichen Gelbildner eingesetzt werden. Hierzu wird auf die zuvor genannten konventionellen Verdicker Bezug genommen.

Die erfindungsgemäßen Polymerzusammensetzungen eignen sich auch für Shampooformulierungen, die zusätzlich übliche Tenside enthalten.

In den Shampooformulierungen können zur Erzielung bestimmter Effekte übliche Konditioniermittel in Kombination mit den erfindungsgemäßen Polymerzusammensetzungen eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die erfindungsgemäßen Polymerzusammensetzungen eignen sich ebenso für die Verwendung in pharmazeutischen Zubereitungen jeder Art und im Überzug solcher pharmazeutischer Zubereitungen.

Ein weiterer Gegenstand der Erfindung ist die Verwendung einer Verbindung der Formel (I) oder der Formel (I.1), wie zuvor definiert, als Hilfsstoff in der Pharmazie.

Typische pharmazeutische Zusammensetzungen enthalten
A) wenigstens eine erfindungsgemäße Polymerzusammensetzung, wie zuvor definiert,
B) wenigstens einen pharmazeutisch akzeptablen Wirkstoff und
C) gegebenenfalls wenigstens einen weiteren, von A) und B) verschiedenen pharmazeutisch akzeptablen Hilfsstoff.

Pharmazeutisch akzeptable Hilfsstoffe C) sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe C) können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, zusätzliche Verdicker, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung von erfindungsgemäßen pharmazeutischen Mitteln können die Wirkstoffe mit einem geeigneten Exzipienten vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendenden Copolymere auch zum Einsatz in oralen Darreichungsformen wie Tabletten, Kapseln, Pulvern, Lösungen. Hier können sie den schwerlöslichen Arzneistoff mit einer erhöhten Bioverfügbarkeit zur Verfügung stellen. Bei der parenteralen Applikation können neben Solubilisaten auch Emulsionen, beispielsweise Fettemulsionen eingesetzt werden.

Pharmazeutische Formulierungen der oben genannten Art können durch Verarbeiten der erfindungsgemäßen Polymerzusammensetzung mit pharmazeutischen Wirkstoffen nach herkömmlichen Methoden und unter Einsatz bekannter und neuer Wirkstoffe erhalten werden.

Der Gehalt an wenigstens einer erfindungsgemäßen Polymerzusammensetzung in den pharmazeutischen Mitteln liegt, abhängig vom Wirkstoff, im Bereich von 0,01 bis 50 Gew.-%, bevorzugt 0,1 bis 40 Gew.-%, besonders bevorzugt 1 bis 30 Gew.-%, bezogen auf das Gesamtgewicht des Mittels.

Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Neben der Anwendung in der Kosmetik und in der Pharmazie eignen sich die erfindungsgemäßen Polymerzusammensetzungen auch im Lebensmittelbereich. Zu den Lebensmittelzubereitungen sind im Rahmen der vorliegenden Erfindung auch Nahrungsergänzungsmittel wie z. B. Lebensmittelfarbstoffe enthaltende Zubereitungen und diätetische Lebensmittel zu verstehen. Darüber hinaus eigenen sich die erfindungsgemäßen Polymerzusammensetzungen auch für Futtermittelzusätze für die Tierernährung.

Außerdem eignen sich die erfindungsgemäßen Polymerzusammensetzungen zur Herstellung von Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

### Überzugsmittel für pharmazeutische Darreichungsformen

Die zuvor beschriebenen Polymerzusammensetzungen eignen sich hervorragend zur Herstellung pharmazeutischer Mittel. Sie dienen dabei z. B. als polymere Filmbildner, insbesondere zur Herstellung von Überzugsmitteln für pharmazeutische Darreichungsformen.

Die Formulierungsgrundlage für erfindungsgemäße Überzugsmittel für pharmazeutische Darreichungsformen kann wenigstens einen weiteren der zuvor bei den pharmazeutischen Formulierungen genannten pharmazeutisch akzeptablen Hilfsstoffe enthalten.

Geeignete Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Weichmacher, Antiklebemittel, Antioxidantien, Stabilisatoren, Porenbildner, Neutralisierungsmittel, Glanzmittel, Farbstoffe, Pigmente, Desinfektions- oder Konservierungsmittel, Verdickungsmittel, etc. Solche Stoffe sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Übliche Mengen der Hilfsstoffe liegen in einem Bereich von jeweils 0 bis 50 Gew.-%, bevorzugt 0 bis 20 Gew.-%, insbesondere 0,01 bis 10 Gew.-%, bezogen auf das Gesamtgewicht des Überzugsmittels.

Die Herstellung der Überzugsmittel kann z. B. durch inniges Vermischen einer erfindungsgemäßen Polymerzusammensetzung mit wenigstens einem Hilfsstoff erfolgen. In einer besonders bevorzugten Ausführungsform wird die erfindungsgemäße Polymerzusammensetzung als solche, ohne vorherige Trocknung, zur Herstellung eines Überzugsmittels eingesetzt. Möglich ist jedoch auch, eine erfindungsgemäße Polymerzusammensetzung zunächst einer Trocknung zu unterziehen und die so erhaltene Polymerzusammensetzung zur Herstellung eines Überzugsmittels einzusetzen. Die Polymerzusammensetzung kann dann z. B. in Pulverform, als Schmelze oder Lösung zur Herstellung des Überzugsmittels eingesetzt werden.

Ein weiterer Gegenstand der Erfindung ist ein Überzugsmittel, enthaltend eine solche wässrige Polymerzusammensetzung oder eine daraus durch Trocknung erhältliche Polymerzusammensetzung.

Ein weiterer Gegenstand der Erfindung ist ein pharmazeutisches Mittel, enthaltend
A) wenigstens eine erfindungsgemäße Polymerzusammensetzung,
B) wenigstens einen pharmazeutisch akzeptablen Wirkstoff, und
C) gegebenenfalls wenigstens einen pharmazeutisch akzeptablen Hilfsstoff.

Die erfindungsgemäßen Polymerzusammensetzungen können in den pharmazeutischen Mitteln z. B. zur Herstellung eines Bindemittels oder eines Überzugsmittels dienen. Die funktionelle Wirkung tritt dabei in der Regel durch Trocknung und/oder Verfilmung des Überzugs- oder Bindemittels ein. Diese Trocknung und/oder Verfilmung kann, unabhängig von dem Auftragsverfahren, durch Zuführung von Energie erfolgen. Dies kann über Konvektion (Wärme), Strahlung (Infrarot oder Mikrowellen) oder Leitung erfolgen. Für den Auftrag als Dispergiermittel eingesetztes Wasser verdampft dabei, gegebenenfalls kann auch ein Vakuum angewendet werden, um das Verdampfen zu beschleunigen.

Das erfindungsgemäße Überzugsmittel kann z. B. in Pulverform oder als Schmelze durch Granulieren, Gießen, Ausstreichen oder mittels Sprühauftrag angewendet werden. Die erfindungsgemäßen Überzugsmittel können zusätzlich wenigstens eine weitere Polymerkomponente enthalten.

Die erfindungsgemäße Formulierung eignet sich zur Verabreichung grundsätzlich beliebiger pharmazeutischer Wirkstoffe, die vorzugsweise in isolierter oder geschützter Form verabreicht werden können, wie Antidepressiva, Betarezeptorenblocker, Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen- Darmtherapeutika, Migränemittel, Mineralstoffpräparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika, Nutraceuticals, Vitamine, Carotinoide und Aminosäuren.

Die erfindungsgemäßen Polymerzusammensetzungen werden bevorzugt zum Überziehen von Tabletten, Extrudaten, Minitabletten, Kapseln, Weichkapseln, Granulaten, Pellets, Mikropellets, Mikrokapseln, Kristallen eingesetzt. Gecoatete Granulate, Pellets, Mikropellets, Mikrokapseln, Kristalle können mit geeigneten Hilfsstoffen abgemischt und zu Tabletten verpresst werden, die in Wasser schnell zerfallen und die gecoateten feinen Formlinge wieder freigeben. Auf diese Weise können so genannte MUPS-Formen, multiple unit particulate systems hergestellt werden. Dies sind Tabletten, die nach der Einnahme zerfallen und die mit einem erfindungsgemäßen Überzugsmittel überzogene Untereinheiten freisetzen. Besondere Bedeutung haben hierbei so genannte oral dispersibles, d. h. Tabletten, die im Mund innerhalb von kurzer Zeit zerfallen und geschmacksmaskierte kleine Formlinge freisetzen.

In einer speziellen Variante werden die erfindungsgemäßen Polymerzusammensetzungen zum Granulieren von Wirkstoffen gegebenenfalls mit entsprechenden Hilfsstoffen verwendet und die Granulate anschließend zu Tabletten verpresst.

Die erfindungsgemäßen Polymerzusammensetzungen können auch zur Herstellung von transdermalen Wirkstoffpflastern oder Sprays herangezogen werden.

Geeignet ist ebenfalls die Einbettung von Agglomeraten in Polyethylenglykol oder Lipide zur Herstellung von Suppositorien oder vaginalen Arzneiformen.

Wirkstoffklassen und Substanzen, die oftmals bitteren Geschmack hervorrufen können und sich vorteilhaft mit dem erfindungsgemäßen Überzugs- und Bindemittel formulieren lassen, sind z. B.:
Analgetika und Antirheumatika, wie Paracetamol, Diclofenac, Aceclofenac, Ibuprofen, Ketoprofen, Flubiprofen, Acetylsalicylsäure, Levacetylmethadol und Oxycodon;
Psychopharmaka, wie Promethazine, Donepezil, Modafinil, Nefazodon, Reboxetin, Sertindol und Sertralin;
Antibiotika, wie Erythromycin, Roxithromycin, Clarithromycin, Grepafloxacin, Ciprofloxacin, Levofloxacin, Sparfloxacin, Trovafloxacin und Nevirapin;
Betablocker, wie Propranolol, Metoprolol, Bisoprolol und Nebivolol; Antidiabetika, wie Metformin, Miglitol und Repaglinid;
H₁-Antihistaminika, wie Diphenhydramin, Fexofenadin und Mizolastin;
H₂ Antihistaminika, wie Cimetidin, Famotidin, Roxatidin, Nizatidin, Ticlopidin, Cetirizin und Ranitidin;
Vitamine wie Thiaminnitrat sowie Chinidin-Sulfat, Amyloprilose-HCl, Pseudoephedrin-HCl, Sildenafil, Topiramat, Granisetron, Rebamipide, Chinin-HCI, etc.

Die erfindungsgemäßen Überzugsmittel weisen eine niedrige Wasserdampf- und Sauerstoffpermeabilität auf und ermöglichen dadurch die Formulierung und Stabilisierung besonders wasserdampfempfindlicher oder sauerstoffempfindlicher Arzneistoffe wie z. B. Acetylsalicylsäure, Enalapril, Cortisonacetat, Omeprazol, Carotinoide. Hierbei hat der Überzug protektiven Charakter.

Darüber hinaus können die erfindungsgemäßen Überzugsmittel zur Trennung inkompatibler Wirk- oder Hilfsstoffe in Darreichungsformen herangezogen werden, indem ein oder mehrere Bestandteile umhüllt werden und so der gegenseitige Kontakt vermieden wird.

Die unerwartet sehr guten anwendungstechnischen Eigenschaften der erfindungsgemäßen Filmüberzüge werden ermöglicht durch eine ausgezeichnete homogene Verfilmung der Polymerzusammensetzung, eine geringe Klebrigkeit der Filme und die gute Flexibilität bzw. Dehnbarkeit der Überzüge, so dass auch bei Quellung des Tabletten- oder Pelletkerns der Filmüberzug nicht reißt. Hierbei überrascht insbesondere die Kombination hohe Flexibilität mit extrem niedriger Klebrigkeit, da normalerweise Polymere entweder hart, d. h. wenig flexibel und nicht klebrig oder weich, d. h. flexibel aber klebrig sind.

Die Polymerzusammensetzungen sind niedrigviskos, so dass hohe Feststoffkonzentrationen in der Sprühsuspension und ein extrem kurzer Sprühprozess realisiert werden können. Die Feststoffkonzentrationen der Sprühsuspensionen betragen üblicherweise 10 bis 60 Gew.-%, bevorzugt 20 bis 50 Gew.-% und insbesondere 25 bis 40 Gew.-%. Die Konzentrationen bezogen auf festes erfindungsgemäß eingesetztes aminogruppenhaltiges Polymer liegen üblicherweise in einem Bereich von 5 bis 50 Gew.-%, bevorzugt von 10 bis 40 Gew.-% und insbesondere von 20 bis 35 Gew.-%. Die niedrigen Viskositäten sorgen für eine sehr feine und gleichmäßige Zerstäubung in der Sprühdüse, ein sehr gutes Spreiten auf der Tabletten- oder Pelletoberfläche und eine schnelle und homogene Verfilmung. Die Einarbeitung von Farbstoffen und Pigmenten erfolgt auf üblichem Weg, ist aber extrem einfach und schnell und ist lösungsmittelfrei. Das einfache Handling resultiert aus der Tatsache, dass die erfindungsgemäß eingesetzten aminogruppenhaltigen Polymerzusammensetzungen die Pigmente in der Sprühsuspension stabilisieren. Innerhalb von etwa 10 min lässt sich so eine sprühfertige Suspension herstellen.

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, USP, JP) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, Farbstoffe, Stabilisatoren, Sprengmittel, Trocknungsmittel, Trübungsmittel, Verdickungsmittel, Wachse, Weichmacher, Aromen, Süßstoffe, Hilfsstoffe zur Permeationserniedrigung etc. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Cantor-Verlag, 1996, dargestellt sind.

Besonders geeignete Weichmacher sind z. B.: Triethylcitrat, Tributylcitrat, Triacetin, Acetyltriethylcitrat, Labrasol, Glycofurol, Polypropylenglykol 400.

Die Permeabilität der Filmüberzüge kann durch Einarbeitung von anorganischen Feststoffen (Pigmenten wie z. B. Talkum, Kaolin, Titandioxid) oder lipophilen organischen Feststoffen wie Fetten Wachsen, Glyceride, Fettsäuren wie z. B. Stearinsäure, Fettalkohole wie z. B. Stearylalkohol weiter herabgesetzt werden.

Zur Herstellung der erfindungsgemäßen pharmazeutischen Mittel können die Wirkstoffe mit geeigneten Hilfsstoffen (Excipients) vermischt oder verdünnt werden. Hilfsstoffe können feste oder halbfeste Materialien sein, die als Vehikel, Träger oder Medium für den Wirkstoff dienen können. Die Zumischung weiterer Hilfsstoffe erfolgt gewünschtenfalls in der dem Fachmann bekannten Weise.

In gleicher Weise können auch veterinärmedizinische Darreichungsformen, insbesondere Rumen-stabile Formen, sowie Darreichungsformen mit Vitaminen, Carotinoiden, Spurenelementen, Nutraceuticals, Aminosäuren und Nahrungsergänzungsstoffen hergestellt werden. Letztere können auch als Lebensmittel oder Supplements gelten.

Ein weiterer Gegenstand der Erfindung ist die Verwendung einer erfindungsgemäßen Polymerzusammensetzung , wie zuvor definiert, zur Membranherstellung, in der Kosmetik, im Pflanzenschutz, für Saatgutcoating, in Lebensmitteln, in Tiernahrung, als Klebrohstoffe, zu Papierherstellung, als Binde- oder Hilfsmittel für Leder und Textil, als mikrobizide Oberflächenbeschichtung, im Vliesstoffbereich, in Wasch- und Reinigungsmittel, zur Herstellung von Anstrichen, im Bausektor.

Die Erfindung wird anhand der im Folgenden beschriebenen Figuren und der Beispiele näher erläutert. Dabei sollen die Figuren und Beispiele nicht als einschränkend für die Erfindung verstanden werden.

In den nachfolgenden Figuren und Beispielen werden folgende Abkürzungen verwendet:
EO: Ethylenoxid
PO: Propylenoxid
BO: Butylenoxid
Mₙ: Zahlenmittleres Molekulargewicht
M_{w}: Gewichtsmittleres Molekulargewicht
PET: Polyethylenterephthalat
T_{L}-Wert: Wert für die Lichtdurchlässigkeit
n.b.: nicht bestimmt
rad/s: Radiant pro Sekunde (radiant per second)
pphm: Gewichtsteile pro 100 Gewichtsteile Monomer (parts per hundred monomer).

### FIGURENBESCHREIBUNG

### Figur 1:

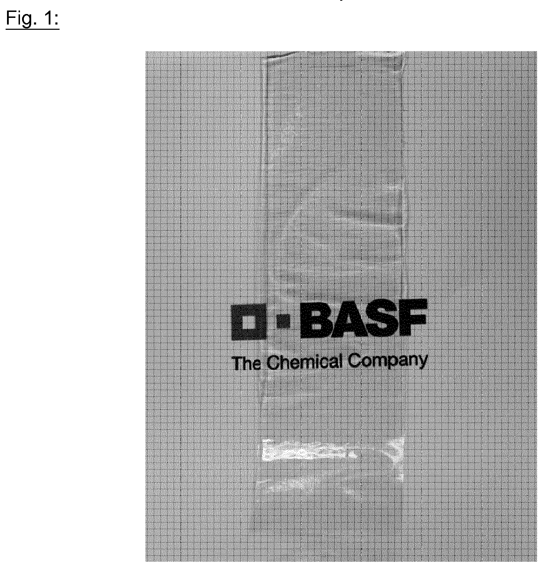

Figur 1 zeigt eine Abbildung mit einer Folie auf Basis der erfindungsgemäßen Polymerzusammensetzung 10 vor einem weißen Hintergrund mit schwarzer Schrift.

### BEISPIELE

### I) Analytik:

### I.a) Bestimmung der Wasserlöslichkeit

Zur Bestimmung der Wasserlöslichkeit wurden 5 g der jeweiligen Polymerzusammensetzung in ein 1 L Becherglas gegeben und 900 mL Wasser, welches zuvor auf 40°C erhitzt wurde, zugegeben. Die Mischung wurde 20 Minuten lang mit einem Magnetrührer bei 40°C gerührt und der pH-Wert mit Natronlauge auf 8 eingestellt. Wasserlösliche Polymergele führten zu transparenten oder leicht trüben Lösungen.

### I.b) Bestimmung des gewichtsgemittelten Molekulargewichts (M_{w}):

Das gewichstsgemittelte Molekulargewicht des Polymers wurde durch Gelpermeationschromatographie (GPC) bestimmt. Dazu wurden die folgenden Geräte und Chromatographiemethoden verwendet:
Standard: Polyacrylsäure, neutralisiert
Elutionsmittel: 0,08 Mol/L Tris, pH 7,0, + 0,15 Mol/L Na Cl + 0,01 Mol/L NaN₃ in deionisiertem Wasser
Fluss: 0,8 mL/min
Säulensatz: 1 Vorsäule (l = 5 cm), 2Trennsäulen (l = je 30 cm)
Säulentemperatur: 35°C
Detektor: DRI (Refractive Index Detector) Agilent 1100

### I.c) Bestimmung der Glasübergangstemperatur (T_{g})

Die Bestimmung der Glasübergangstemperatur erfolgte mittels dynamischer Differenzkalorimetrie (DSC: Differential Scanning Calorimetrie) in an sich bekannter Weise. Die DSC-Messung erfolgte an thermisch konditionierten Proben, die vor der eigentlichen Messung auf 100°C aufgeheizt und schnell abgekühlt wurden. Die Bestimmung der Glasübergangstemperatur erfolgte unter Stickstoff in offenen Aluminiumtiegeln bei einer Aufheizrate und Abkühlrate von 20 K/min.

### I.d) Bestimmung des Wassergehalts nach Trocknung

Der Wassergehalt des teilchenförmigen Polymers wurde coulometrisch durch Karl-Fischer-Titration bestimmt.

### II) Herstellungsbeispiele:

### Allgemeine Herstellungsvorschrift:

In einem Glasreaktor, ausgestattet mit drei Zuläufen, Stickstoffeinlass und einem Ankerrührer, wurde die Polyetherkomponente (PE), der Regler (R) und das Lösungsmittel (LM) in einer Menge gemäß Tabelle 1 bzw. Tabelle 2 vorgelegt, für ein paar Minuten mit Stickstoff gespült und auf 75°C erwärmt. Zur Vorlage wurden anschließend bei 75°C und unter Rühren bei 100 Umdrehungen/Minute innerhalb von 4 Stunden die Zuläufe 1 - 3 simultan zugegeben. Zulauf 1 enthielt Monomer (M), Zulauf 2 enthielt einen Starter (S), gelöst in einer kleinen Menge nicht-ionischem Tensid (PE) und/oder Lösungsmittel (LM) und der Zulauf 3 enthielt eine weitere Menge Regler (R) und gegebenenfalls Lösungsmittel. Nach der Zugabe der Zuläufe 1, 2 und 3 wurde die Mischung zur Nachpolymerisation für eine weitere Stunde bei 75°C und bei 100 Umdrehungen/Minute gerührt. Anschließend wurde das Polymerisat in ein Becherglas gegossen und sofort auf Raumtemperatur abgekühlt.

Zur Herstellung der erfindungsgemäßen Polymerzusammensetzungen wurden folgende Einsatzstoffe verwendet:
- M1:: Acrylsäure
- M2:: Methacrylsäure
- PE1:: beidseitig endständig CₓH₂ₓ₊₁/C_{y}H_{2y+1}-terminiertes Polyethylenoxid mit einer freien OH-Gruppe und x, y = 6 - 14
- PE2:: C₁₃-Oxoalkoholethoxylat mit 20 EO
- PE3:: propoxylierte Ethylenglykole mit einem EO-Anteil von 50%
- PE4:: Polyethylenglycol, Molekulargewicht von 400 g/mol
- PE5:: Polypropylenglycol, Molekulargewicht von 400 g/mol
- PE6:: Alkylpolyglucosid
- R1:: 2-Mercaptoethanol
- LM1:: Wasser
- LM2:: Isopropanol
- S1:: tert-Butyl peroxyneodekanoat (Reinheit: 97%) (CAS-Nr. 26748-41-4)
- S2:: 2,2'-Azobis(2-methylpropionamidine)dihydrochloride (CAS-Nr. 2997-92-4)

Die Herstellung der erfindungsgemäßen Polymerzusammensetzungen der Beispiele 1 bis 15 erfolgte nach der oben beschriebenen allgemeinen Herstellungsvorschrift. Als Monomerkomponente (M) wurde Acrylsäure oder Methacrylsäure verwendet. Die eingesetzte (Meth)acrylsäure und die jeweilige Polyetherkomponente (PE) wurden in den angegebenen Gewichtsteilen zugegeben, wobei die Menge des Lösungsmittels (LM), des Starters (S) sowie des Reglers (R) in den Polymermischungen stark variiert wurden. Die Mengen (in [g]) der Einsatzstoffe sind in den nachfolgenden Tabellen 1 und 2 zusammengefasst. Die Beispiele 14 und 15 sind nicht erfindungsgemäß.

Die Polymerzusammensetzung der Beispiele 1 bis 9 wurden zunächst in Form von Gelen bzw. von lösungsmittelhaltigen Feststoffen erhalten. Nach der Synthese wurden jeweils ca. 100 g der Polymerzusammensetzund in eine Aluminiumschale gegossen und im Trockenschrank zunächst bei 80°C und Umgebungsdruck und anschließend bei 50°C und 100 mbar weiter getrocknet. Das so erhaltene feste Polymermaterial wurde in einem Mörser zerkleinert. Die Konsistenz und die analytischen Parameter der daraus resultierenden festen Polymerzusammensetzungen sind in der nachfolgenden Tabelle 3 zusammengefasst.

**Tabelle 1**

| Bsp.-Nr. | Vorlage | | Zulauf 1 | Zulauf 2 | | Zulauf 3 | |
|---|---|---|---|---|---|---|---|
| | PE [g] (PE-Nr.) | LM [g] (LM-Nr.) | M [g] | S [g] (S-Nr.) | LM [g] (LM-Nr.) | R [g] | LM [g] (LM-Nr.) |
| 1 | 120 (1) | 162 (1) | 360 (1) | 8.45 (1) | 30 (2) | 18 (1) | -- |
| 2 | 120 (1) | 162 (1) | 297 (1) + 63 (2) | 9.1 (2) | 41 (1) | 18 (1) | -- |
| 3 | 120 (1) | 162 (1) | 252 (1) + 108 (2) | 9.1 (2) | 41 (1) | 18 (1) | -- |
| 4 | 120 (1) | 162 (1) | 164(1)+ 196 (2) | 9.1 (2) | 41 (1) | 18 (1) | -- |
| 5 | 200 (1) | 263 (1) | 158 (1) + 442 (2) | 15.2 (2) | 75 (1) | 30 (1) | -- |
| 6 | 120 (1) | 162 (1) | 360 (2) | 9.1 (2) | 41 (1) | 18 (1) | -- |
| 7 | 160 (1) | 162 (1) | 146 (1) + 174 (2) | 8.1 (2) | 42 (1) | 16 (1) | -- |
| 8 | 150 (1) | 243 (1) | 273 (1) + 327 (2) | 15.2 (2) | 75 (1) | 30 (1) | -- |
| 9 | 120(1) | 231 (1) | 273 (1) + 327 (2) | 15.2 (2) | 75 (1) | 30 (1) | -- |
| 10 | 256 (1) | 259 (1) | 512(1) | 12 (1) | 41 (2) | 26 (1) | -- |

**Tabelle 2**

| Bsp.-Nr. | Vorlage | | Zulauf 1 | Zulauf 2 | | Zulauf 3 | |
|---|---|---|---|---|---|---|---|
| | PE [g] (PE-Nr.) | LM [g] (LM-Nr.) | M [g] | S [g] (S-Nr.) | LM [g] (LM-Nr.) | R [g] | LM [g] (LM-Nr.) |
| 11 | 250 (2) | 247(1) | 500(1) | 3.55 (2) | 46 (1) | 10 (1) + 27 (2) | 28 (1) |
| 12 | 250 (3) | 247 (1) | 500 (1) | 3.55 (2) | 46 (1) | 10 (1) + 27 (2) | 28 (1) |
| 13 | 250 (4) | 247 (1) | 500 (1) | 3.55(2) | 46 (1) | 10 (1) + 27 (2) | 28 (1) |
| 14 | 250 (5) | 247 (1) | 500 (1) | 3.55 (2) | 46 (1) | 10 (1) + 27 (2) | 28 (1) |
| 15 | 250 (6) | 247 (1) | 500 (1) | 3.55 (2) | 46 (1) | 10 (1) + 27 (2) | 28 (1) |

**Tabelle 3**

| **Bsp.-Nr.** | **Mw [g/mol]** | **Form** | **Tg [°C]** | **H₂O conc. [%]** |
|---|---|---|---|---|
| 1 | 5700 | Granulat | 32 | 1.6 |
| 2 | 3800 | Granulat | 44 | 1.2 |
| 3 | 4200 | Granulat | 51 | 1.4 |
| 4 | 3500 | Granulat | 60 | 1.8 |
| 5 | 4100 | Granulat | 96 | n.b. |
| 6 | 3500 | Granulat | 108 | 5.5 |
| 7 | 3700 | Granulat | 39 | 1.9 |
| 8 | 4200 | Granulat | 74 | n.b. |
| 9 | 4500 | Granulat | 90 | n.b. |
| 10 | 4400 | Film | 13 | n.b. |

| | | | | |
|---|---|---|---|---|
| n.b. = nicht bestimmt | | | | |

### III) Beispiel zur Herstellung von Polymerfilmen:

Die Polymerzusammensetzung des Beispiels 10 wurde durch Erwärmen auf 80°C vollständig verflüssigt und mit einer 120 µm Rakel auf eine flache Si-Oberfläche ausgestrichen. Anschließend wurde der Film für 24 Stunden bei Raumtemperatur getrocknet. Es wurde ein flexibler Film erhalten, der bei pH 8 und 40°C gut wasserlöslich war.

Figur 1 zeigt eine Abbildung mit einer Folie auf Basis der erfindungsgemäßen Polymerzusammensetzung 10 vor einem weißen Hintergrund mit schwarzer Schrift.

### IV) Beispiel zur Herstellung eines wirkstoffhaltigen Polymerfilms:

Die Polymerzusammensetzung des Beispiels 10 wurde durch Erwärmen auf 70°C verflüssigt. Dann wurden 1% (w/w) des Bacillus Sp Protease P3111 (Sigma Aldrich) Enzyms unter Rühren zugegeben, bis eine homogene Mischung entstand. Die flüssige Polymerzusammensetzung wurde mit einer 120 µm Rakel auf eine Si-Oberfläche ausgestrichen und sofort auf Raumtemperatur gekühlt. Anschließend wurde der Film für 24 Stunden bei Raumtemperatur getrocknet. Es wurde ein flexibler Film erhalten, der bei pH 8 und 40°C gut unter Freisetzung des eingebetteten Enzyms wasserlöslich war (gemessen nach der AAPF Protease Aktivitätsmethode) (AAPF = Alanin(A)-Alanin(A)-Proline(P)-Phenylalanin(F)).

## Patentansprüche

1. Verfahren zur Herstellung einer Polymerzusammensetzung, bei dem man
a) eine Monomerzusammensetzung M) bereitstellt, die
A) wenigstens eine α,β-ethylenisch ungesättigte Carbonsäure, und
B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
enthält,
wobei die Komponente A) teilweise oder vollständig durch C) wenigstens eine ungesättigte Sulfonsäure oder ungesättigte Phosphonsäure ersetzt sein kann,
b) die in Schritt a) bereitgestellte Monomerzusammensetzung M) einer radikalischen Polymerisation in Gegenwart wenigstens einer Polyetherkomponente PE) unterzieht, die keine copolymerisierbare Doppelbindung aufweist und die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, wobei, wenn die Polyetherkomponente PE) ein Polyetherol mit Propylenoxid-Wiederholungseinheiten oder einen Mono- oder Di-(C₁-C₆-alkylether) eines Polyetherols mit Propylenoxid-Wiederholungseinheiten umfasst, der Anteil dieser Propylenoxid-Wiederholungseinheiten im Mittel höchstens 10 Einheiten pro Molekül beträgt und die Polyetherole PE) Ethylenoxid-Homopolymere oder Ethylenoxid/Propylenoxid-Copolymere sind.

2. Verfahren nach Anspruch 1, wobei man
c) das in Schritt b) erhaltene Polymerisat in eine Folie, in eine feste Beschichtung auf einem Substrat oder in Teilchen überführt.

3. Verfahren nach einem der vorhergehenden Ansprüche, wobei die Monomerzusammensetzung M) keine vernetzend wirkenden Monomere B) enthält, die zwei oder mehr als zwei polymerisierbare a, β-ehtylenisch ungesättigte Doppelbildungen pro Molekül aufweisen.

4. Verfahren nach einem der vorhergehenden Ansprüche, wobei die α,β-ethylenisch ungesättigte Carbonsäure A) ausgewählt ist unter Acrylsäure, Methacrylsäure, Ethacrylsäure, α-Chloracrylsäure, Crotonsäure, Maleinsäure, Itaconsäure, Citraconsäure, Mesaconsäure, Glutaconsäure, Aconitsäure, Fumarsäure und Mischungen davon.

5. Verfahren nach einem der vorhergehenden Ansprüche, wobei die Monomerzusammensetzung M) als Komponente C) wenigstens eine ungesättigte Sulfonsäure oder ungesättigte Phosphonsäure enthält, vorzugsweise ausgewählt unter 2-Acrylamido-2-methylpropansulfonsäure, Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, Vinylphosphonsäure, Allylphosphonsäure sowie Mischungen davon.

6. Verfahren nach einem der vorhergehenden Ansprüche, wobei die Monomerzusammensetzung M) zusätzlich wenigstens ein Monomer D) enthält, ausgewählt unter Verbindungen der allgemeinen Formeln (I.a) und (I.b) 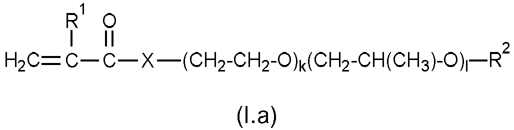 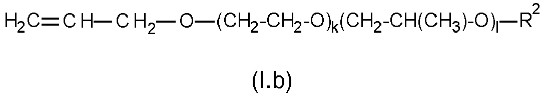 worin
die Reihenfolge der Alkylenoxideinheiten beliebig ist,
k und l unabhängig voneinander für eine ganze Zahl von 0 bis 1000 stehen, wobei die Summe aus k und l mindestens 2 beträgt, vorzugsweise mindestens 5 beträgt,
R¹ für Wasserstoff oder C₁-C₈-Alkyl steht,
R² für Wasserstoff, C₁-C₃₀-Alkyl, C₂-C₃₀-Alkenyl oder C₅-C₈-Cycloalkyl steht,
X für O oder eine Gruppe der Formel NR³ steht, worin R³ für H, Alkyl, Alkenyl, Cycloalkyl, Heterocycloalkyl, Aryl oder Hetaryl steht.

7. Verfahren nach einem der vorhergehenden Ansprüche, wobei die Monomerzusammensetzung M) zusätzlich wenigstens ein Comonomer enthält, ausgewählt unter
E) Vinylaromaten,
F) C₂-C₈ Monoolefinen, nichtaromatischen Kohlenwasserstoffen mit wenigstens zwei konjugierten Doppelbindungen,
G) Estern α,β-ethylenisch ungesättigter Mono- und Dicarbonsäuren mit C₁-C₃₀-Alkanolen,
H) Verbindungen mit einer radikalisch polymerisierbaren α,β-ethylenisch ungesättigten Doppelbindung und wenigstens einer kationogenen und/oder kationischen Gruppe pro Molekül,
I) Estern von Vinylalkohol oder Allylalkohol mit C₁-C₃₀-Monocarbonsäuren,
K) Amidgruppen-haltigen Monomeren,
L) Estern α,β-ethylenisch ungesättigter Mono- und Dicarbonsäuren mit C₂-C₃₀-Alkanediolen, Amiden α,β-ethylenisch ungesättigter Mono- und Dicarbonsäuren mit C₂-C₃₀-Aminoalkoholen mit einer primären oder sekundären Aminogruppe,
M) α,β-ethylenisch ungesättigten Nitrilen,
N) Vinylhalogeniden, Vinylidenhalogeniden,
O) Monomeren mit Harnstoffgruppen,
und Mischungen davon.

8. Verfahren nach einem der vorhergehenden Ansprüche, wobei die Polyetherkomponente PE) wenigstens ein Polyetherol mit einem zahlenmittleres Molekulargewicht im Bereich von etwa 200 bis 100000 g/mol oder einen Mono- oder Di-(C₁-C₂-alkylether) davon umfasst.

9. Verfahren nach einem der vorhergehenden Ansprüche, wobei die Polyetherkomponente PE) wenigstens ein Polyethergruppen-haltiges Tensid umfasst, ausgewählt unter Alkylpolyoxyalkylenethern, Arylpolyoxyalkylenethern, Alkylarylpolyoxyalkylenethern, alkoxylierten tierischen und/oder pflanzlichen Fette und/oder Ölen, Fettaminalkoxylaten, Fettsäureamidalkoxylaten, Fettsäurediethanolamidalkoxylaten, Polyoxyethylensorbitanfettsäureestern, Alkylpolyethersulfaten, Arylpolyethersulfaten, Alkylarylpolyethersulfaten, Alkylpolyethersulfonaten, Arylpolyethersulfonaten, Alkylarylpolyethersulfonaten, Alkylpolyetherphosphaten, Arylpolyetherphosphaten, Alkylarylpolyetherphosphaten, Glycerinethersulfonaten, Glycerinethersulfaten, Monoglycerid(ether)sulfaten, Fettsäureamidethersulfaten, Polyoxyalkylensorbitanfettsäureestern und Mischungen davon.

10. Verfahren zur Herstellung einer Polymerzusammensetzung, wie in einem der Ansprüche 1 bis 9 definiert, in Form einer Folie, bei dem man
a) eine Monomerzusammensetzung M) bereitstellt, die
A) wenigstens eine α,β-ethylenisch ungesättigte Carbonsäure, und
B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
enthält,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, und
c) das in Schritt b) erhaltene Polymerisat, gegebenenfalls nach Zugabe wenigstens eines Wirkstoffes und/oder wenigstens eines Zusatzstoffes, einer Folienbildung unterzieht, vorzugsweise ausgewählt unter Blasformen, Thermoformen, Gießen und Kalandrieren.

11. Verfahren zur Herstellung einer Polymerzusammensetzung, wie in einem der Ansprüche 1 bis 9 definiert, in Form einer festen Beschichtung auf einem Substrat, bei dem man
a) eine Monomerzusammensetzung M) bereitstellt, die
A) wenigstens eine α,β-ethylenisch ungesättigte Carbonsäure, und
B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
enthält,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, und
c) das in Schritt b) erhaltene Polymerisat, gegebenenfalls nach Zugabe wenigstens eines Wirkstoffes und/oder wenigstens eines Zusatzstoffes und/oder wenigstens eines Lösungsmittels und gegebenenfalls unter Erwärmen, in eine fließfähige Form bringt, auf ein Substrat aufträgt und verfestigen lässt.

12. Verfahren zur Herstellung einer Polymerzusammensetzung, wie in einem der Ansprüche 1 bis 9 definiert, in Teilchenform, bei dem man
a) eine Monomerzusammensetzung M) bereitstellt, die
A) wenigstens eine α,β-ethylenisch ungesättigte Carbonsäure, und
B) weniger als 0,1 Gew.-%, bezogen auf das Gesamtgewicht der Monomerzusammensetzung M) vernetzend wirkende Monomere, die zwei oder mehr als zwei polymerisierbare α,β-ethylenisch ungesättigte Doppelbindungen pro Molekül aufweisen,
enthält,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon, und
c) das in Schritt b) erhaltene Polymerisat, gegebenenfalls nach Zugabe wenigstens eines Zusatzstoffes und/oder wenigstens eines Lösungsmittels und gegebenenfalls unter Erwärmen, in eine fließfähige Form bringt und daraus eine teilchenförmige Zusammensetzung formt.

13. Polymerzusammensetzung in fester Form, erhältlich durch ein Verfahren wie in einem der Ansprüche 1 bis 12 definiert.

14. Polymerzusammensetzung in Form einer Folie, wie in Anspruch 13 definiert oder erhältlich durch ein Verfahren wie in einem der Ansprüche 1 bis 10 definiert.

15. Polymerzusammensetzung in Form einer Beschichtung auf einem Substrat, wie in Anspruch 13 definiert oder erhältlich durch ein Verfahren wie in einem der Ansprüche 1 bis 9 und 11 definiert.

16. Polymerzusammensetzung in Form einer Beschichtung auf einem Substrat, wie in Anspruch 13 definiert oder erhältlich durch ein Verfahren wie in einem der Ansprüche 1 bis 9 und 11 definiert, enthaltend wenigstens ein Enzym.

17. Verwendung einer Polymerzusammensetzung, wie in einem der Ansprüche 13 bis 16 definiert oder erhältlich durch ein Verfahren wie in einem der Ansprüche 1 bis 12 definiert,
- in Wasch- und Reinigungsmitteln,
- in Geschirrspülmitteln und in Klarspülern,
- in Hygieneprodukten,
- in kosmetischen Mitteln,
- in pharmazeutischen Mitteln,
- in Pflanzenschutzmitteln,
- in Netzmitteln,
- in Lacken, Beschichtungsmitteln, Klebstoffen, Lederbehandlungs- oder Textilbehandlungsmitteln, etc.,
- bei der Erschließung und/oder Ausbeutung unterirdischer Erdöl- und/oder Erdgaslagerstätten.

18. Verwendung nach Anspruch 17 in einem enzymhaltigen Waschmittel-, Reinigungsmittel- oder Geschirrspülmittel.

19. Umhüllung oder Beschichtung für eine Waschmittel-, Reinigungsmittel- oder Geschirrspülmittelportion, umfassend oder bestehend aus einer Polymerzusammensetzung, wie in einem der Ansprüche 13 bis 16 definiert oder erhältlich durch ein Verfahren wie in einem der Ansprüche 1 bis 13 definiert.

20. Klebstoffzusammensetzung, umfassend oder bestehend aus einer Polymerzusammensetzung, erhältlich durch ein Verfahren wie in einem der Ansprüche 1 bis 12 definiert.

## Claims

1. A process for preparing a polymer composition, in which
a) a monomer composition M) is provided, comprising
A) at least one α,β-ethylenically unsaturated carboxylic acid, and
B) less than 0.1% by weight, based on the total weight of the monomer composition M), of crosslinking monomers having two or more than two polymerizable α,β-ethylenically unsaturated double bonds per molecule,
where component A) may be partly or fully replaced by C) at least one unsaturated sulfonic acid or unsaturated phosphonic acid,
b) the monomer composition M) provided in step a) is subjected to a free-radical polymerization in the presence of at least one polyether component PE) which does not have a copolymerizable double bond and is selected from polyetherols having a number-average molecular weight of at least 200 g/mol and the mono- and di-(C₁-C₆-alkyl) ethers thereof, surfactants containing polyether groups and mixtures thereof, wherein, if the polyether component PE) comprises a polyetherol having repeat propylene oxide units or a mono- or di-(C₁-C₆-alkyl ether) of a polyetherol having repeat propylene oxide units, the proportion of these repeat propylene oxide units averages not more than 10 units per molecule and the polyetherols PE) are ethylene oxide homopolymers or ethylene oxide/propylene oxide copolymers.

2. The process according to claim 1, wherein
c) the polymer obtained in step b) is converted to a film, to a solid coating on a substrate or to particles.

3. The process according to either of the preceding claims, wherein the monomer composition M) does not comprise any crosslinking monomers B) having two or more than two polymerizable α,β-ethylenically unsaturated double bonds per molecule.

4. The process according to any of the preceding claims, wherein the α,β-ethylenically unsaturated carboxylic acid A) is selected from acrylic acid, methacrylic acid, ethacrylic acid, α-chloroacrylic acid, crotonic acid, maleic acid, itaconic acid, citraconic acid, mesaconic acid, glutaconic acid, aconitic acid, fumaric acid and mixtures thereof.

5. The process according to any of the preceding claims, wherein the monomer composition M) comprises, as component C), at least one unsaturated sulfonic acid or unsaturated phosphonic acid, preferably selected from 2-acrylamido-2-methylpropanesulfonic acid, vinylsulfonic acid, allylsulfonic acid, sulfoethyl acrylate, sulfoethyl methacrylate, sulfopropyl acrylate, sulfopropyl methacrylate, 2-hydroxy-3-acryloyloxypropylsulfonic acid, 2-hydroxy-3-methacryloyloxypropylsulfonic acid, styrenesulfonic acid, vinylphosphonic acid, allylphosphonic acid and mixtures thereof.

6. The process according to any of the preceding claims, wherein the monomer composition M) additionally comprises at least one monomer D) selected from compounds of the general formulae (I.a) and (I.b) 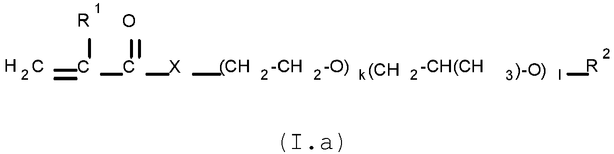 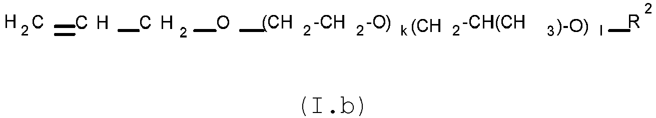 in which
the sequence of the alkylene oxide units is arbitrary,
k and l are each independently an integer from 0 to 1000, where the sum of k and 1 is at least 2, preferably at least 5,
R¹ is hydrogen or C₁-C₈-alkyl,
R² is hydrogen, C₁-C₃₀-alkyl, C₂-C₃₀-alkenyl or C₅-C₈-cycloalkyl,
X is O or a group of the formula NR³ in which R³ is H, alkyl, alkenyl, cycloalkyl, heterocycloalkyl, aryl or hetaryl.

7. The process according to any of the preceding claims, wherein the monomer composition M) additionally comprises at least one comonomer selected from
E) vinylaromatics,
F) C₂-C₈ monoolefins, nonaromatic hydrocarbons having at least two conjugated double bonds,
G) esters of α,β-ethylenically unsaturated mono- and dicarboxylic acids with C₁-C₃₀-alkanols,
H) compounds having one free-radically polymerizable α,β-ethylenically unsaturated double bond and at least one cationogenic and/or cationic group per molecule,
I) esters of vinyl alcohol or allyl alcohol with C₁-C₃₀-monocarboxylic acids,
K) monomers containing amide groups,
L) esters of α,β-ethylenically unsaturated mono- and dicarboxylic acids with C₂-C₃₀-alkanediols, amides of α,β-ethylenically unsaturated mono- and dicarboxylic acids with C₂-C₃₀-amino alcohols having a primary or secondary amino group,
M) α,β-ethylenically unsaturated nitriles,
N) vinyl halides, vinylidene halides,
O) monomers having urea groups,
and mixtures thereof.

8. The process according to any of the preceding claims, wherein the polyether component PE) comprises at least one polyetherol having a number-average molecular weight in the range from about 200 to 100 000 g/mol or a mono- or di-(C₁-C₂-alkyl ether) thereof.

9. The process according to any of the preceding claims, wherein the polyether component PE) comprises at least one surfactant containing polyether groups, selected from alkyl polyoxyalkylene ethers, aryl polyoxyalkylene ethers, alkylaryl polyoxyalkylene ethers, alkoxylated animal and/or vegetable fats and/or oils, fatty amine alkoxylates, fatty acid amide alkoxylates, fatty acid diethanolamide alkoxylates, polyoxyethylene sorbitan fatty acid esters, alkyl polyether sulfates, aryl polyether sulfates, alkylaryl polyether sulfates, alkyl polyether sulfonates, aryl polyether sulfonates, alkylaryl polyether sulfonates, alkyl polyether phosphates, aryl polyether phosphates, alkylaryl polyether phosphates, glyceryl ether sulfonates, glyceryl ether sulfates, monoglyceride (ether) sulfates, fatty acid amide ether sulfates, polyoxyalkylene sorbitan fatty acid esters and mixtures thereof.

10. A process for preparing a polymer composition as defined in any of claims 1 to 9 in the form of a film, in which
a) a monomer composition M) is provided, comprising
A) at least one α,β-ethylenically unsaturated carboxylic acid, and
B) less than 0.1% by weight, based on the total weight of the monomer composition M), of crosslinking monomers having two or more than two polymerizable α,β-ethylenically unsaturated double bonds per molecule,
b) the monomer composition M) provided in step a) is subjected to a free-radical polymerization in the presence of at least one polyether component PE) selected from polyetherols having a number-average molecular weight of at least 200 g/mol and the mono- and di-(C₁-C₆-alkyl) ethers thereof, surfactants containing polyether groups and mixtures thereof, and
c) the polymer obtained in step b), optionally after addition of at least one active ingredient and/or at least one additive, is subjected to a film-forming operation, preferably selected from blow-molding, thermoforming, casting and calendering.

11. A process for preparing a polymer composition as defined in any of claims 1 to 9 in the form of a solid coating on a substrate, in which
a) a monomer composition M) is provided, comprising
A) at least one α,β-ethylenically unsaturated carboxylic acid, and
B) less than 0.1% by weight, based on the total weight of the monomer composition M), of crosslinking monomers having two or more than two polymerizable α,β-ethylenically unsaturated double bonds per molecule,
b) the monomer composition M) provided in step a) is subjected to a free-radical polymerization in the presence of at least one polyether component PE) selected from polyetherols having a number-average molecular weight of at least 200 g/mol and the mono- and di-(C₁-C₆-alkyl) ethers thereof, surfactants containing polyether groups and mixtures thereof, and
c) the polymer obtained in step b), optionally after addition of at least one active ingredient and/or at least one additive and/or at least one solvent and optionally while heating, is converted to a free-flowing form, applied to a substrate and allowed to solidify.

12. A process for preparing a polymer composition as defined in any of claims 1 to 9 in particulate form, in which
a) a monomer composition M) is provided, comprising
A) at least one α,β-ethylenically unsaturated carboxylic acid, and
B) less than 0.1% by weight, based on the total weight of the monomer composition M), of crosslinking monomers having two or more than two polymerizable α,β-ethylenically unsaturated double bonds per molecule,
b) the monomer composition M) provided in step a) is subjected to a free-radical polymerization in the presence of at least one polyether component PE) selected from polyetherols having a number-average molecular weight of at least 200 g/mol and the mono- and di-(C₁-C₆-alkyl) ethers thereof, surfactants containing polyether groups and mixtures thereof, and
c) the polymer obtained in step b), optionally after addition of at least one additive and/or at least one solvent and optionally while heating, is converted to a free-flowing form and a particulate composition is formed therefrom.

13. A polymer composition in solid form, obtainable by a process as defined in any of claims 1 to 12.

14. A polymer composition in the form of a film as defined in claim 13 or obtainable by a process as defined in any of claims 1 to 10.

15. The polymer composition in the form of a coating on a substrate as defined in claim 13 or obtainable by a process as defined in any of claims 1 to 9 and 11.

16. The polymer composition in the form of a coating on a substrate as defined in claim 13 or obtainable by a process as defined in any of claims 1 to 9 and 11, comprising at least one enzyme.

17. The use of a polymer composition as defined in any of claims 13 to 16 or obtainable by a process as defined in any of claims 1 to 12
- in washing and cleaning compositions,
- in dishwashing detergents and in rinse aids,
- in hygiene products,
- in cosmetic compositions,
- in pharmaceutical compositions,
- in crop protection compositions,
- in wetting agents,
- in lacquers, coating compositions, adhesives, leather treatment compositions or textile care compositions, etc.,
- in the development and/or exploitation of underground mineral oil and/or natural gas deposits.

18. The use according to claim 17 in an enzyme-containing washing composition, cleaning composition or dishwashing composition.

19. A sheath or coating for a washing composition portion, cleaning composition portion or dishwashing composition portion, comprising or consisting of a polymer composition as defined in any of claims 13 to 16 or obtainable by a process as defined in any of claims 1 to 13.

20. An adhesive composition comprising or consisting of a polymer composition obtainable by a process as defined in any of claims 1 to 12.

## Revendications

1. Procédé pour la préparation d'une composition de polymère, dans lequel
a) on met à disposition une composition M) de monomères qui contient
A) au moins un acide carboxylique éthyléniquement α,β-insaturé, et
B) moins de 0,1 % en poids, par rapport au poids total de la composition M) de monomères, de monomères à effet réticulant, qui présentent deux ou plus de deux doubles liaisons éthyléniquement α,β-insaturées polymérisables par molécule,
le composant A) pouvant être remplacé partiellement ou totalement par C) au moins un acide sulfonique insaturé ou au moins un acide phosphonique insaturé,
b) on soumet la composition M) de monomères mise à disposition dans l'étape a) à une polymérisation radicalaire en présence d'au moins un composant de type polyéther PE), qui ne présente pas de double liaison copolymérisable et qui est choisi parmi des polyétherols dotés d'un poids moléculaire moyen en nombre d'au moins 200 g/mole et leurs mono-(C₁₋₆-alkyléthers) et di-(C₁₋₆-alkyléthers), des tensioactifs contenant des groupes polyéther et des mélanges correspondants, où, lorsque le composant de type polyéther PE) comprend un polyétherol comportant des motifs répétitifs de type oxyde de propylène ou un mono- (C₁₋₆-alkyléther) ou un di-(C₁₋₆-alkyléther) d'un polyétherol comportant des motifs répétitifs de type oxyde de propylène, la proportion de ces motifs répétitifs de type oxyde de propylène est en moyenne d'au plus 10 motifs par molécule et les polyétherols PE) étant des homopolymères d'oxyde d'éthylène ou des copolymères oxyde d'éthylène/oxyde de propylène.

2. Procédé selon la revendication 1, où
c) on transforme le polymérisat obtenu dans l'étape b) en une feuille, en un revêtement solide sur un substrat ou en particules.

3. Procédé selon l'une quelconque des revendications précédentes, la composition M) de monomères ne contenant pas de monomères B) à effet réticulant qui présentent deux ou plus de deux doubles liaisons éthyléniquement α,β-insaturées polymérisables par molécule.

4. Procédé selon l'une quelconque des revendications précédentes, l'acide carboxylique éthyléniquement α,β-insaturé A) étant choisi parmi l'acide acrylique, l'acide méthacrylique, l'acide éthacrylique, l'acide α-chloroacrylique, l'acide crotonique, l'acide maléique, l'acide itaconique, l'acide citraconique, l'acide mésaconique, l'acide glutaconique, l'acide aconitique, l'acide fumarique et des mélanges correspondants.

5. Procédé selon l'une quelconque des revendications précédentes, la composition M) de monomères contenant en tant que composant C) au moins un acide sulfonique insaturé ou au moins un acide phosphonique insaturé, de préférence choisi parmi l'acide 2-acrylamido-2-méthylpropanesulfonique, l'acide vinylsulfonique, l'acide allylsulfonique, l'acrylate de sulfoéthyle, le méthacrylate de sulfoéthyle, l'acrylate de sulfopropyle, le méthacrylate de sulfopropyle, l'acide 2-hydroxy-3-acryloxypropylsulfonique, l'acide 2-hydroxy-3-méthacryloxypropylsulfonique, l'acide styrènesulfonique, l'acide vinylphosphonique, l'acide allylphosphonique ainsi que des mélanges correspondants.

6. Procédé selon l'une quelconque des revendications précédentes, la composition M) de monomères contenant de plus au moins un monomère D), choisi parmi des composés de formules générales (I.a) et (I.b) 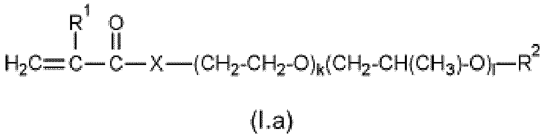 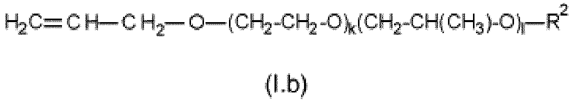 dans lesquelles
l'ordre des motifs de type oxyde d'alkylène est arbitraire,
k et l représentent indépendamment l'un de l'autre un nombre entier de 0 à 1 000, la somme de k et l étant d'au moins 2, de préférence d'au moins 5,
R¹ représente hydrogène ou C₁₋₈-alkyle,
R² représente hydrogène, C₁₋₃₀-alkyle, C₂₋₃₀-alcényle ou C₅₋₈-cycloalkyle,
X représente O ou un groupe de formule NR³, dans laquelle R³ représente H, alkyle, alcényle, cycloalkyle, hétérocycloalkyle, aryle ou hétaryle.

7. Procédé selon l'une quelconque des revendications précédentes, la composition M) de monomères contenant de plus au moins un comonomère, choisi parmi
E) des composés aromatiques vinyliques,
F) des monooléfines en C₂₋₈, des hydrocarbures non aromatiques comportant au moins deux doubles liaisons conjuguées,
G) des esters d'acides monocarboxyliques et dicarboxyliques éthyléniquement α,β-insaturés avec des C₁₋₃₀-alcanols,
H) des composés comportant une double liaison éthyléniquement α,β-insaturée polymérisable par voie radicalaire et au moins un groupe cationogène et/ou cationique par molécule,
I) des esters d'alcool vinylique ou d'alcool allylique avec des acides monocarboxyliques en C₁₋₃₀,
K) des monomères contenant des groupes amide,
L) des esters d'acides monocarboxyliques et dicarboxyliques éthyléniquement α,β-insaturés avec des C₂₋₃₀-alcanediols, des amides d'acides monocarboxyliques et dicarboxyliques éthyléniquement α,β-insaturés avec des C₂₋₃₀-aminoalcools dotés d'un groupe amino primaire ou secondaire,
M) des nitriles éthyléniquement α,β-insaturés,
N) des halogénures de vinyle, des halogénures de vinylidène,
O) des monomères comportant des groupes urée,
et des mélanges correspondants.

8. Procédé selon l'une quelconque des revendications précédentes, le composant de type polyéther PE) comprenant au moins un polyétherol doté d'un poids moléculaire moyen en nombre dans la plage d'environ 200 à 100 000 g/mole ou un mono- (C₁₋₂-alkyléther) ou un di- (C₁₋₂-alkyléther) correspondant.

9. Procédé selon l'une quelconque des revendications précédentes, le composant de type polyéther PE) comprenant au moins un tensioactif contenant des groupes polyéther, choisi parmi les alkylpolyoxyalkylèneéthers, les arylpolyoxyalkylèneéthers, les alkylarylpolyoxyalkylèneéthers, les graisses et/ou les huiles alcoxylées animales et/ou végétales, les alcoxylates d'amines grasses, les alcoxylates d'amides d'acides gras, les alcoxylates de diéthanolamides d'acides gras, les esters d'acides gras de polyoxyéthylènesorbitane, les alkylpolyéthersulfates, les arylpolyéthersulfates, les alkylarylpolyéthersulfates, les alkylpolyéthersulfonates, les arylpolyéthersulfonates, les alkylarylpolyéthersulonfates, les alkylpolyétherphosphates, les arylpolyétherphosphates, les alkylarylpolyétherphosphates, les glycérineéthersulfonates, les glycérineéthersulfates, les monoglycéride(éther)sulfates, les éthersulfates d'amides d'acides gras, les esters d'acides gras de polyoxyalkylènesorbitane et des mélanges correspondants.

10. Procédé pour la préparation d'une composition de polymère, tel que défini selon l'une quelconque des revendications 1 à 9, sous forme d'une feuille, dans lequel
a) on met à disposition une composition M) de monomères qui contient
A) au moins un acide carboxylique éthyléniquement α,β-insaturé, et
B) moins de 0,1 % en poids, par rapport au poids total de la composition M) de monomères, de monomères à effet réticulant, qui présentent deux ou plus de deux doubles liaisons éthyléniquement α,β-insaturées polymérisables par molécule,
b) on soumet la composition M) de monomères mise à disposition dans l'étape a) à une polymérisation radicalaire en présence d'au moins un composant de type polyéther PE), qui est choisi parmi des polyétherols dotés d'un poids moléculaire moyen en nombre d'au moins 200 g/mole et leurs mono-(C₁₋₆-alkyléthers) et di-(C₁₋₆-alkyléthers), des tensioactifs contenant des groupes polyéther et des mélanges correspondants, et
c) on soumet le polymérisat obtenu dans l'étape b), éventuellement après ajout d'au moins un principe actif et/ou d'au moins un additif, à une formation de feuille, de préférence choisie parmi un moulage par soufflage, un thermoformage, un coulage et un calandrage.

11. Procédé pour la préparation d'une composition de polymère, tel que défini selon l'une quelconque des revendications 1 à 9, sous forme d'un revêtement solide sur un substrat, dans lequel
a) on met à disposition une composition M) de monomères qui contient
A) au moins un acide carboxylique éthyléniquement α,β-insaturé, et
B) moins de 0,1 % en poids, par rapport au poids total de la composition M) de monomères, de monomères à effet réticulant, qui présentent deux ou plus de deux doubles liaisons éthyléniquement α,β-insaturées polymérisables par molécule,
b) on soumet la composition M) de monomères mise à disposition dans l'étape a) à une polymérisation radicalaire en présence d'au moins un composant de type polyéther PE), qui est choisi parmi des polyétherols dotés d'un poids moléculaire moyen en nombre d'au moins 200 g/mole et leurs mono-(C₁₋₆-alkyléthers) et di-(C₁₋₆-alkyléthers), des tensioactifs contenant des groupes polyéther et des mélanges correspondants, et
c) on amène le polymérisat obtenu dans l'étape b), éventuellement après ajout d'au moins un principe actif et/ou d'au moins un additif et/ou d'au moins un solvant et éventuellement avec chauffage, sous une forme fluide, on l'applique sur un substrat et on laisse solidifier.

12. Procédé pour la préparation d'une composition de polymère, tel que défini selon l'une quelconque des revendications 1 à 9, sous forme de particules, dans lequel
a) on met à disposition une composition M) de monomères qui contient
A) au moins un acide carboxylique éthyléniquement α,β-insaturé, et
B) moins de 0,1 % en poids, par rapport au poids total de la composition M) de monomères, de monomères à effet réticulant, qui présentent deux ou plus de deux doubles liaisons éthyléniquement α,β-insaturées polymérisables par molécule,
b) on soumet la composition M) de monomères mise à disposition dans l'étape a) à une polymérisation radicalaire en présence d'au moins un composant de type polyéther PE), qui est choisi parmi des polyétherols dotés d'un poids moléculaire moyen en nombre d'au moins 200 g/mole et leurs mono-(C₁₋₆-alkyléthers) et di-(C₁₋₆-alkyléthers), des tensioactifs contenant des groupes polyéther et des mélanges correspondants, et
c) on amène le polymérisat obtenu dans l'étape b), éventuellement après ajout d'au moins un additif et/ou d'au moins un solvant et éventuellement avec chauffage, sous une forme fluide et on forme avec celui-ci une composition particulaire.

13. Composition de polymère sous forme solide, pouvant être obtenue par un procédé tel que défini dans l'une quelconque des revendications 1 à 12.

14. Composition de polymère sous forme d'une feuille, telle que définie dans la revendication 13 ou pouvant être obtenue par un procédé tel que défini dans l'une quelconque des revendications 1 à 10.

15. Composition de polymère sous forme d'un revêtement sur un substrat, telle que définie dans la revendication 13 ou pouvant être obtenue par un procédé tel que défini dans l'une quelconque des revendications 1 à 9 et 11.

16. Composition de polymère sous forme d'un revêtement sur un substrat, telle que définie dans la revendication 13 ou pouvant être obtenue par un procédé tel que défini dans l'une quelconque des revendications 1 à 9 et 11, contenant au moins une enzyme.

17. Utilisation d'une composition de polymère, telle que définie dans l'une quelconque des revendications 13 à 16 ou pouvant être obtenue par un procédé tel que défini dans l'une quelconque des revendications 1 à 12,
- dans des produits de lavage et de nettoyage,
- dans des produits pour laver la vaisselle et dans des produits de rinçage,
- dans des produits d'hygiène,
- dans des produits cosmétiques,
- dans des produits pharmaceutiques,
- dans des produits phytosanitaires,
- dans des agents mouillants,
- dans des vernis, des agents de revêtement, des adhésifs, des produits de traitement du cuir ou de traitement de textiles, etc.,
- lors de l'aménagement et/ou de l'exploitation de gisements souterrains de pétrole et/ou de gaz naturel.

18. Utilisation selon la revendication 17 dans un produit de lavage, un produit de nettoyage ou un produit pour laver la vaisselle.

19. Enveloppe ou revêtement pour une portion de produit de lavage, de produit de nettoyage ou de produit pour laver la vaisselle, comprenant ou étant constitué(e) d'une composition de polymère telle que définie dans l'une quelconque des revendications 13 à 16 ou pouvant être obtenue par un procédé tel que défini dans l'une quelconque des revendications 1 à 13.

20. Composition d'adhésif, comprenant ou étant constituée d'une composition de polymère, pouvant être obtenue par un procédé tel que défini dans l'une quelconque des revendications 1 à 12.
